(19) Europäisches Patentamt
European Patent Office
Office européen des brevets

(11) **EP 1 920 071 B1**

(12) **EUROPEAN PATENT SPECIFICATION**

(45) Date of publication and mention
of the grant of the patent:
**26.12.2018  Bulletin 2018/52**

(51) Int Cl.:
***C12Q 1/68*** *(2018.01)*

(21) Application number: **06813244.8**

(22) Date of filing: **28.07.2006**

(86) International application number:
**PCT/US2006/029475**

(87) International publication number:
**WO 2007/016367 (08.02.2007 Gazette 2007/06)**

(54) **NEOPLASTIC DISEASE-RELATED METHODS, KITS, SYSTEMS AND DATABASES**

NEOPLASTISCHE, KRANKHEITSBEZOGENE VERFAHREN, KITS, SYSTEME UND DATENBANKEN

PROCEDES, KITS, SYSTEMES ET BASES DE DONNEES RELATIFS A DES MALADIES NEOPLASIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.07.2005  US 703681 P**

(43) Date of publication of application:
**14.05.2008  Bulletin 2008/20**

(73) Proprietor: **Siemens Healthcare Diagnostics Inc. Tarrytown, NY 10591 (US)**

(72) Inventors:
• **WIRTZ, Ralph, M.**
**50677 Köln (DE)**
• **AVERDICK, Manuela**
**57803 Krefeld (DE)**
• **BRÜCKL, Wolfgang**
**91054 Erlangen (DE)**
• **WEIN, Axel**
**91054 Erlangen (DE)**
• **THIEL, Robert, P.**
**Oxford, CT 06478 (US)**

(74) Representative: **Schweitzer, Klaus et al Plate Schweitzer Zounek Industriepark Kalle-Albert Rheingaustrasse 196 65203 Wiesbaden (DE)**

(56) References cited:
US-A1- 2003 082 652    US-A1- 2004 018 525
US-A1- 2005 019 798    US-A1- 2005 048 526
US-A1- 2005 136 493

• N. YUKAWA ET AL: "Plasma concentration of tissue inhibitor of matrix metalloproteinase 1 in patients with colorectal carcinoma", BRITISH JOURNAL OF SURGERY, vol. 88, no. 12, 1 December 2001 (2001-12-01), pages 1596-1601, XP055060128, ISSN: 0007-1323, DOI: 10.1046/j.0007-1323.2001.01930.x
• HOLTEN ANDERSEN ET AL: "High preoperative plasma tissue inhibitor of metalloproteinase-1 levels are associated with short survival of patients with colorectal cancer.", CLINICAL CANCER RESEARCH, vol. 6, no. 11, 1 November 2000 (2000-11-01), pages 4292-4299, XP055060143, ISSN: 1078-0432
• E. L. KAPLAN ET AL: "Nonparametric Estimation from Incomplete Observations", JOURNAL OF THE AMERICAN STATISTICAL ASSOCIATION, vol. 53, no. 282, 1 June 1958 (1958-06-01) , pages 457-481, XP055060152, ISSN: 0162-1459, DOI: 10.1080/01621459.1958.10501452

**Description**

FIELD OF THE INVENTION

In one embodiment, the invention provides methods for predicting a clinical outcome related to a patient suffering from or at risk of developing a neoplastic disease comprising: (a) determining a predictor value algorithmically using patient values for (1) at least one marker selected from the group consisting of tumor markers, immune markers, and acute phase markers, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis); and (b) predicting the clinical outcome of the neoplastic disease by evaluating the predictor value.

BACKGROUND OF THE INVENTION

Colorectal cancer (CRC) is the second-most prevalent type of cancer, and is the second-leading cause of cancer-related deaths in industrialized Western countries. An estimated 50,000 new CRC cases are diagnosed annually in Germany alone.

About 75% percent of patients who are diagnosed with CRC undergo curative treatment. The long term survival of CRC patients depends on the tumor stage and the potential development of synchronous or metachronous distant metastases. The 5-year-survival rate of CRC patients exceeds 90% in the UICC stage I (limited invasion without regional lymph node metastasis), but decreases to below 20 % in the UICC stage IV (presence of distant metastasis). Neoadjuvant radiochemotherapy is recommended in UICC stage II und III rectal cancer and adjuvant chemotherapy in UICC stage III which add to prevent locoregional recurrences in rectal cancer and to distant recurrences in colon cancer. However, these strategies are less effective to prevent distant recurrence in rectal cancer and adjuvant chemotherapy is not recommended (outside clinical studies) in R0 resected colorectal cancer presenting in UICC stage IV at diagnosis. Chemotherapy can lead to a partial remission of distant metastases, and can enable secondary curative surgeries and thereby result in long-term survival (five year overall survival) of about 30%. Approximately 25.000 metastatic colorectal cancer patients receive palliative chemotherapy in Germany every year. Response rates of up to 50% have been achieved by the application of modern chemotherapy regimens such as 5-Fluorourical (5-FU), folinic acid (FA), irinotecan and oxaliplatin. For up to 15% of the patients with non-resectable metastases prior to chemotherapy, a secondary R0 resection of the liver or lung metastases is possible and leads to long term survival. Clinical decisions on the therapeutic procedure and extent of resectional treatment in colorectal carcinoma are presently based on imaging and on conventional histopathological features. The diagnostic accuracy of these approaches is limited, which leads to surgical interventions that are often more radical than required, or to chemotherapeutic treatment of patients who do not benefit from this harsh regimen.

As CRC progresses, it can metastasize to the liver and lower a patient's chances of survival. Indeed, hepatic metastases are a major cause of mortality in colorectal cancer patients. However, to date, a detailed analysis of how tumor cells invade the liver and of the interaction of disseminated tumor cells in the liver with the surrounding non-neoplastic liver tissue has not been performed.

Assessing the severity and progression of cancerous disease is difficult, and most often entails biopsying. Biopsying involves possible clinical complications and technological difficulties. Moreover, serial sampling to assess early effectiveness of treatment, and elaborate imaging technologies (e.g. computer tomography), clinically are not feasible for routine use. Consequently the development of less invasive and expensive methods, that identify effective regimens before or shortly after first treatment, is of high clinical value. Analyzing predictive factors would lead to a tumor-tailored individualized therapy with an increase in response to chemotherapy and survival and a decrease in toxicity and economic values.

Hanke, et al., British Journal of Cancer (2003) 88, 1248-50("*Hanke*"), discloses that testing levels of serum levels of collagen (IV) and (VI), tenascin-C, MMP-2, the MMP-9/TIMP-1 complex, and free TIMP-1 taken from patients suffering from colorectal cancer metastatic to the liver. *Hanke* concludes that serum MMP-2 appears to reflect tumor resorption, while serum TIMP-1 may reflect tumor expansion.

United States Patent Application Document No. 20030219842 discloses a method of monitoring the progression of disease or cancer treatment effectiveness in a cancer patient by measuring the level of the extracellular domain (ECD) of the epidermal growth factor receptor (EGFR) in a sample taken from the cancer patient, preferably before treatment, at the start of treatment, and at various time intervals during treatment, wherein a decrease in the level of the ECD of the EGFR in the cancer patient compared with the level of the ECD of the EGFR in normal control individuals serves as an indicator of cancer advancement or progression and/or a lack of treatment effectiveness for the patient.

United States Patent Application Document No. 20030180819 discloses a method of monitoring the progression of disease, or the effectiveness of cancer treatment, in a cancer patient by measuring the levels of one or more analytes of the plasminogen activator (uPA) system, namely, uPA, PAI-1 and the complex of uPA:PAI-1, in a sample taken from the cancer patient, preferably, before treatment, at the start of treatment, and at various time intervals during treatment.

United States Patent Application Document No. 20040157278 discloses a method for detecting the presence of colorectal cancer in an individual, wherein: colorectal cancer is detected by detecting the presence of Reg1α or TIMP1 nucleic acid or amino acid molecules in a clinical sample obtained from the patient and Reg1α or TIMP1 expression is indicative of the presence of colorectal cancer.

United States Patent Application Document No. 20040146921 discloses a method for providing a patient diagnosis for colon cancer, comprising the steps of: (a) determining the level of expression of one or more genes or gene products in a first biological sample taken from the patient; (b) determining the level of expression of one or more genes or gene products in at least a second biological sample taken from a normal patient sample; and (c) comparing the level of expression of one or more genes or gene products in the first biological sample with the level of expression of one or more genes or gene products in the second biological sample; wherein a change in the level of expression of one or more genes or gene products in the first biological sample compared to the level of expression of one or more genes or gene products in the second biological sample is a diagnostic of the disease.

United States Patent Application Document No. 20040146879 discloses nucleic acid sequences and proteins encoded thereby, as well as probes derived from the nucleic acid sequences, antibodies directed to the encoded proteins, and diagnostic and prognostic methods for detecting and monitoring cancer, especially colon cancer. The sequences disclosed in United States Patent Application Document No. 20040146879 have been found to be differentially expressed in samples obtained from colon cancer cell lines and/or colon cancer tissue.

United States Patent No. 6,262,333 discloses nucleic acid sequences and proteins encoded thereby, as well as probes derived from the nucleic acid sequences, antibodies directed to the encoded proteins, and diagnostic methods for detecting cancerous cells, especially colon cancer cells.

US 2004/0018525 A1 discloses a method which is able to detect multiple diagnostic and prognostic markers for neoplastic diseases simultaneously. In the method for prediction, diagnosis or prognosis for malignant neoplasia at least two markers are detected. The markers are genes and fragments thereof or genomic nucleic acid sequences that are located on one chromosomal region which is altered in malignant neoplasia. Alternatively, the markers are a) genes and b) receptor and ligand, or members of the same transduction pathway, or members of synergistic signal transduction pathway, or members of antagonistic signal transduction pathways, or transcription factor and transcription factor binding site.

US 2003/0082652 A1 discloses a method for monitoring colorectal cancer patients for the persistency of minimal residual disease (MRD) or for the recurrence of their cancer disease. In the method, the amount of total, complexed and/or free TIMP-1 in patient samples is determined as a first parameter. In a preferred embodiment, at least one second parameter different from any form of TIMP-1 is determined. From the first and second parameter a combined parameter is calculated which indicates the likelihood of an individual of having MRD and/or recurrent cancer. The second parameter preferably represents the concentration of a tumor marker which is selected from the group consisting of CEA, soluble u-PAR, cathepsin B, cathepsin H, HER2-neu, CA 15-3 and YKL 40, CA 19-9 and CA 242.

In the article of N. Yukawa et al., Plasma concentration of tissue inhibitor of matrix metalloproteinase 1 in patients with colorectal carcinoma, in: Brit. J. of Surgery 88 [2001] 1596-1601, a method is disclosed for determining the plasma level of tissue inhibitor of matrix metalloproteinase (TIMP-1) in samples from patients suffering from colorectal carcinoma who underwent resection of the primary tumor. It was found that the plasma TIMP-1 level was associated significantly with depth of invasion and metastasis to lymph nodes and liver. Also determined was the serum concentration of carcinoembryonic antigen (CEA) and of carbohydrate antigen 19-9 (CA 19-9). CEA and CA 19-9 are both tumor markers.

Notwithstanding the diagnostic, predicative, and prognostic methods described above, the need continues to exist for improved predictive methods which facilitate an accurate and affordable assessment of whether a patient will respond positively to a particular anti-cancer treatment regimen. Cancer patients cannot afford the time and adverse effects associated with current trial and error therapy selection and inaccurate and risky biopsies.

Reliable predictive markers for a chemotherapy response would lead to an individually tailored therapy, and would increase the beneficial outcome (e.g. median overall or progression free survival time) and the rate of secondary curative metastatic resection. However, to date, no such predictive markers in the palliative setting have been validated sufficiently.

## SUMMARY OF THE INVENTION

In one embodiment, the invention provides computer-implemented methods for predicting a clinical outcome related to a patient suffering from a neoplastic disease, namely colorectal cancer, comprising: (a) determining a predictor value algorithmically using patient sample values for (1) at least one marker selected from the group consisting of tumor markers, immune markers, and acute phase markers, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis); and (b) predicting the clinical outcome of the colorectal cancer by evaluating the predictor value, wherein the marker of (1) and the marker of (2) are not the same, wherein the predictor value is (1) determined using an algorithm derived by Cox Regression Analysis and (2) is used to assess the probability that the patient will respond favorably to an antineoplastic treatment regimen. Each of the aforementioned markers is defined hereinafter.

"Predicting a clinical outcome related to a patient suffering from or at risk of developing a neoplastic disease" means predicting: (1) whether a patient who suffers from a neoplastic disease will respond to one or more neoplastic disease treatment regimens; (2) the probability and length of survival of a patient who suffers from a neoplastic disease; and (3) predicating the probability that the patient will develop a neoplastic disease and the likely progression of that neoplastic disease.

"Respond to one or more neoplastic disease regimens" means that the disease treatment regimen is effective in treating a neoplastic disease. Response is defined according to WHO as complete remission (CR), partial remission (PR), non response as stable disease (SD) or progressive disease (PD) according to the size of a indicator lesion, measured in two dimensions.

Predictor values are determined algorithmically by Cox Regression Analysis or by using linear or nonlinear function algorithms.

In another embodiment, the disclosure provides a method for assessing the prognosis of a patient suffering from, or at risk of developing, a neoplastic disease comprising evaluating predictor values determined at one or more time points, wherein: (a) predictor values are determined algorithmically using patient sample values for (1) at least one marker selected from the group consisting of tumor markers , immune markers, and acute phase markers, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis); and (b) the patient's prognosis is assessed by evaluating the predictor values.

Predictor values, and evaluation of patient sample values that are determined in accordance with methods of the invention: (1) correlate to at least tumor control or a primary clinical response to an anti-neoplastic disease treatment regimen, time to neoplastic disease progression, and overall survival; and (2) are applicable to metastatic and non-metastatic cancers.

In one embodiment, methods of the invention predict at least a tumor control or a clinical response to a treatment regimen directed against advanced CRC, less advanced CRC, and neoplastic lesions of different origins (such as breast, ovary, bladder, colon, pancreatic, lung, breast, gastric, head and neck, or prostate cancer).

Neoplastic disease-related markers used in methods of the invention include nucleic or amino acids detected in biopsy samples, body fluids, whole blood samples, and most preferably in serum or plasma samples. Such markers include genes and gene products (e.g., peptides, protein fragments, precursor proteins or mature and/or post-translationally modified proteins) which are expressed by malignant cells and/or surrounding, non-neoplastic stroma cells. In methods of the invention, these gene products can be detected in body fluids before, during or after therapeutic intervention.

While not wishing to be bound by any theory, we have discovered that certain fibrotic processes are indicative of cancer progression. For advanced cancer stages, these fibrotic processes can be accompanied by acute phase reactions of the liver tissue (i.e., cancer-associated tissue reactions). We have found that ECM genes, genes associated with tissue remodeling, or expression products of such genes are very informative with regard to clinical response and overall survival assessment in oncology, particularly if combined with tumor or immune system- related markers. Thus, in methods of the invention, a combination of molecular markers indicating pathological changes of the liver and tumor related markers can be used to assess the clinical outcome of cancerous disease.

Further, we have determined that the detection of either ECM genes, genes associated with tissue remodeling, or expression products of such genes in pretreatment samples is indicative of malignant tissue and disease progression and can be used for prognosis and prediction of tumor response to treatment. Detection of such genes or gene products in serially-obtained samples, such as serum or plasma samples, is indicative of the presence of malignant tissue and/or regression and recurrence of disease.

Again, while we do not wish to be bound by any theory, we conclude that the "injury response" of liver tissue, as detected by measuring fibrotic processes, is a surrogate indicator of neoplasms. This issue is of great clinical relevance with regard to therapeutic decisions made at the earlier stages of tumor development (e.g. therapy management in stage UICC I-III (Dukes A to C) colorectal cancer patients), where no distant metastasis can be detected. For example, for colorectal cancer, a substantial portion of patients develop distant metastasis in the liver without presenting as lymph node-positive during surgical resection. We conclude that evidence of fibrotic processes in the liver is an indicator for high risk patients who do need more radical treatment notwithstanding a positive prognosis based, e.g., on negative lymph node indicators which have been determined surgically.

Methods of the invention enable a health care provider to: (1) predict, prior to therapy, how a patient suffering from a neoplastic disease will respond to an anti-neoplastic treatment regimen; (2) evaluate the status or progress of a neoplastic disease; (3) assess the likelihood and length of survival of a patient suffering from a neoplastic disease; (4) assess the time to progression (TTP) of a neoplastic disease; (5) evaluating toxicity and side effects to an applied chemotherapy; (6) evaluate tissue remodeling implicated in the onset of a neoplastic disease; (7) determine optimum treatment regimens for patients that are predisposed to, or suffer from, a neoplastic disease; (8) design clinical programs useful in monitoring the status or progress of a neoplastic disease in one or more patients; (9) facilitate point of care or remote diagnoses of neoplastic diseases and monitor the status or progress of a neoplastic disease at one or more time points.

In accordance with the invention, based on predictor values and evaluations of patient sample values, a health care provider may, e.g., select either combined targeted therapies, such as small molecule inhibitors which target the kinase domain (e.g. Iressa®, Tarceva®, Vatalanib), an antibody regimen (e.g. bevacizumab, trastuzumab or cetuximab), or a chemotherapy regimen (such as a 5'FU based regimen) or combined chemotherapy regimens including at least one of the above mentioned drugs and oxaliplatin, irinotecan, mitomycin or gemcitabine.

In a preferred embodiment, predictor values are determined by Cox Regression Analysis of discrete and combined marker values corresponding to threshold levels of TIMP-1, Gastrin, Tenascin, Collagen VI, and uPA in a colorectal cancer patient serum sample, and the predictor values are used in a ROC analysis to ascertain the probability that the patient will respond favorably to a given treatment.

In another preferred embodiment, predictor values are determined by Cox Regression Analysis of discrete and combined values corresponding to threshold levels of TIMP-1, Gastrin, Tenascin, Collagen VI, and uPA in a colorectal cancer patient serum sample, and the predictor values are bifurcated and used to generate Kaplan Meier curves which reflect the patient's likelihood of survival

In another preferred embodiment, predictor values are determined by algorithmic analysis of discrete and combined values corresponding to threshold levels of Her-2/neu, EGFr, and VEGF165 in a colorectal cancer patient serum sample, and the predictor values are used to predict the patient's likelihood of survival. Elevated or abased individual levels of one or more of these markers, when analyzed in accordance with the invention, correlate with a decreased chance of patient survival.

In still another preferred embodiment, neoplastic disease predictor values are determined using discrete and combined values corresponding to threshold levels of markers which include MMP-2, Collagen VI, Tenascin and VEGF. Elevated or abased individual levels of any of these markers, when analyzed in accordance with the invention, correlate with a decreased chance of patient survival.

We have discovered that genes that relate to the aforementioned markers represent biological motifs that affect general tissue organization and that display characteristics of disease-associated tissue, particularly in neoplastic cells. Methods of the invention can detect these neoplastic disease-associated phenomena on a DNA, RNA, and protein level.

In still another preferred embodiment of methods of the invention, predictor values are determined at two or more time points and the patient's response to the anti-neoplastic treatment regimen is evaluated by comparing the predictor values determined at each time point.

In still another preferred embodiment, neoplastic disease predictor values are determined using discrete and combined values corresponding to threshold levels of markers which include MMP-2, Gastrin, TIMP-1, CA-19-9, or EGFr. Elevated or abased individual levels of any of these markers, when analyzed in accordance with the invention, correlate with a decreased chance of patient survival.

In still another preferred embodiment, neoplastic disease predictor values are determined using discrete and combined values corresponding to threshold levels of markers in a marker panel that includes at least one extracellular matrix and matrix metalloproteinase marker and VEGF. A decrease in the individual level of the extracellular matrix marker and an increase in the individual level of the matrix metalloproteinase marker, in the absence of VEGF, correlates with an increased chance of patient survival. Conversely, a decrease in the individual level of the extracellular matrix marker and an increase in the individual level of the matrix metalloproteinase marker, when coupled with detection of VEGF, correlate with a decreased chance of patient survival.

Linear or nonlinear function algorithms used to generate predictor values in connection with methods of the invention can be derived by correlating reference neoplastic disease-related marker data using, e.g., either discriminant function analysis or nonparametric regression analysis. For example, linear or nonlinear function algorithms used in the invention can be derived by:

(a) compiling a data set comprising neoplastic disease-related marker data for a first group of subjects, wherein the marker data includes data related to (1) at least one tumor marker or at least one immune marker or at least one acute phase marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis);

(b) deriving a linear or nonlinear function algorithm from the compiled data set through application of at least one analytical methodology selected from the group consisting of discriminant function analysis, nonparametric regression analysis, classification trees, support vector machines, K-nearest neighbor and shrunken centroids and neural networks;

(c) calculating validation predictor values for a second group of subjects by inputting data comprising neoplastic disease-related marker data for the second group of subjects into the algorithm derived in step (b);

(d) comparing validation predictor values calculated in step (c) with neoplastic disease-related scores for the second group of subjects; and

(e) if the validation predictor values determined in step (c) do not correlate within a clinically-acceptable tolerance level with validation predictor values for the second group of subjects, performing the following operations (i)-(iii) until such tolerance is satisfied: (i) modifying the algorithm on a basis or bases comprising (1) revising the data set for the first group of subjects, and (2) revising or changing the analytical methodology (ii) calculating validation predictor values for the second group of subjects by inputting data comprising neoplastic disease-related marker data for the second group of subjects into the modified algorithm (iii) assessing whether validation predictor values calculated using the modified algorithm correlate with predictor values for the second group of subjects within the clinically-acceptable tolerance level. Analytical methodologies used in the aforementioned derivation may include discriminant function analysis and nonparametric regression analysis, as well as techniques such as classification trees, neural networks, support vector machines, K-nearest neighbor and shrunken centroids.

The invention also provides a data structure stored in a computer-readable medium that may be read by a microprocessor and that comprises at least one code that uniquely identifies an algorithm which generates a predictor value in a manner described herein.

In another embodiment, the invention provides a kit comprising one or more immunoassays comprising monoclonal and/or polyclonal antibodies or any immunoreactive fragments thereof that specifically bind to the marker, wherein the immunoassays detect and determine levels of (1) at least one tumor marker or at least one immune marker or at least one acute phase marker; and (2) at least one marker that is (i) an extracellular matrix (ECM) marker (ii) a marker that is indicative of extracellular matrix synthesis (fibrogenesis); or (iii) a marker that is indicative of extracellular matrix degradation (fibrolysis).

In another embodiment, the invention provides a kit comprising:

(a) a data structure comprising a data field containing data representing an algorithm which generates predictor values that can be used to predict a patient's response to an anti-neoplastic treatment regimen, said data structure being stored in a computer-readable medium that may be read by a microprocessor and that comprises at least one code that uniquely identifies an algorithm which generates a predictor value in a manner described herein; and

(b) one or more immunoassays that detect and determine levels of (1) at least one tumor marker or at least one immune marker; and (2) at least one marker that is (i) an extracellular matrix (ECM) marker (ii) a marker that is indicative of extracellular matrix synthesis (fibrogenesis); or (iii) a marker that is indicative of extracellular matrix degradation (fibrolysis).

In still another embodiment, the invention provides computer-implementable methods and systems for determining whether a composition is useful in the treatment of a neoplastic disease.

In still another embodiment, the invention provides computer-implementable methods and systems useful in making a medical expense decision relating to the treatment of a neoplastic disease.

These and other embodiments of the invention are described further in the detailed description of the invention.

BRIEF DESCRIPTION OF THE FIGURES

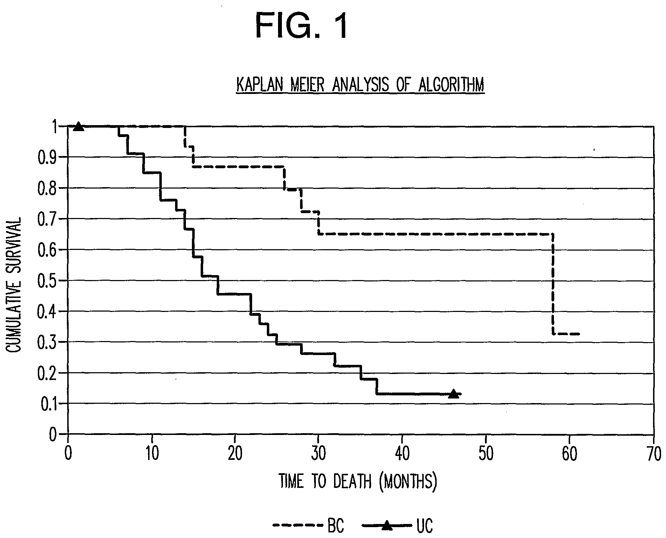

FIG. 1 illustrates Kaplan-Meier survival curves which were generated in connection with the experiment of Example 2 herein.

FIGS. 2 to 8 the results of the single parameter Kaplan Meier Analysis by using the Cut-off values for each of the selected markers as displayed in FIG. 1.

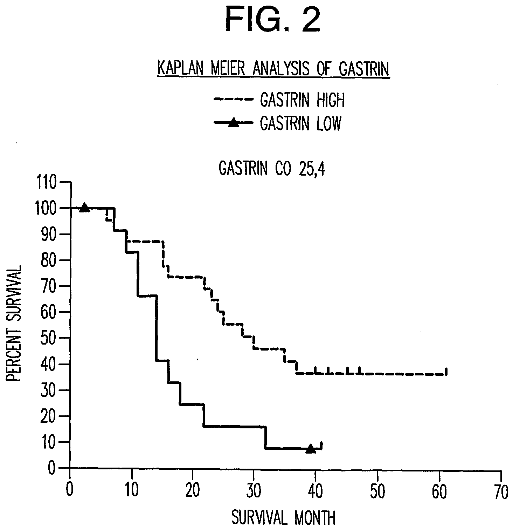

FIG. 2 illustrates a Kaplan Meier Analysis of Gastrin.

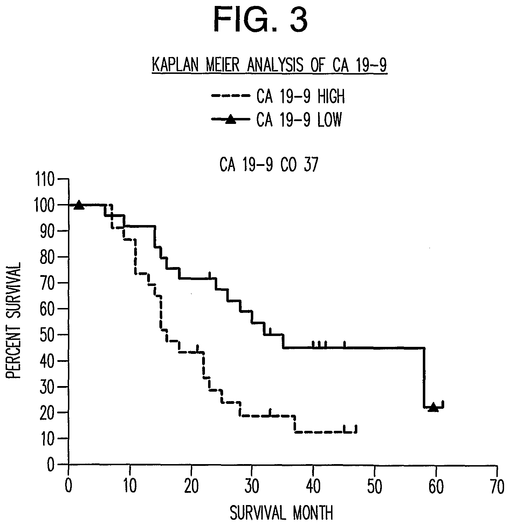

FIG. 3 illustrates a Kaplan Meier Analysis of CA 19-9.

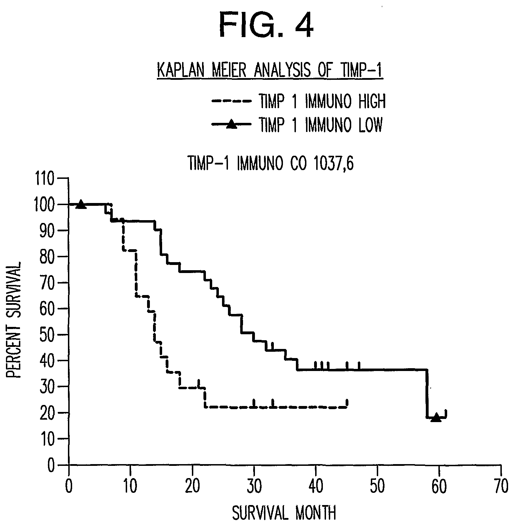

FIG. 4 illustrates a Kaplan Meier Analysis of TIMP-1.

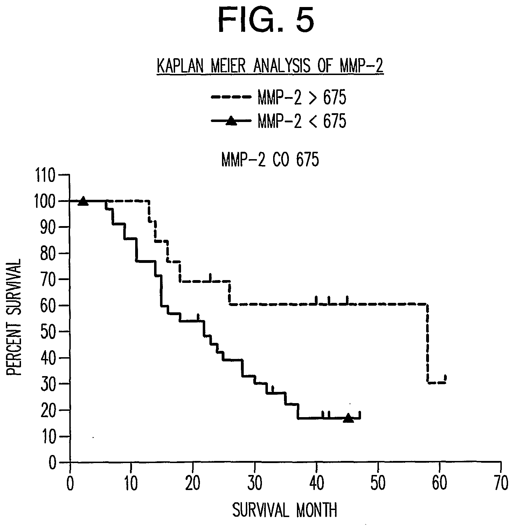

FIG. 5 illustrates a Kaplan Meier Analysis of MMP-2.

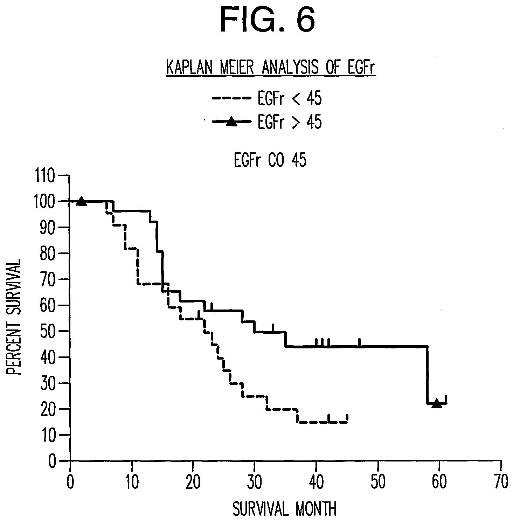

FIG. 6 illustrates a Kaplan Meier Analysis of EGFr.

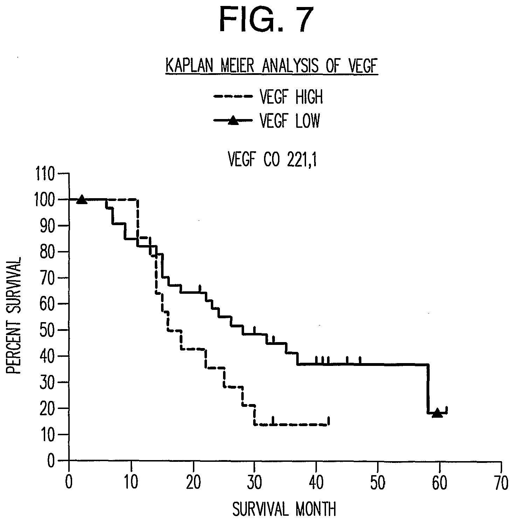

FIG. 7 illustrates a Kaplan Meier Analysis of VEGF.

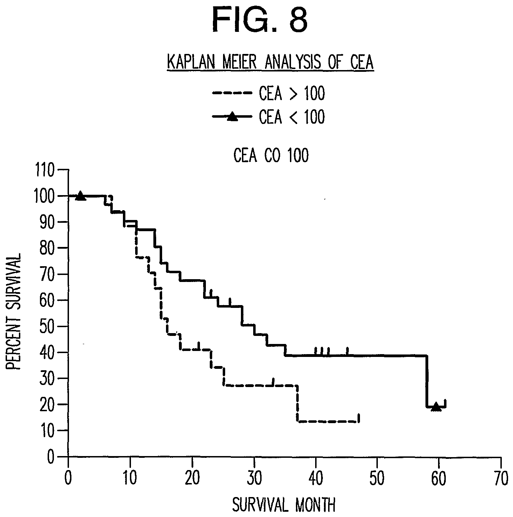

FIG. 8 illustrates a Kaplan Meier Analysis of CEA.

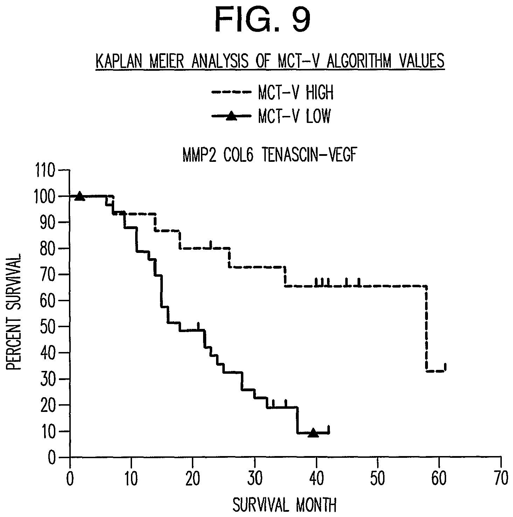

FIG. 9 illustrates a Kaplan Meier Analysis of the respective "MCT-V" algorithm values.

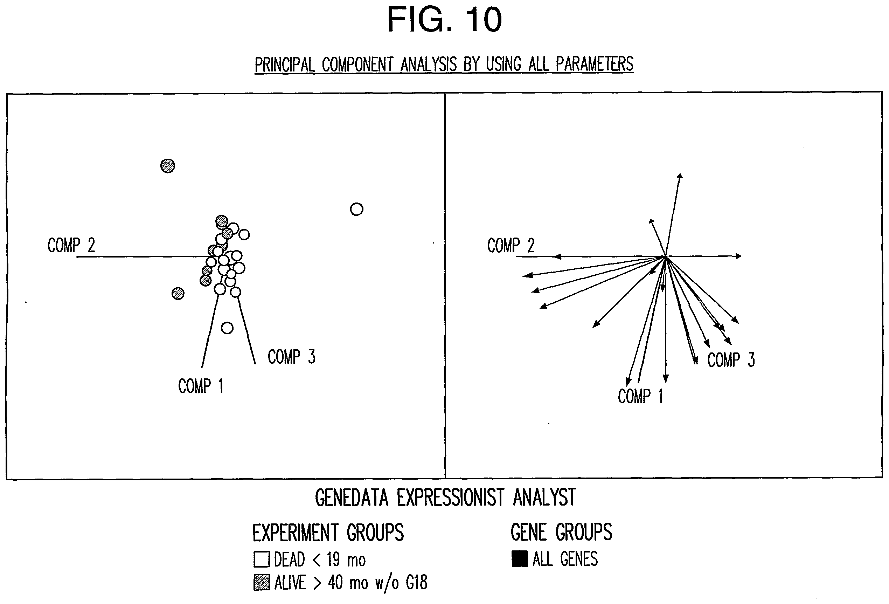

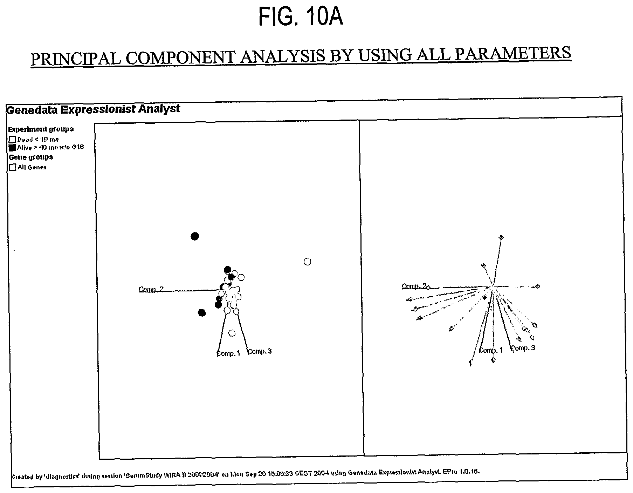

FIG. 10 illustrates the initial partitioning into two groups when using all 17 parameters identified in Tables 4A and 4B.

FIG. 10A illustrates the initial partitioning into two groups when using all 17 parameters identified in Tables 4A and 4B. "Survivors" are displayed as green balls and "non-survivors" are displayed as red balls.

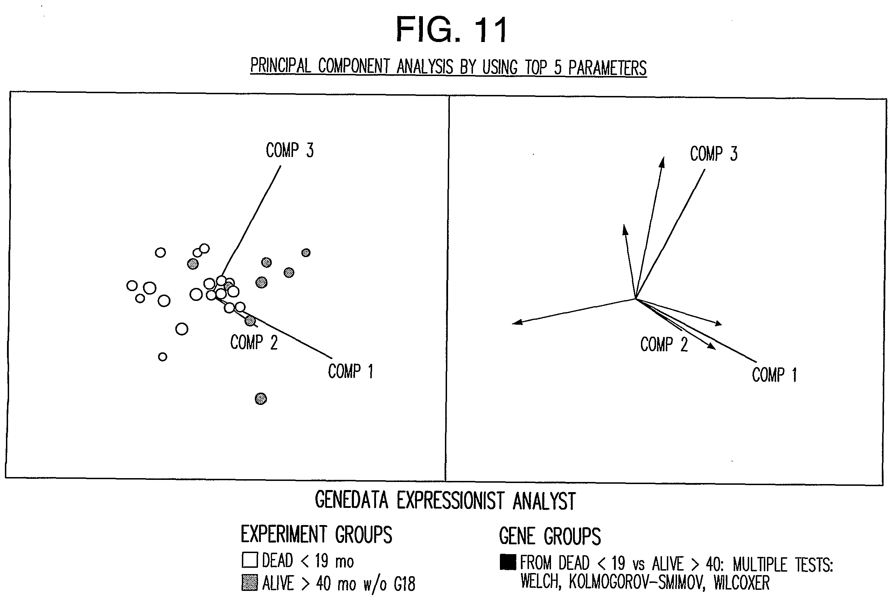

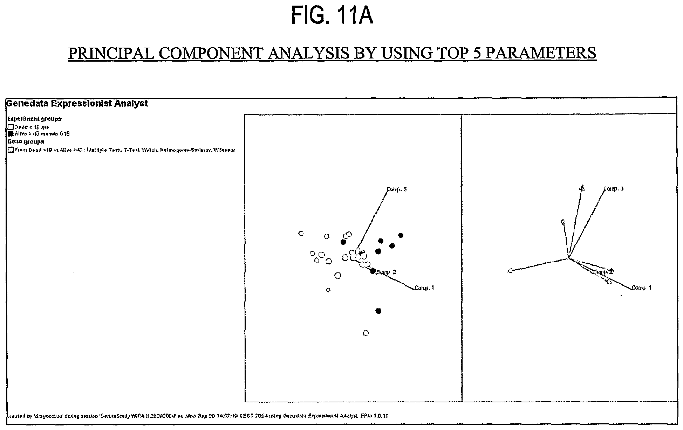

FIG. 11 illustrates the improved partitioning into two groups by Principal Component Analysis (PCA) when using the Top 5 discriminating parameters (i.e. CEA, initial tumor size, Collagen VI, MMP-2 and Gastrin) depicted in Tables 4A and 4B.

FIG. 11A illustrates the improved partitioning into two groups by Principal Component Analysis (PCA) when using the Top 5 discriminating parameters (i.e. CEA, initial tumor size, Collagen VI, MMP-2 and Gastrin) depicted in Tables 4A and 4B. "Survivors" are displayed as green balls and "non-survivors" are displayed as red balls.

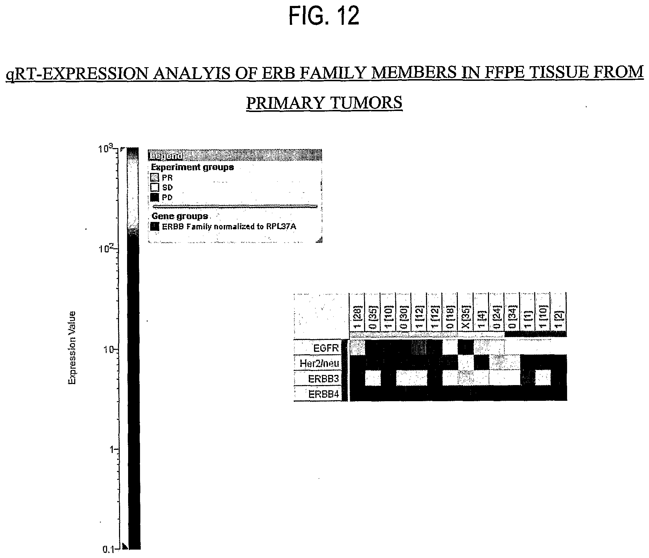

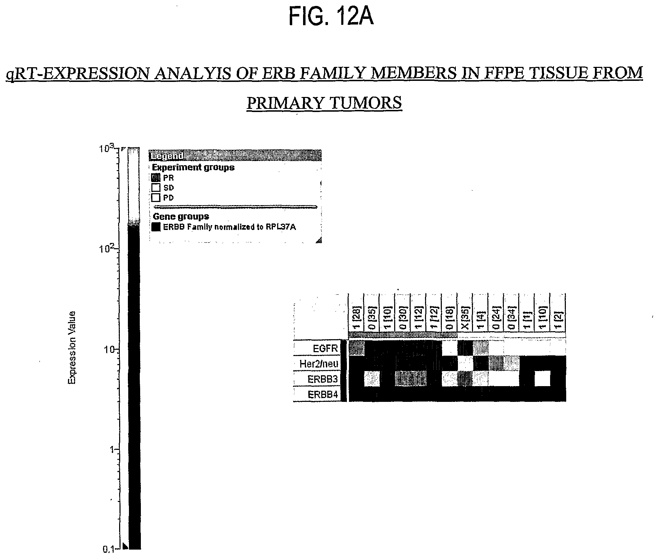

FIG. 12 illustrates the relative expression of the ERB receptor tyrosine kinase family members in FFPE tissues from primary tumor resectates of patients as described in Example 1 and as determined by qRT-PCR profiling.

FIG. 12A illustrates the relative expression of the ERB receptor tyrosine kinase family members in FFPE tissues from primary tumor resectates of patients as described in Example 1 and as determined by qRT-PCR profiling.

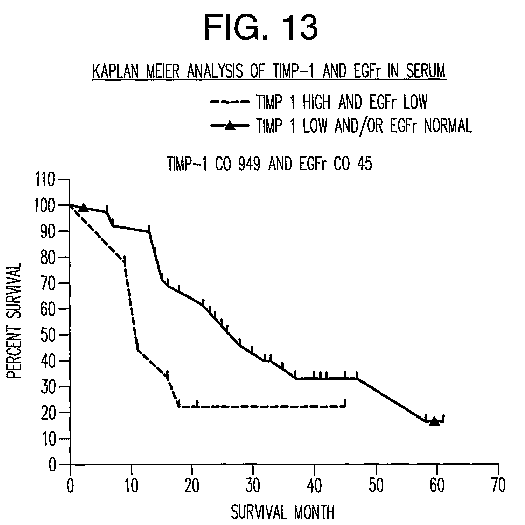

FIG. 13 illustrates Kaplan-Meier survival curves of combined analysis of serum levels of TIMP-1 and EGFr

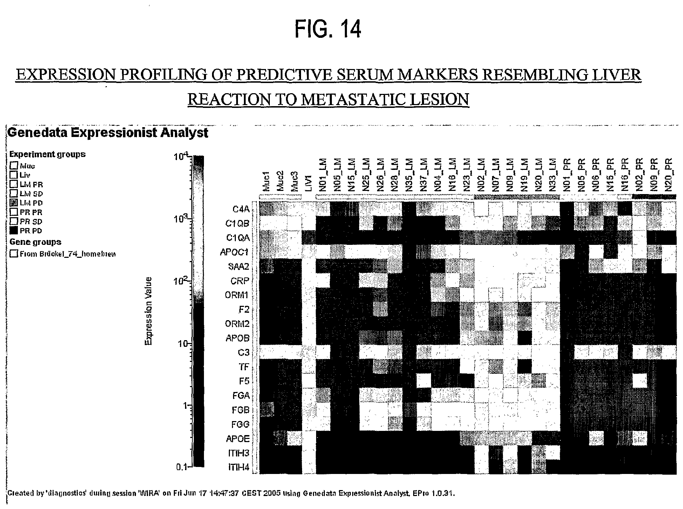

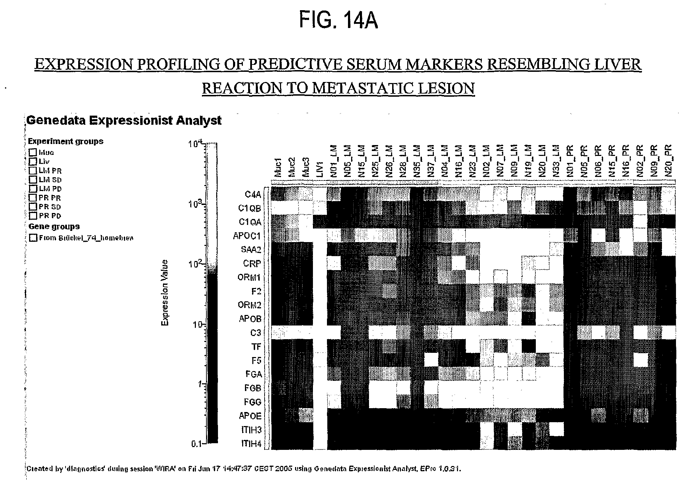

FIGS. 14 and 14A illustrates the relative expression of acute phase , immune markers and co-regulated markers in fresh tumor samples of patients as described in Example 1 and as determined by Affymetrix GeneChip analysis.

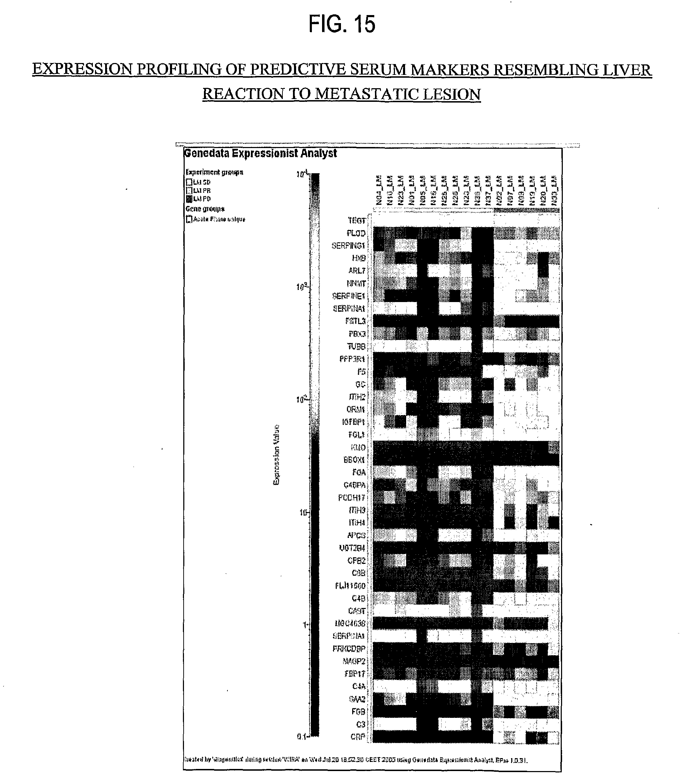

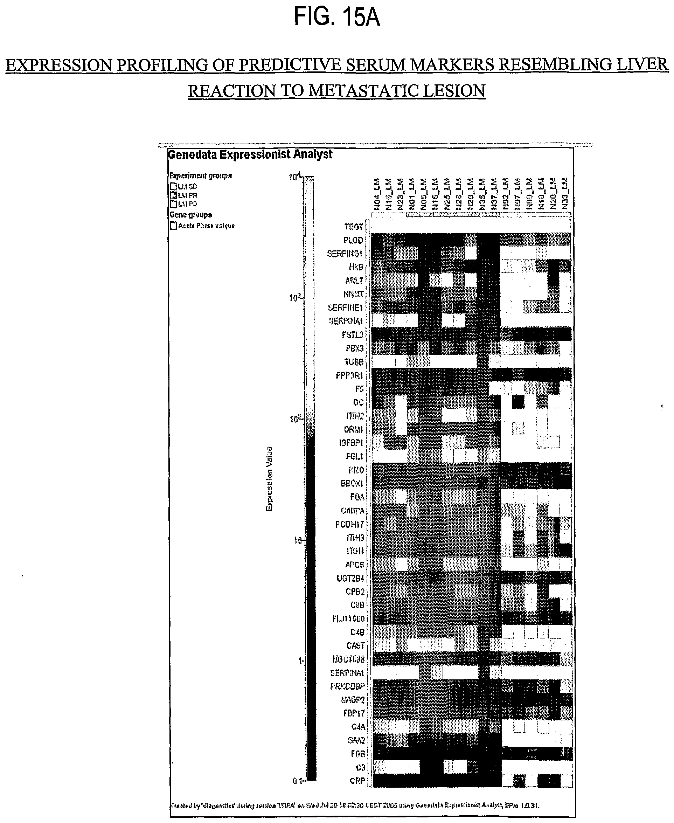

FIGS. 15 and 15A illustrates the relative expression of acute phase , immune markers and co-regulated markers in fresh tumor samples of patients as described in Example 1 and as determined by Affymetrix GeneChip analysis.

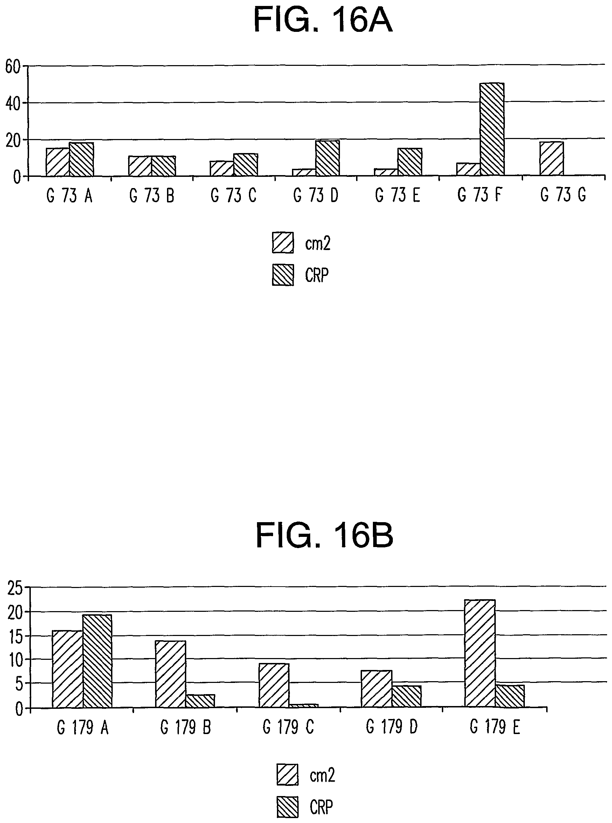

FIGS. 16A and 16B illustrate serial measurements of serum samples of several patients revealed an increase in serum levels of CRP [mg/1] in patients who suffered progression of metastatic disease lateron as depicted by tumor size changes [$cm^2$].

BRIEF DESCRIPTION OF THE TABLES

Table 1 lists the antibodies used to detect the ECM, fibrosis and fibrogenesis marker which were used within this invention.

Table 2 lists representative nucleotide sequences which can be expressed to yield markers which are useful in methods of the invention and which have been used to derive algorithms described within this patent application.

Table 3 lists tumor sizes as adjusted by computer tomography at each therapy cycle to assess tumor response to treatment.

Tables 4A and 4B display experimental data as determined by duplicate or triplicate measurements for each of the 17 indicated markers in the pretreatment serum sample.

Table 5 presents the results of a cox regression analysis using all variables including imputed data.

Tables 6A and 6B list Cox Regression Parameter estimates and ROC coordinates which were determined in accordance with the experiment of Example 2.

Table 7 lists the assessment of the MCT-V Algorithm values.

Table 8 lists a comparison of survival curves illustrated in FIGS. 2 through 8.

Table 9 displays the results of multiple statistical testing to discriminate patients with metastatic CRC surviving for more than 40 month or less than 18 month since primary treatment by assessing serum parameters.

Table 10 displays the results of multiple statistical testing to discriminate patients with metastatic CRC whose metastatic lesions respond to 5' FU based regimen (Partial Response) or do not respond (Stable Disease and Progressive Disease) by determining RNA of EGFR family member in FFPE tissue samples.

Table 11 displays experimental data as determined by duplicate or triplicate measurements for TIMP-1 and EGFr in the pretreatment serum sample and combined analysis thereof.

Table 12 lists representative nucleotide sequences of acute phase, immune markers and markers which can be expressed to yield markers which are useful in methods of the invention.

Table 13 displays expression levels of acute phase and immune markers discriminating between responding and non responding tumors as determined by gene expression profiling by using Affymetrix GeneChip HG U133A.

## DETAILED DESCRIPTION OF THE INVENTION

As used herein, the following terms have the following respective meanings.

"Acute phase markers" include but are not limited to CRP, Ceruloplasmin, Fibrinogen, Haptoglobin, Ferritin, Lipopolysaccharide binding protein (LBP), Procalcitonin, bradykinin, Histamine, Serotonin, Leukotriens (e.g. LTB4), Interleukins Tumor Necrosis Factor alpha and Prealbumin. Acute phase markers indicate inflammatory diseases of diverse origin. Elevated levels of acute phase proteins have been described for colorectal paients. Glojnaric et al. (2001) Clin. Chem. Lab. Med. 2001; Feb. 39 (2) 129-133, showed that colorectal carcinoma caused an increase in serum levels of multiple acute phase reactants. In their study, serum amyloid A protein showed the most powerful reaction in preoperative disease stage, with the mean value of 330 mg/l (range 7-2506 mg/1) as compared to the normal values of less than 1.2 mg/l obtained in 30 healthy adults. Glojnaric describes serum amyloid A protein as showing the best specificity for colorectal carcinoma of all the acute phase proteins studied (83-100%), and also indicate that it has a sensitivity of 100%. A non-exclusive list of exemplary acute phase markers are listed in Table 12.

"Prognostic Markers" as used herein refers to factors that provide information about the clinical outcome of patients with or without treatment. The information provided by prognostic markers is not affected by therapeutic interference.

"Predictive Markers" as used herein refers to factors that provide information about the possible response of a tumor to a distinct therapeutic agent or regimen.

The term "marker" or "biomarker" refers a biological molecule, e.g., a nucleic acid, peptide, hormone, etc., whose presence or concentration can be detected and correlated with a known condition, such as a disease state.

Staging is a method to describe how advanced a cancer is. Staging for colorectal cancer takes into account the depth of invasion into the colon wall, and spread to lymph nodes and other organs. Stage 0 (Carcinoma in Situ): Stage 0 cancer is also called carcinoma in situ. This is a precancerous condition, usually found in a polyp. Stage I (Dukes A): The cancer has spread through the innermost lining of the colon to the second and third layers of the colon wall. It has not spread outside the colon. Stage II (Dukes B): The cancer has spread through the colon wall outside the colon to nearby tissues. Stage III (Dukes C): Cancer has spread to nearby lymph nodes, but not to other parts of the body. Stage IV: Cancer has spread to other parts of the body, e.g. metastasized to the liver or lungs. According to UICC, stages are further subdivided according to T and N.

"Antibody" includes polyclonal or monoclonal antibodies or any fragment thereof. Monoclonal and/or polyclonal antibodies may be used in methods and systems of the invention. "Antibody" or other similar term as used herein includes a whole immunoglobulin that is either monoclonal or polyclonal, as well as immunoreactive fragments that specifically bind to the marker, including Fab, Fab', F(ab')$_2$ and F(v). The term "Antibody" also includes binding-proteins. Preferred serum marker antibodies are described hereinafter.

The human fluid samples used in the assays of the invention can be any samples that contain patient markers, e.g. blood, serum, plasma, urine, sputum or broncho alveolar lavage (BAL) or any other body fluid or stool. Typically a serum or plasma sample is employed.

Antibodies used in the invention can be prepared by techniques generally known in the art, and are typically generated to a sample of the markers - either as an isolated, naturally occurring protein, as a recombinantly expressed protein, or a synthetic peptide representing an antigenic portion of the natural protein. The second antibody is conjugated to a detector group, e.g. alkaline phosphatase, horseradish peroxidase, a fluorescent dye or any other labeling moiety generally useful to detect biomolecules in assays. Conjugates are prepared by techniques generally known in the art.

"Immunoassays" determine the presence of a patient marker in a biological sample by reacting the sample with an antibody that binds to the serum marker, the reaction being carried out for a time and under conditions allowing the formation of an immunocomplex between the antibodies and the serum markers. The quantitative determination of such an immunocomplex is then performed.

In one version, the antibody used is an antibody generated by administering to a mammal (e.g., a rabbit, goat, mouse, pig, etc.) an immunogen that is a serum marker, an immunogenic fragment of a serum marker, or an anti-serum marker-binding idiotypic antibody. Other useful immunoassays feature the use of serum marker-binding antibodies generally (regardless of whether they are raised to one of the immunogens described above). A sandwich immunoassay format may be employed which uses a second antibody that also binds to a serum marker, one of the two antibodies being immobilized and the other being labeled.

Preferred immunoassays detect an immobilized complex between a serum marker and a serum marker-binding antibody using a second antibody that is labeled and binds to the first antibody. Alternatively, the first version features a sandwich format in which the second antibody also binds a serum marker. In the sandwich immunoassay procedures, a serum marker -binding antibody can be a capture antibody attached to an insoluble material and the second a serum marker -binding antibody can be a labeling antibody. The above-described sandwich immunoassay procedures can be used with the antibodies described hereinafter.

The assays used in the invention can be used to determine a blood marker, e.g., a plasma or serum marker in samples including urine, plasma, serum, peritoneal fluid or lymphatic fluid. Immunoassay kits for detecting a serum marker can also be used in the invention, and comprise a serum marker -binding antibody and the means for determining binding of the antibody to a serum marker in a biological sample. In preferred embodiments, the kit includes one of the second antibodies or the competing antigens described above.

"Reference neoplastic disease and blood marker data" and "neoplastic disease data" include but are not limited to serum or plasma data indicative of disease status, but also refers to expression data from tissues or biopsies and the respective expression analysis of said samples. These data comprise protein, peptide, RNA and DNA data. The reference neoplastic disease data refers to cohort of patients with well characterized clinical status and outcome. This enables comparative analysis.

"Validation predictor values" may be calculated by inputing data comprising neoplastic disease-related marker data for a group of subjects into the algorithm in case of incomplete marker determinations.

"Discriminant function analysis" is a technique used to determine which variables discriminate between two or more naturally occurring mutually exclusive groups. The basic idea underlying discriminant function analysis is to determine whether groups differ with regard to a set of predictor variables which may or may not be independent of each other, and then to use those variables to predict group membership (e.g., of new cases).

Discriminant function analysis starts with an outcome variable that is categorical (two or more mutually exclusive levels). The model assumes that these levels can be discriminated by a set of predictor variables which, like ANOVA (analysis of variance), can be continuous or categorical (but are preferably continuous) and, like ANOVA assumes that the underlying discriminant functions are linear. Discriminant analysis does not "partition variation". It does look for canonical correlations among the set of predictor variables and uses these correlates to build eigenfunctions that explain percentages of the total variation of all predictor variables over all levels of the outcome variable.

The output of the analysis is a set of linear discriminant functions (eigenfunctions) that use combinations of the predictor variables to generate a "discriminant score" regardless of the level of the outcome variable. The percentage of total variation is presented for each function. In addition, for each eigenfunction, a set of Fisher Discriminant Functions are developed that produce a discriminant score based on combinations of the predictor variables within each level of the outcome variable.

Usually, several variables are included in a study in order to see which variable contribute to the discrimination between groups. In that case, a matrix of total variances and co-variances is generated. Similarly, a matrix of pooled within-group variances and co-variances may be generated. A comparison of those two matrices via multivariate F tests is made in order to determine whether or not there are any significant differences (with regard to all variables) between groups. This procedure is identical to multivariate analysis of variance or MANOVA. As in MANOVA, one could first perform the multivariate test, and, if statistically significant, proceed to see which of the variables have significantly different means across the groups.

For a set of observations containing one or more quantitative variables and a classification variable defining groups of observations, the discrimination procedure develops a discriminant criterion to classify each observation into one of the groups. In order to get an idea of how well a discriminant criterion "performs", it is necessary to classify (*a priori*) different cases, that is, cases that were not used to estimate the discriminant criterion. Only the classification of new cases enables an assessment of the predictive validity of the discriminant criterion.

In order to validate the derived criterion, the classification can be applied to other data sets. The data set used to derive the discriminant criterion is called the training or calibration data set or patient training cohort. The data set used to validate the performance of the discriminant criteria is called the validation data set or validation cohort.

The discriminant criterion (function(s) or algorithm), determines a measure of generalized squared distance. These distances are based on the pooled co-variance matrix. Either Mahalanobis or Euclidean distance can be used to determine proximity. These distances can be used to identify groupings of the outcome levels and so determine a possible reduction of levels for the variable.

A "pooled co-variance matrix" is a numerical matrix formed by adding together the components of the covariance matrix for each subpopulation in an analysis.

A "predictor" is any variable that may be applied to a function to generate a dependent or response variable or a "predictor value". In one embodiment of the instant invention, a predictor value may be a discriminant score determined through discriminant function analysis of two or more patient blood markers (e.g., plasma or serum markers). For example, a linear model specifies the (linear) relationship between a dependent (or response) variable $Y$, and a set of predictor variables, the $X$'s, so that

$$Y = b_0 + b_1X_1 + b_2X_2 + ... + b_kX_k$$

In this equation $b_0$ is the regression coefficient for the intercept and the $b_i$ values are the regression coefficients (for variables 1 through k) computed from the data.

"Classification trees" are used to predict membership of cases or objects in the classes of a categorical dependent variable from their measurements on one or more predictor variables. Classification tree analysis is one of the main techniques used in so-called Data Mining. The goal of classification trees is to predict or explain responses on a categorical dependent variable, and as such, the available techniques have much in common with the techniques used in the more traditional methods of Discriminant Analysis, Cluster Analysis, Nonparametric Statistics, and Nonlinear Estimation.

The flexibility of classification trees makes them a very attractive analysis option, but this is not to say that their use is recommended to the exclusion of more traditional methods. Indeed, when the typically more stringent theoretical and distributional assumptions of more traditional methods are met, the traditional methods may be preferable. But as an exploratory technique, or as a technique of last resort when traditional methods fail, classification trees are, in the opinion of many researchers, unsurpassed. Classification trees are widely used in applied fields as diverse as medicine (diagnosis), computer science (data structures), botany (classification), and psychology (decision theory). Classification trees readily lend themselves to being displayed graphically, helping to make them easier to interpret than they would be if only a strict numerical interpretation were possible.

"Neural Networks" are analytic techniques modeled after the (hypothesized) processes of learning in the cognitive system and the neurological functions of the brain and capable of predicting new observations (on specific variables) from other observations (on the same or other variables) after executing a process of so-called learning from existing data. Neural Networks is one of the Data Mining techniques. The first step is to design a specific network architecture (that includes a specific number of "layers" each consisting of a certain number of "neurons"). The size and structure of the network needs to match the nature (e.g., the formal complexity) of the investigated phenomenon. Because the latter is obviously not known very well at this early stage, this task is not easy and often involves multiple "trials and errors."

The neural network is then subjected to the process of "training." In that phase, computer memory acts as neurons that apply an iterative process to the number of inputs (variables) to adjust the weights of the network in order to optimally predict the sample data on which the "training" is performed. After the phase of learning from an existing data set, the new network is ready and it can then be used to generate predictions.

In one embodiment of the invention, neural networks can comprise memories of one or more personal or mainframe computers or computerized point of care device.

"Cox Regression Analysis" is a statistical technique whereby Cox proportional-hazards regression is used to analyze the effect of several risk factors on survival. The probability of the endpoint (death, or any other event of interest, e.g. recurrence of disease) is called the hazard. The hazard is modeled as:

$$H(t) = H_0(t) \times \exp(b_1 X_1 + b_2 X_2 + b_3 X_3 + \ldots + b_k X_k)$$

where $X_1 \ldots X_k$ are a collection of predictor variables and $H_0(t)$ is the baseline hazard at time t, representing the hazard for a person with the value 0 for all the predictor variables.

By dividing both sides of the above equation by $H_0(t)$ and taking logarithms, we obtain:

$$\ln\left(\frac{H(t)}{H_0(t)}\right) = b_1 X_1 + b_2 X_2 + b_3 X_3 + \ldots + b_k X_k$$

$H(t) / H_0(t)$ is the hazard ratio. The coefficients $b_i \ldots b_k$ are estimated by Cox regression, and can be interpreted in a similar manner to that of multiple logistic regression.

If the covariate (risk factor) is dichotomous and is coded 1 if present and 0 if absent, then the quantity $\exp(b_i)$ can be interpreted as the instantaneous relative risk of an event, at any time, for an individual with the risk factor present compared with an individual with the risk factor absent, given both individuals are the same on all other covariates. If the covariate is continuous, then the quantity $\exp(b_i)$ is the instantaneous relative risk of an event, at any time, for an individual with an increase of 1 in the value of the covariate compared with another individual, given both individuals are the same on all other covariates.

"Kaplan Meier curves" are a nonparametric (actuarial) technique for estimating time-related events (the survivorship function). 1 Ordinarily, Kaplan Meier curves are used to analyze death as an outcome. It may be used effectively to analyze time to an endpoint, such as remission. Kaplan Meier curves are a univariate analysis, an appropriate starting technique, and estimate the probability of the proportion of individuals in remission at a particular time, starting from the initiation of active date (time zero), is especially applicable when length of follow-up varies from patient to patient, and takes into account those patients lost during follow-up or not yet in remission at end of a clinical study (e.g., censored patients, where the censoring is non-informative). Kaplan Meier is therefore useful in evaluating remissions following loosing a patient. Since the estimated survival distribution for the cohort study has some degree of uncertainty, 95% confidence intervals may be calculated for each survival probability on the "estimated" curve.

A variety of tests (log-rank, Wilcoxan and Gehen) may be used to compare two or more Kaplan-Meier "curves" under certain well-defined circumstances. Median remission time (the time when 50% of the cohort has reached remission), as well as quantities such as three, five, and ten year probability of remission, can also be generated from the Kaplan-Meier analysis, provided there has been sufficient follow-up of patients.

Kaplan-Meier and Cox regression analysis can be performed by using commercially available software packages, e.g., Graph Pad Prism™ and SPSS version11.

"Computer" refers to a combination of a particular computer hardware system and a particular software operating system. A computer or computerized system of the invention can comprise handheld calculator. Examples of useful hardware systems include those with any type of suitable data processor. The term "computer" also includes, but is not limited to, personal computers (PC) having an operating system such as DOS, Windows®, OS/2 ® or Linux®; Macintosh ® computers; computers having JAVA®-OS as the operating system; and graphical workstations such as the computers of Sun Microsystems® and Silicon Graphics ®, and other computers having some version of the UNIX operating system such as AIX® or SOLARIS® of Sun Microsystems®; embedded computers executing a control scheduler as a thin version of an operating system, a handheld device; any other device featuring known and available operating system; as well as any type of device which has a data processor of some type with an associated memory.

While the invention will be described in the general context of computer-executable instructions of a computer program that runs on a personal computer, those skilled in the art will recognize that the invention also may be implemented in combination with other program modules. Generally, program modules include routines, programs, components, and data structures that perform particular tasks or implement particular abstract data types. Moreover, those skilled in the art will appreciate that the invention may be practiced with other computer system configurations, including hand-held devices, multi-processor systems, microprocessor-based or programmable consumer electronics, minicomputers, mainframe computers, and the like. The invention may also be practiced in distributed computing environments where tasks are performed by remote processing devices that are linked through a communications network. In a distributed computing environment, program modules may be located in both local and remote memory storage devices.

A purely illustrative system for implementing the invention includes a conventional personal computer, including a processing unit, a system memory, and a system bus that couples various system components including the system memory to the processing unit. The system bus may be any of several types of bus structure including a memory bus or memory controller, a peripheral bus, and a local bus using any of a variety of conventional bus architectures such as PCI, VESA, Microchannel, ISA and EISA, to name a few. The system memory includes a read only memory (ROM) and random access memory (RAM). A basic input/output system (BIOS), containing the basic routines that helps to transfer information between elements within the personal computer, such as during start-up, is stored in ROM.

The personal computer further includes a hard disk drive, a magnetic disk drive, e.g., to read from or write to a removable disk, and an optical disk drive, e.g., for reading a CD-ROM disk or to read from or write to other optical media. The hard disk drive, magnetic disk drive, and optical disk drive are connected to the system bus by a hard disk drive interface, a magnetic disk drive interface, and an optical drive interface, respectively. The drives and their associated computer-readable media provide nonvolatile storage of data, data structure, computer-executable instructions, etc. for the personal computer. Although the description of computer-readable media above refers to a hard disk, a removable magnetic disk and a CD, it should be appreciated by those skilled in the art that other types of media which are readable by computer, such as magnetic cassettes, flash memory card, digital video disks, Bernoulli cartridges, and the like, may also be used in the exemplary operating environment.

A number of program modules may be stored in the drive's RAM, including an operating system, one or more application programs, other program modules, and program data. A user may enter commands and information into the personal computer through a keyboard and a pointing device, such as a mouse. Other input devices may include a microphone, joystick, game pad, satellite dish, scanner, or the like. These and other input devices are often connected to the processing unit through a serial port interface that is coupled to the system bus, but may be connected by other interfaces, such as a parallel port, game port or a universal serial bus (USB). A monitor or other type of display device is also connected to the system bus via an interface, such as a video adapter. In addition to the monitor, personal computers typically include other peripheral output devices (not shown), such as speakers and printers.

The personal computer may operate in a networked environment using logical connections to one or more remote computers, such as a remote computer. The remote computer may be a server, a router, a peer device or other common network node, and typically includes many or all of the elements described relative to the personal computer. Logical connections include a local area network (LAN) and a wide area network (WAN). Such networking environments are commonplace in offices, enterprise-wide computer networks (such as hospital computers), intranets and the Internet.

When used in a LAN networking environment, the personal computer can be connected to the local network through a network interface or adapter. When used in a WAN networking environment, the personal computer typically includes a modem or other means for establishing communications over the wide area network, such as the Internet. The modem, which may be internal or external, is connected to the system bus via the serial port interface. In a networked environment, program modules depicted relative to the personal computer, or portions thereof, may be stored in the remote memory storage device. It will be appreciated that the network connections shown are exemplary and other means of establishing a communications link between the computers may be used.

One purely illustrative implementation platform of the present invention is a system implemented on an IBM compatible personal computer having at least eight megabytes of main memory and a gigabyte hard disk drive, with Microsoft Windows as the user interface and any variety of data base management software including Paradox. The application software implementing predictive functions can be written in any variety of languages, including but not limited to C++, and is stored on computer readable media as defined hereinafter. A user enters commands and information reflecting patient markers into the personal computer through a keyboard and a pointing device, such as a mouse.

In a preferred embodiment, the invention provides a data structure stored in a computer-readable medium, to be read by a microprocessor comprising at least one code that uniquely identifies predictor functions and values derived as described hereinafter. Examples of preferred computer usable media include: nonvolatile, hard-coded type mediums such as read only memories (ROMs) or erasable, electrically programmable read only memories (EEPROMs), recordable type mediums such as floppy disks, hard disk drives and CD-ROMs, and transmission type media such as digital and analog communication links.

A "data structure" can include a collection of related data elements, together with a set of operations which reflect the relationships among the elements. A data structure can be considered to reflect the organization of data and its storage allocation within a device such as a computer.

Thus, a data structure may comprise an organization of information, usually in memory, for better algorithm efficiency, such as queue, stack, linked list, heap, dictionary, and tree, or conceptual unity, such as the name and address of a person. It may include redundant information, such as length of the list or number of nodes in a subtree. A data structure may be an external data structure, which is efficient even when accessing most of the data is very slow, such as on a disk. A data structure can be a passive data structure which is only changed by external threads or processes, in contrast to an active data structure. An active or functional data structure has an associated thread or process that performs internal operations to give the external behavior of another, usually more general, data structure. A data structure also can be a persistent data structure that preserves its old versions, that is, previous versions may be queried in addition to the latest version. A data structure can be a recursive data structure that is partially composed of smaller or simpler instances of the same data structure. A data structure can also be an abstract data type, i.e., set of data values and associated operations that are precisely specified independent of any particular implementation.

These examples of data structures, as with all exemplified embodiments herein, are illustrative only and are in no way limiting.

A system of the invention may comprise a handheld device useful in point of care applications or may be a system that operates remotely from the point of patient care. In either case the system can include companion software programmed in any useful language to implement methods of the invention in accordance with algorithms or other analytical techniques described herein.

"Point of care testing" refers to real time predictive testing that can be done in a rapid time frame so that the resulting test is performed faster than comparable tests that do not employ this system. Point of care testing can be performed rapidly and on site, such as in a doctor's office, at a bedside, in a stat laboratory, emergency room or other such locales, particularly where rapid and accurate results are required. The patient can be present, but such presence is not required. Point of care includes, but is not limited to: emergency rooms, operating rooms, hospital laboratories and other clinical laboratories, doctor's offices, in the field, or in any situation in which a rapid and accurate result is desired.

The term "patient" refers to an animal, preferably a mammal, and most preferably a human.

A "health care provider" or "health care decision maker" comprises any individual authorized to diagnose or treat a patient, or to assist in the diagnosis or treatment of a patient. In the context of identifying useful new drugs to treat liver disease, a health care provider can be an individual who is not authorized to diagnose or treat a patient, or to assist in the diagnosis or treatment of a patient.

"Tumor markers", "immune markers", "acute phase markers", "extracellular matrix (ECM) markers", "markers that are indicative of extracellular matrix synthesis (fibrogenesis)", and "markers that are indicative of extracellular matrix degradation (fibrolysis)" are referred to herein collectively as "markers", "neoplastic-disease-related markers", and "cancer associated markers". These markers: (1) include, e.g., a nucleic acid, peptide, protein, or gene fragment that can be detected and correlated with a known condition (such as a disease status); and (2) "blood markers" and "blood markers, e.g., plasma and serum markers". As used herein, markers include nucleic acids, peptides, proteins, fragments of polypeptides, or nucleic acid sequence which exhibit an over- or under-expression in a subject suffering from cancer of at least around 10% in cancer cells, in non-cancerous stroma cells, in tissue, or in serum obtained from an individual suffering from cancer, when compared to levels of comparable markers obtained from a subject that either does not suffer from cancer or who suffers from a more or less advanced cancer .

One example of a marker panel used in methods of the invention includes:

(1) at least one marker selected from the group consisting of tumor markers, immune markers, and acute phase markers, including but not limited to CEA, CA15-3, CA19-9, members of the EGFR superfamily (e.g., EGFr, HER-2/neu, HER-3 and HER-4), ERBB3, ERBB4, c-Kit, KDR, FLT4, FLT3, c-Met, members of the FGFR superfamily (FGFR1, FGFR2, FGFR3, FGFR4), members of the FGFR ligand family (e.g., FGF-1, FGF-2, FGF-3, FGF-4, FGF-5, FGF-6, FGF-7and FGF-9 and related splice variants), members of the growth factor family (such as VEGR and VEGF alpha), members of the VEGFR superfamily, e.g., KDR, FLT4, FLT3, members of the VEGFR ligand family including VEGFA, VEGFB, VEGFC and VEGFD, shedded domains of members of growth factors (including family members such as VEGF-A, VEGF-B, VEGF-C (preferably VEGF alpha isoforms such as VEGF189, VEGF165, VEGF121, etc.), and VEGFC, hormones (such as Gastrin), interleukin receptors (such as IL2R), interleukins (such as IL6), complement factors, acute phase proteins (such as CRP; ORM1, ORM2, serum amyloid A2, amyloid P component); and

(2) at least one marker that is:

(i) an extracellular matrix (ECM) marker selected from the group consisting of collagens, basal adhesion proteins (fibronectins, laminins), entactin, proteoglycans, and glycosaminoglycans such as PIIINP, members of the collagen superfamily, e.g., Collagen I, Collagen II, Collagen III, Collagen IV, Collagen V,Collagen VI, Collagen V, Collagen VI, Collagen VII, Collagen VIII, Collagen IX, Collagen X, Collagen XI, Collagen XII, and Tenascin, Laminin, HA; or

(ii) a marker that is indicative of extracellular matrix synthesis (fibrogenesis) selected from the group consisting of preforms of collagens, basal adhesion proteins (fibronectins, laminins), entactin, proteoglycans, and glycosaminoglycans or prepro-peptides thereof such as PIIINP, Collagen IV, Collagen VI, Tenascin, Laminin, Hyaluron (HA); or

(iii) a marker that is indicative of extracellular matrix degradation (fibrolysis) selected from the group consisting of the MMP superfamily (including MMP-1, MMP-2, MMP-3, MMP-7, MMP-8, MMP-9, MMP-12, MMP-13, MMP-14, MMP-15, MMP-16, MMP-17, MMP-19, MMP-20, MMP-24 and MMP-26, preferably MMP-2, MMP-3, MMP-7, MMP-9, MMP-12, MMP-24 and MMP-26); MMP-9/TIMP-1 complex ,or associated inhibitors thereof such as TIMP-1, TIMP-2, TIMP-3, and TIMP-4 .

One example of a marker panel used in methods of the invention includes the combination of:

(1) at least one marker selected from the group consisting of tumor markers, including but not limited to CEA, CA15-3, CA19-9, members of the EGFR superfamily (e.g., EGFr, HER-2/neu, HER-3 and HER-4), ERBB3, ERBB4, c-Kit, KDR, FLT4, FLT3, c-Met, members of the FGFR superfamily (FGFR1, FGFR2, FGFR3, FGFR4), members of the FGFR ligand family (e.g., FGF-1, FGF-2, FGF-3, FGF-4, FGF-5, FGF-6, FGF-7and FGF-9 and related splice variants), members of the growth factor family (such as VEGR and VEGF alpha), members of the VEGFR superfamily, e.g., KDR, FLT4, FLT3, members of the VEGFR ligand family including VEGFA, VEGFB, VEGFC and VEGFD, shedded domains of members of growth factors (including family members such as VEGF-A, VEGF-B, VEGF-C (preferably VEGF alpha isoforms such as VEGF189, VEGF165, VEGF121, etc.), and VEGFC, hormones (such as Gastrin); and/or

(2) at least one marker selected from the group consisting of immune markers including but not limited interleukin receptors (such as IL2R), interleukins (such as IL6), complement factors; and/or

(3) at least one marker selected from the group consisting of acute phase markers including but not limited to acute phase proteins (such as CRP; ORM1, ORM2, serum amyloid A2, amyloid P component) and coregulated genes (APOB, APOC1, APOE, C1QA, C1QB, C3, C4A, CRP, F2, F5, FGA, FGB, FGG, ITIH3, ITIH4, TF, ARL7, BBOX1, C4B, C4BPA, C8B, CAST, CPB2, FBP17, FGL1, FLJ11560, FSTL3, GC, HXB, IGFBP1, ITIH2, KMO, MAGP2, MGC4638, NNMT, PBX3, PCDH17, PLOD, PPP3R1, PRKCDBP, SERPINA1, SERPINE1, SERPING1, TEGT, TUBB, UGT2B4); and/or

(4) at least one marker that is:

(i) an extracellular matrix (ECM) marker selected from the group consisting of collagens, basal adhesion proteins (fibronectins, laminins), entactin, proteoglycans, and glycosaminoglycans such as PIIINP, members of the collagen superfamily, e.g., Collagen I, Collagen II, Collagen III, Collagen IV, Collagen V,Collagen VI, Collagen V, Collagen VI, Collagen VII, Collagen VIII, Collagen IX, Collagen X, Collagen XI, Collagen XII, and Tenascin, Laminin, HA; or

(ii) a marker that is indicative of extracellular matrix synthesis (fibrogenesis) selected from the group consisting of preforms of collagens, basal adhesion proteins (fibronectins, laminins), entactin, proteoglycans, and glycosaminoglycans or prepro-peptides thereof such as PIIINP, Collagen IV, Collagen VI, Tenascin, Laminin, Hyaluron (HA); or

(iii) a marker that is indicative of extracellular matrix degradation (fibrolysis) selected from the group consisting of the MMP superfamily (including MMP-1, MMP-2, MMP-3, MMP-7, MMP-8, MMP-9, MMP-12, MMP-13, MMP-14, MMP-15, MMP-16, MMP-17, MMP-19, MMP-20, MMP-24 and MMP-26, preferably MMP-2, MMP-3, MMP-7, MMP-9, MMP-12, MMP-24 and MMP-26); MMP-9/TIMP-1 complex ,or associated inhibitors thereof such as TIMP-1, TIMP-2, TIMP-3, and TIMP-4 .

Preferably, the panel includes at least two markers, and more preferably three markers, with each marker being from a different set and different from each other.
Preferred marker panels used in methods of the invention include:

(1) at least one marker selected from the group consisting of serum tumor markers, serum immune markers, and acute phase markers including but not limited to: CEA, CA15-3, CA19-9, members EGFr, HER-2/neu, VEGF alpha, Gastrin, IL2R, IL6, CRP, ORM1, ORM2, serum amyloid A2 (SAA2), amyloid P component, C4A, C1QB, C1QA, APOC1, F2, APOB, C3, TF, F5, FGA, FGB, FGG, APOE, ITIH3, ITIH4; and
(2) at least one marker that is (i) a liver ECM marker selected from the group consisting of PIIINP, Collagen IV, Collagen VI, Tenascin, Laminin, HA (ii) a marker that is indicative of liver fibrogenesis selected from the group consisting of prepro-peptides thereof such as PIIINP, Collagen IV, Collagen VI, Tenascin, Laminin, HA, or (iii) a marker that is indicative of liver fibrolysis selected from the group consisting of MMP-2, MMP-3, MMP-7, MMP-9, MMP-12, MMP-24, MMP-9/TIMP-1, and uPA.

The expression of MMP-7 and MMP-12 is pronounced in colorectal cancer and, if determined on a RNA-level, correlates with negative outcome.

A "comparative data set" can comprise any data reflecting any qualitative or quantitative indicia of a neoplastic disease. In one embodiment, the comparative data set can comprise one or more numerical values, or range of numerical values, associated with decreases and elevations in levels (1) of at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

Comparative data set marker levels are typically determined by comparison to normal (healthy) or threshold levels of markers in subjects comprising reference cohorts.

For example, the normal range of TIMP-1 in sera is between about 424 to about 1037 ng/ml. The normal range of Collagen VI in sera is between about 1.2 and about 7.2 ng/ml. The normal range of HA in sera is between about 5.4 to about 34.7 ng/ml. The normal range of Laminin in sera is between about 6.3 to about 3.7 ng/ml. The normal range of MMP-2 in sera of all ages is between about 388 to about 1051 ng/ml (mean 668 ng/ml; median 647 ng/1). The normal range of MMP-9 in sera of all ages is between about 201.6 to about 1545 ng/ml (mean 719 ng/ml; median 683ng/l). The normal range of PIIINP in sera of all ages is between about 0.9 to about 25.6 ng/ml (mean 5.84 ng/ml). The normal range of Tenascin in sera of all ages is between about 206.9 to about 1083.2 ng/ml (mean 455 ng/ml). The normal range of Collagen IV in sera of all ages is between about 66 and about 315 ng/ml (mean 183 ng/ml). The normal range of HER-2/neu in sera is less than about 15 ng/ml.

Normal (healthy) or threshold levels are less that around 163 pg/ml of VEGF165, (95% fall below), less than around 5ng/ml for CEA, less than around 20 U/ml for CA 15-3, less than around 28-115 $\mu$E/ml for Gastrin, less than around 15ng/ml for shedded Her-2/neu, and above around 45 ng/ml for EGFr

A decreased EGFR level is one which is less than the normal or threshold range of EGFR, i.e., around 45-78 ng/ml. Similarly, an increased TIMP-1 level is one which is greater than normal TIMP-1 levels of less than around 1037 ng/ml (Immuno1-Format). An increased HER-2/neu level is one which is greater than normal HER-2/neu levels of less than around 15 ng/ml. Similarly, an increased CEA level is one which is greater than the disease-adjusted CEA level of around 499 ng/ml, while the normal CEA level is around 5ng/ml.

In particular, shorter time to progression and shorter overall survival are found in patients with metastatic colorectal cancer who have EGFR levels that are less than the control range of about 45-78 ng/ml, low levels of Tenascin below a cutoff range of about 1083 ng/ml and/or low levels of Collagen VI below a cutoff range of about 7,2 ng/ml combined with elevated HER-2/neu levels, wherein elevated refers to levels that are greater than the control value of about less than about 15 ng/ml, TIMP-1 levels above the cutoff range of about 1037 ng/ml (Immuno-Format) or above about 250ng/ml (ELISA-Format), elevated levels of VEGF165 above a cutoff range of about 221 pg/ml and /or Gastrin levels above about 25.4 pg/ml.

A comparative data set which relates to altered serum levels of tumor markers indicative of cancerous disease may include or identify a combination of elevated serum HER-2/neu levels (e.g., greater than the normal level of less than around 15 ng/ml) and/or decreased EGFR ECD levels (e.g., less than the normal range of around 45-78 ng/ml) and/or high levels of VEGF (e.g. for the VEGFA isoform 165, greater than the normal level of less than around 221 pg/ml), as values indicative of a shorter time to progression and shorter overall survival time.

"Supplementary markers" include but are not limited to patient weight, sex, age and expression profiling data of fresh and fixed tumor tissue.

- Preferably, markers are obtained from a body fluid sample or a tissue sample. Suitable body fluids include, but are not limited to, pleural fluid samples, pulmonary or bronchial lavage fluid samples, synovial fluid samples, peritoneal fluid samples, stool, bone marrow aspirate samples, lymph, cerebrospinal fluid, ascites fluid samples, amniotic fluid samples, sputum samples, bladder washes, semen, urine, saliva, tears, blood and blood components serum and plasma, and the like. Serum is a preferred body fluid sample. Suitable tissue samples also include various types of tumor or cancer tissue, or organ tissue, such as those taken at biopsy.

"One or more numerical values, or range of numerical values that are associated with a neoplastic disease.

"Predicting a clinical outcome related to a patient suffering from or at risk of developing a neoplastic disease " has been defined previously.

"Respond to one or more neoplastic disease treatment regimens" has been defined previously.

"Making a medical expense decision relating to the treatment of a neoplastic disease" includes but is not limited to a decision by an insurer relating to either reimbursement for a neoplastic disease treatment regimen or an assessment of insurance rates or other charges or payments.

The invention provides computer-implementable methods and systems for determining whether a composition is useful in the treatment of a neoplastic disease. For example, one or more compounds are administered to one or subjects (preferably mammals, and most preferably humans) suffering from a neoplastic disease and the subject's response to the neoplastic disease treatment regimen is used to assess the efficacy of the compound as an anti-neoplastic disease agent.

The term "neoplastic disease" is used to describe the pathological process that results in the formation and growth of a neoplasm, i.e., an abnormal tissue that grows by cellular proliferation more rapidly than normal tissue and continues to grow after the stimuli that initiated the new growth cease. Neoplastic diseases exhibit partial or complete lack of structural organization and functional coordination with the normal tissue, and usually form a distinct mass of tissue which may be benign (benign tumor) or malignant (carcinoma). The term "cancer" is used as a general term to describe any of various types of malignant neoplastic disease, most of which invade surrounding tissues, may metastasize to several sites and are likely to recur after attempted removal and to cause death of the patient unless adequately treated. As used herein, the term cancer is subsumed under the term neoplastic disease.

As used herein, "fibrotic processes" or "fibrosis" refers to the formation of fibrous tissues as a reaction or as a repair process that may occur during diseases of diverse origin (including cancerous diseases and inflammation) and/or treatment. The formation of fibrous tissue may replace other tissue and the resulting "scar tissue" may affect the functionality of the respective organ in a detectable manner. As part of this invention, these processes can be detected in the primary lesions and metastatic lesions of cancerous disease. This refers to the fact that ECM remodeling (e.g., destruction of the basement membranes during early invasion steps) encapsulates tumor cells and results in the formation of a tumor bed.

Scientific advances demonstrate that general pathogenic processes in the liver such as fibrotic processes involve proliferation and activation of hepatic stellate cells (also called lipocytes, fat-storing or Ito cells), which synthesize and secrete excess extracellular matrix proteins. However, fibrotic processes are not restricted to the liver tissue. Fibrosis refers to the formation of fibrous tissues as a reaction or as a repair process that may occur during disease of diverse origin (including inflammation) and/or treatment. The formation of fibrous tissue may replace other tissue and the resulting "scar tissue" may affect the functionality of the respective organ in a detectable manner. In the liver, fibrotic changes are common for diseases of multiple etiologies, e.g., chronic viral hepatitis B and C, alcoholic liver disease, as well as autoimmune and genetic liver diseases. All of these diseases lead to clinical problems via the common final pathway of progressive liver fibrosis and the eventual development of cirrhosis.

Hepatic fibrosis is a reversible accumulation of extracellular matrix in response to chronic injury in which nodules have not yet developed, whereas cirrhosis implies an irreversible process, in which thick bands of matrix fully encircle the parenchyma, forming nodules. Assessment of dynamic processes in diseased tissues by serial determination of serum parameters enables effective monitoring of disease status and response to treatment.

Methods of the invention can assess changes within samples taken from a patient at different time points before, during, or after treatment. Predictor values determined based on such serial sampling are compared to predictor values calculated using normal or adjusted disease-associated levels.

Fibrosis-like activity in the liver may discontinue temporarily due to changes in neoplastic tissue caused by treatment. Treatment-related inflammatory processes may also be induced due to pronounced cell death of cancerous or non -cancerous cells and invasion of immune cells; marker protein expression (EGFRs, VEGFRs, VEGF ligands, etc.) may be reduced in response to toxic or cytostatic treatment of tumor or stroma cells. Therefore, an assessment of changes related to fibrotic process may give additional information over a single time point adjustment of e.g. pretreatment samples.

"Validation cohort marker score values" means a numerical score derived from the linear combination of the discriminant weights obtained from the training cohort and marker values for each patient in the validation cohort

"Patient marker cut-off values" means the value of a marker of combination of markers at which a predetermined sensitivity or specificity is achieved. "Positive Predictive Value" ("PPV"): means the probability of having a disease given that a maker value (or set of marker values) is elevated above a defined cutoff

"Receiver Operator Characteristic Curve" ("ROC"): is a graphical representation of the functional relationship between the distribution of a marker's sensitivity and 1-specificity values in a cohort of diseased persons and in a cohort of non-diseased persons.

"Area Under the Curve" ("AUC") is a number which represents the area under a Receiver Operator Characteristic curve. The closer this number is to one, the more the marker values discriminate between diseased and non-diseased cohorts

"McNemar Chi-square Test" ("The McNemar $\chi^2$ test") is a statistical test used to determine if two correlated proportions (proportions that share a common numerator but different denominators) are significantly different from each other.

A "nonparametric regression analysis" is a set of statistical techniques that allows the fitting of a line for bivariate data that make little or no assumptions concerning the distribution of each variable or the error in estimation of each variable. Examples are: Theil estimators of location, Passing-Bablok regression, and Deming regression.

"Cut-off values" or "Threshold values" are numerical value of a marker (or set of markers) that defines a specified sensitivity or specificity.

The term "equivalent", with respect to a nucleotide sequence, is understood to include nucleotide sequences encoding functionally equivalent polypeptides. Equivalent nucleotide sequences will include sequences that differ by one or more nucleotide substitutions, additions or deletions, such as allelic variants and therefore include sequences that differ due to the degeneracy of the genetic code. "Equivalent" also is used to refer to amino acid sequences that are functionally equivalent to the amino acid sequence of a mammalian homolog of a blood (e.g., sera) marker protein, but which have different amino acid sequences, e.g., at least one, but fewer than 30, 20, 10, 7, 5, or 3 differences, e.g., substitutions, additions, or deletions.

As used herein, the terms "neoplastic disease serum marker gene" refers to a nucleic acid which: (1) encodes neoplastic disease blood (e.g., serum) marker proteins, including neoplastic disease serum marker proteins identified herein; and (2) which are associated with an open reading frame, including both exon and (optionally) intron sequences. A "neoplastic disease serum marker gene" can comprise exon sequences, though it may optionally include intron sequences which are derived from, for example, a related or unrelated chromosomal gene. The term "intron" refers to a DNA sequence present in a given gene which is not translated into protein and is generally found between exons. A gene can further include regulatory sequences, e.g., a promoter, enhancer and so forth. " "Neoplastic disease serum marker gene" includes but is not limited to nucleotide sequences which are complementary, equivalent, or homologous to SEQ ID NOS: 1-42 of Table 2.

"Homology", "homologs of', "homologous", or "identity" or "similarity" refers to sequence similarity between two polypeptides or between two nucleic acid molecules, with identity being a more strict comparison. Homology and identity can each be determined by comparing a position in each sequence which may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences.

The term "percent identical" refers to sequence identity between two amino acid sequences or between two nucleotide sequences. Identity can each be determined by comparing a position in each sequence which may be aligned for purposes of comparison.

When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences. Other techniques for determining sequence identity are well-known and described in the art.

Preferred nucleic acids used in the instant invention have a sequence at least 70%, and more preferably 80% identical and more preferably 90% and even more preferably at least 95% identical to, or complementary to, a nucleic acid sequence of a mammalian homolog of a gene that expresses a marker as defined previously. Particularly preferred nucleic acids used in the instant invention have a sequence at least 70%, and more preferably 80% identical and more preferably 90% and even more preferably at least 95% identical to, or complementary to, a nucleic acid sequence of a mammalian homolog of a gene that expresses a marker as defined previously.

Immunoassays.

Serum immunoassays to detect and measure levels of (1) at least one tumor marker or at least one immune marker or at least one acute phase marker, and (2) at least one marker that is (i) an ECM marker (ii) a marker that is indicative of extracellular matrix synthesis (fibrogenesis), or (iii) a marker that is indicative of extracellular matrix degradation (fibrolysis) can be made in accordance with the protocols described hereinafter. Supplementary markers including weight, sex and age, and expression profiling data of fresh and fixed tumor tissue, can also be assessed in determining predictor values in accordance with methods of the invention.

Levels of (1) at least one tumor marker or at least one immune marker or one acute phase marker, and (2) at least one marker that is (i) an ECM marker (ii) a marker that is indicative of extracellular matrix synthesis (fibrogenesis), or (iii) a marker that is indicative of extracellular matrix degradation (fibrolysis) can be measured using sandwich immunoassays. Two antibodies can be reacted with human fluid samples, wherein the capture antibody specifically binds to one epitope of the marker. The second antibody of different epitope specificity is used to detect this complex. Preferably, the antibodies are monoclonal antibodies, although also polyclonal antibodies can be employed. Both antibodies used in the assays specifically bind to the analyte protein.

For example, Her 2-neu ELISA (Bayer) can be used to detect the extracellular domain of Her-2/neu in serum samples of cancer patients by utilizing two mouse monoclonal antibodies directed against the extracellular domain. EGFr ELISA (Bayer) can be used to detect the extracellular domain of EGFr in serum samples of cancer patients by utilizing two mouse monoclonal antibodies directed against the extracellular domain uPA ELISA (Bayer) can be used to detect the uPA in serum samples of cancer patients by utilizing two mouse monoclonal antibodies directed against the secreted portion of the protein. CA 19-9 (Bayer) can be used to detect CA 19-9 in serum samples of cancer patients by utilizing two mouse monoclonal antibodies directed against the secreted portion of the protein. CA 15-3® (Bayer) can be used to detect the Muc-1 protein in serum samples of cancer patients by utilizing two mouse monoclonal antibodies directed against the the Muc-1 gene product.

Additionally, an assay for collagen IV can use a monoclonal antibody from Fuji (IV-4H12)(Accession No. FERM BP-2847) paired with a polyclonal antibody from Biodesign (T59106R)(Biodesign Catalog No.: T59106R). Assays can be heterogeneous immunoassays employing a magnetic particle separation technique.

An assay for PIIINP can use a Bayer monoclonal antibody deposited under the Budapest Treaty on May 24, 2004 with the American Type Culture Collection, 10801 University Boulevard, Manassas, VA 20110-2209 (ATCC PTA-6013) paired with a monoclonal antibody from Hoechst (Accession No. ECCAC 87042308).

Table 1 below lists the antibodies used to detect the ECM, fibrosis and fibrogenesis marker which were used within this invention.

TABLE 1

Antibody useful for ECM, fibrosis and fibrogenesis Panel

| Marker Gene | Reagent | Ab Clone | Supplier/Developer |
|---|---|---|---|
| Collagen IV | R1 | IV-4H12 | ICN |
| Collagen IV | R2 | T59106R | Biodesign |
| PIIINP | R1 | P3P 296/3/27 | Dade Behring |
| PIIINP | R2 | 35J23 | TSD |
| Collagen VI | R1 | 34C6 | TSD |
| Collagen VI | R2 | 34F9 | TSD |
| TIMP-1 | R1 | PRU-T9 | Prof. Clark (UK) |
| TIMP-1 | R2 | 11E7C6 | Connex |
| Tenascin | R1 | 23G1 | TSD |
| Tenascin | R2 | 23G2 | TSD |
| Laminin | R1 | 67A23 | TSD |
| Laminin | R2 | 67F8 | TSD |
| MMP2 | R1 | 85C1 | TSD |
| MMP2 | R2 | VB31B4 | Prof. Windsor (USA) |
| MMP-9 / TIMP-1 | R1 | 11E7C6 | Connex |
| MMP-9 / TIMP-1 | R2 | 277.13 | Bayer Pharmaceuticals |
| Hyaloronic Acid | R1 | HABP* | Bovine |
| Hyaloronic Acid | R2 | HABP* | Bovine |

*Hyaloronic Acid Binding Protein isolated from bovine nasal cartilage

Table 2 below lists representative nucleotide sequences which can be expressed to yield markers which are useful in methods of the invention.

TABLE 2

| Representative Nucleotide Sequences | | | | |
|---|---|---|---|---|
| **Gene Symbol** | **Gene Description** | **Ref. Sequences** | **Unigene_ID** | **OMIM** |
| MMP-2 | matrix metalloproteinase 2 preproprotein | NM_004530 | Hs. 111301 | 120360 |
| MMP3 | matrix metalloproteinase 3 preproprotein | NM_002422 | Hs. 83326 | 185250 |
| MMP7 | matrix metalloproteinase 7 preproprotein | NM_002423 | Hs. 2256 | 178990 |
| MMP9 | matrix metalloproteinase 9 preproprotein | NM_004994 | Hs. 151738 | 120361 |

(continued)

| | Representative Nucleotide Sequences | | | |
|---|---|---|---|---|
| **Gene Symbol** | **Gene Description** | **Ref. Sequences** | **Unigene_ID** | **OMIM** |
| MMP12 | matrix metalloproteinase 12 preproprotein | NM_002426 | Hs. 1695 | 601046 |
| MMP24 | matrix metalloproteinase 24 (membrane-inserted) | NM_006690 | Hs. 3743 | 604871 |
| COL1A1 | alpha 1 type I collagen preproprotein | NM_000088 | Hs. 172928 | 120150 |
| COL2A1 | alpha 1 type II collagen isoform 1 | NM_001844 | Hs.81343 | 120140 |
| COL3A1 | alpha 1 type III collagen | NM_000090 | Hs. 119571 | 120180 |
| COL4A1 | alpha 1 type IV collagen preproprotein | NM_001845 | Hs. 119129 | 120130 |
| COL4A2 | alpha 2 type IV collagen preproprotein | NM_001846 | Hs.75617 | 120090 |
| COL4A3 | alpha 3 type IV collagen isoform 1, precursor | NM_000091 | Hs.530 | 120070 |
| COL4A4 | alpha 4 type IV collagen precursor | NM_000092 | Hs.180828 | 120131 |
| COL4A5 | alpha 5 type IV collagen isoform 1, precursor | NM_000495 | Hs. 169825 | 303630 |
| COL4A6 | type IV alpha 6 collagen isoform A precursor | NM_001847 | Hs.408 | 303631 |
| COL5A1 | alpha 1 type V collagen preproprotein | NM_000093 | Hs. 146428 | 120215 |
| COL5A2 | alpha 2 type V collagen preproprotein | NM_000393 | Hs.82985 | 120190 |
| COL5A3 | collagen, type V, alpha 3 preproprotein | NM_015719 | Hs.235368 | 120216 |
| COL6A1 | alpha 1 type VI collagen preproprotein | NM_001848.1 | Hs.474053 | 120220 |
| COL6A2 | alpha 2 type VI collagen isoform 2C2 precursor | NM_001849 | Hs. 159263 | 120240 |
| COL6A3 | alpha 3 type VI collagen isoform 1 precursor | NM_004369 | Hs.80988 | 120250 |
| COL7A1 | alpha 1 type VII collagen precursor | NM_000094 | Hs.1640 | 120120 |
| COL8A1 | alpha 1 type VIII collagen precursor | NM_001850 | Hs.114599 | 120251 |
| COL9A1 | alpha 1 type IX collagen isoform 1 precursor | NM_001851 | Hs.154850 | 120210 |
| COL9A2 | alpha 2 type IX collagen | NM_001852 | Hs.37165 | 120260 |
| COL9A3 | alpha 3 type IX collagen | NM_001853 | Hs.53563 | 120270 |
| COL10A 1 | collagen, type X, alpha 1 precursor | NM_000493 | Hs. 179729 | 120110 |
| COL11A 1 | alpha 1 type XI collagen isoform A preproprotein | NM_001854 | Hs.82772 | 120280 |
| COL13A 1 | alpha 1 type XIII collagen isoform 1 | NM_005203 | Hs.211933 | 120350 |
| COL14A 1 | alpha 1 type XIV collagen precursor | NM_021110 | Hs.36131 | 120324 |
| COL15A 1 | alpha 1 type XV collagen precursor | NM_001855 | Hs.83164 | 120325 |
| COL16A 1 | alpha 1 type XVI collagen precursor | NM_001856 | Hs.26208 | 120326 |
| COL17A 1 | alpha 1 type XVII collagen | NM_000494 | Hs.117938 | 113811 |
| COL18A 1 | alpha 1 type XVIII collagen precursor | NM_016214 | Hs.78409 | 120328 |
| COL19A 1 | alpha 1 type XIX collagen precursor | NM_001858 | Hs.89457 | 120165 |
| LAMA2 | laminin alpha 2 subunit precursor | NM_000426 | Hs.323511 | 156225 |
| LAMA3 | laminin alpha 3 subunit precursor | NM_000227 | Hs.83450 | 600805 |
| LAMA4 | laminin, alpha 4 precursor | NM_002290 | Hs.78672 | 600133 |
| LAMA5 | laminin alpha 5 | NM_005560 | Hs.312953 | 601033 |
| LAMB1 | laminin, beta 1 precursor | NM_002291 | Hs.82124 | 150240 |
| LAMB2 | lamin B2 | NM_032737 | Hs.76084 | 150341 |

(continued)

| Representative Nucleotide Sequences | | | | |
|---|---|---|---|---|
| **Gene Symbol** | **Gene Description** | **Ref. Sequences** | **Unigene_ID** | **OMIM** |
| LAMB2 | laminin, beta 2 precursor | NM_002292 | Hs.90291 | 150325 |
| LAMB3 | laminin subunit beta 3 precursor | NM_000228 | Hs.75517 | 150310 |
| LAMC1 | laminin, gamma 1 precursor | NM_002293 | Hs.214982 | 150290 |
| LAMC2 | laminin, gamma 2 isoform a precursor | NM_005562 | Hs.54451 | 150292 |
| LAMC3 | laminin, gamma 3 precursor | NM_006059 | Hs.69954 | 604349 |
| HXB | tenascin C (hexabrachion) | NM_002160 | Hs.289114 | 187380 |
| TIMP-1 | tissue inhibitor of metalloproteinase 1 precursor | NM_003254 | Hs.5831 | 305370 |
| PLAU | plasminogen activator, urokinase | NM_002658 | Hs.77274 | 191840 |
| VEGF | vascular endothelial growth factor alpha | NM_003376 | Hs.73793 | 192240 |
| CEACA M1 | carcinoembryonic antigen-related cell adhesion molecule 1 (biliary glycoprotein) | NM_001712 | Hs.50964 | 109770 |
| MUC1 | mucin 1, transmembrane | NM_002456 | Hs.89603 | 158340 |
| MUC1 | mucin 1, transmembrane | NM_182741 | Hs.89603 | 158340 |
| IL2RA | interleukin 2 receptor, alpha chain precursor | NM_000417 | Hs.1724 | 147730 |
| IL6 | interleukin 6 (interferon, beta 2) | NM_000600 | Hs.93913 | 147620 |
| GAS | gastrin precursor | NM_000805 | Hs.2681 | 137250 |

Antibodies for the detection of (1) at least one tumor marker or at least one immune marker or at least one acute phase marker, and (2) at least one marker that is (i) an ECM marker (ii) a marker that is indicative of extracellular matrix synthesis (fibrogenesis), or (iii) a marker that is indicative of extracellular matrix degradation (fibrolysis), can be made in accordance with the Expression of Polynucleotide Protocol and Hybridoma Development Protocol described in detail below.

Expression of Polynucleotides:

To express the nucleotides listed in Table 2 and other neoplastic disease-related marker genes, the genes can be inserted into an expression vector which contains the necessary elements for the transcription and translation of the inserted coding sequence. Methods which are well known to those skilled in the art can be used to construct expression vectors containing sequences encoding neoplastic disease-related marker polypeptides and appropriate transcriptional and translational control elements. These methods include *in vitro* recombinant DNA techniques, synthetic techniques, and *in vivo* genetic recombination. Such techniques are described, for example, in Sambrook et al., MOLECULAR CLONING: A LABORATORY MANUAL, 2d ed., (1989) and *in* Ausubel et al., CURRENT PROTOCOLS IN MOLECULAR BIOLOGY, John Wiley & Sons, New York, N.Y. (1989).

A variety of expression vector/host systems can be utilized to contain and express sequences encoding a neoplastic disease-related marker polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those regions of the vector enhancers, promoters, 5' and 3' untranslated regions which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a "Liver fibrosis gene" polypeptide, vectors based on SV40 or EBV can be used with an appropriate selectable marker.

Bacterial and Yeast Expression Systems:

In bacterial systems, a number of expression vectors can be selected depending upon the use intended for neoplastic disease-related marker polypeptide. For example, when a large quantity of neoplastic disease-related marker polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the neoplastic disease-related marker polypeptide can be ligated into the vector in frame with sequences for the amino terminal Met and the subsequent 7 residues of β-galactosidase so that a hybrid protein is produced. pIN vectors [Van Heeke & Schuster, J. Biol. Chem. 264, 5503-5509, (1989)] or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used.

Plant and Insect Expression Systems:

If plant expression vectors are used, the expression of sequences encoding neoplastic disease-related marker polypeptides can be driven by any of a number of promoters. For example, viral promoters such as the 35S and 19S promoters of CaMV can be used alone or in combination with the omega leader sequence from TMV [Takamatsu, EMBO J. 6, 307-311, (1987)]. Alternatively, plant promoters such as the small subunit of RUBISCO or heat shock promoters can be used [Coruzzi et al., EMBO J. 3, 1671-1680, (1984); Broglie et al., Science 224, 838-843, (1984); Winter et al., Results Probl. Cell Differ. 17, 85-105, (1991)]. These constructs can be introduced into plant cells by direct DNA transformation or by pathogen-mediated transfection. Such techniques are described in a number of generally available reviews (e.g., MCGRAW HILL YEARBOOK OF SCIENCE AND TECHNOLOGY, McGraw Hill, New York, N.Y., pp. 191-196, (1992)).].

An insect system also can be used to express a neoplastic disease-related marker polypeptide. For example, in one such system Autographa californica nuclear polyhedrosis virus (AcNPV) is used as a vector to express foreign genes in Spodoptera frugiperda cells or in Trichoplusia larvae. Sequences encoding neoplastic disease-related marker polypeptides can be cloned into a nonessential region of the virus, such as the polyhedrin gene, and placed under control of the polyhedrin promoter. Successful insertion of neoplastic disease-related marker polypeptide will render the polyhedrin gene inactive and produce recombinant virus lacking coat protein. The recombinant viruses can then be used to infect S. frugiperda cells or Trichoplusia larvae in which neoplastic disease-related marker polypeptides can be expressed [Engelhard et al., Proc. Nat. Acad. Sci. 91, 3224-3227, (1994)].

Mammalian Expression Systems:

A number of viral-based expression systems can be used to express neoplastic disease-related marker polypeptides in mammalian host cells. For example, if an adenovirus is used as an expression vector, sequences encoding neoplastic disease-related marker polypeptides can be ligated into an adenovirus transcription/translation complex comprising the late promoter and tripartite leader sequence. Insertion in a nonessential E1 or E3 region of the viral genome can be used to obtain a viable virus which is capable of expressing a neoplastic disease-related marker polypeptides in infected host cells [Logan & Shenk, Proc. Natl. Acad. Sci. 81, 3655-3659, (1984)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding neoplastic disease-related marker polypeptides. Such signals include the ATG initiation codon and adjacent sequences. In cases where sequences encoding a neoplastic disease-related marker polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used [Scharf et al., Results Probl. Cell Differ. 20, 125-162, (1994)].

Host Cells:

A host cell strain can be chosen for its ability to modulate the expression of the inserted sequences or to process the expressed neoplastic disease-related marker polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for Post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express neoplastic disease-related marker polypeptides can be transformed using expression vectors which can contain viral origins of replication and/or endogenous expression elements and a selectable marker gene on the same or on a separate vector. Following the introduction of the vector, cells can be allowed to grow for 12 days in an enriched medium before they are switched to a selective medium. The purpose of the selectable marker is to confer resistance to selection, and its presence allows growth and recovery of cells which successfully express the introduced neoplastic disease-related marker polypeptide gene sequences. Resistant clones of stably transformed cells can be proliferated using tissue culture techniques appropriate to the cell type. See, for example, Freshney R.I., ed., ANIMAL CELL CULTURE (1986)

Any number of selection systems can be used to recover transformed cell lines. These include, but are not limited to, the herpes simplex virus thymidine kinase (Wigler et al., Cell 11, 223-232, (1977)] and adenine phosphoribosyltransferase [Lowy et al., Cell 22, 817-823, (1980)] genes which can be employed in tk- or aprt- cells, respectively. Also, antimetabolite, antibiotic, or herbicide resistance can be used as the basis for selection. For example, dhfr confers resistance to methotrexate [Wigler et al., Proc. Natl. Acad. Sci. 77, 3567-3570, (1980)], npt confers resistance to the aminoglycosides, neomycin and G418 [Colbere-Garapin et al., J. Mol. Biol. 150, 114, (1981)], and als and pat confer resistance to chlorsulfuron and phosphinotricin acetyltransferase, respectively. Additional selectable genes have been described. For example, trpB allows cells to utilize indole in place of tryptopHAn, or hisD, which allows cells to utilize histinol in place of histidine [Hartman & Mulligan, Proc. Natl. Acad. Sci. 85, 8047-8051, (1988)]. Visible markers such as anthocyanins, β-glucuronidase and its substrate GUS, and luciferase and its substrate luciferin, can be used to identify transformants and to quantify the amount of transient or stable protein expression attributable to a specific vector system [Rhodes et al., Methods Mol. Biol. 55, 121-131, (1995)].

Detecting Expression and Gene Products:

Although the presence of marker gene expression suggests that a neoplastic disease-related marker polypeptide gene is also present, the presence and expression of that gene may need to be confirmed. For example, if a sequence encoding a neoplastic disease-related marker polypeptide is inserted within a marker gene sequence, transformed cells containing sequences which encode a neoplastic disease-related marker polypeptide can be identified by the absence of marker gene function. Alternatively, a marker gene can be placed in tandem with a sequence encoding a neoplastic disease-related marker polypeptide under the control of a single promoter. Expression of the marker gene in response to induction or selection usually indicates expression of the neoplastic disease-related marker polypeptide.

Alternatively, host cells which contain a neoplastic disease-related marker polypeptides and which express a neoplastic disease-related marker polypeptide can be identified by a variety of procedures known to those of skill in the art. These procedures include, but are not limited to, DNA-DNA or DNA-RNA hybridization and protein bioassay or immunoassay techniques which include membrane, solution, or chip-based technologies for the detection and/or quantification of nucleic acid or protein. For example, the presence of a polynucleotide sequence encoding a neoplastic disease-related marker polypeptide can be detected by DNA-DNA or DNA-RNA hybridization or amplification using probes or fragments or fragments of polynucleotides encoding a neoplastic disease-related marker polypeptide. Nucleic acid amplification-based assays involve the use of oligonucleotides selected from sequences encoding a neoplastic disease-related marker polypeptide to detect transformants which contain a neoplastic disease-related marker polypeptide.

A variety of protocols for detecting and measuring the expression of a neoplastic disease-related marker polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a neoplastic disease-related marker polypeptide can be used, or a competitive binding assay can be employed. These and other assays are described in Hampton et al., SEROLOGICAL METHODS: A LABORATORY MANUAL, APS Press, St. Paul, Minn., (1990) and Maddox et al., J. Exp. Med. 158, 1211-1216, (1983).

A wide variety of labels and conjugation techniques are known by those skilled in the art and can be used in various nucleic acid and amino acid assays. Means for producing labeled hybridization or PCR probes for detecting sequences related to polynucleotides encoding neoplastic disease-related marker polypeptides include oligo labeling, nick translation, end-labeling, or PCR amplification using a labeled nucleotide. Alternatively, sequences encoding a neoplastic disease-related marker polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

Expression and Purification of Polypeptides:

Host cells transformed with nucleotide sequences encoding a neoplastic disease-related marker polypeptide can be cultured under conditions suitable for the expression and recovery of the protein from cell culture. The polypeptide produced by a transformed cell can be secreted or stored intracellular depending on the sequence and/or the vector used. As will be understood by those of skill in the art, expression vectors containing polynucleotides which encode neoplastic disease-related marker polypeptides can be designed to contain signal sequences which direct secretion of soluble neoplastic disease-related marker polypeptides through a prokaryotic or eukaryotic cell membrane or which direct the membrane insertion of membrane-bound neoplastic disease-related marker polypeptides.

As discussed above, other constructions can be used to join a sequence encoding a neoplastic disease-related marker polypeptides to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the neoplastic disease-related marker polypeptide also can be used to facilitate purification. One such expression vector provides for expression of a fusion protein containing a neoplastic disease-related marker polypeptide and 6 histidine residues preceding a thioredoxin or an enterokinase cleavage site. The histidine residues facilitate purification by IMAC (immobilized metal ion affinity chromatography, as described in Porath et al., Prot. Exp. Purif. 3, 263-281 (1992)), while the enterokinase cleavage site provides a means for purifying the Liver fibrosis gene" polypeptide from the fusion protein. Vectors which contain fusion proteins are disclosed in Kroll et al., DNA Cell Biol. 12, 441-453, (1993)

Chemical Synthesis:

Sequences encoding a neoplastic disease-related marker polypeptide can be synthesized, in whole or in part, using chemical methods well known in the art (see Caruthers et al., Nucl. Acids Res. Symp. Ser. 215-223, (1980) and Horn et al. Nucl. Acids Res. Symp. Ser. 225-232, (1980). Alternatively, a neoplastic disease-related marker polypeptide itself can be produced using chemical methods to synthesize its amino acid sequence, such as by direct peptide synthesis using solid-phase techniques [Merrifield, J. Am. Chem. Soc. 85, 2149-2154, (1963) and Roberge et al., Science 269, 202-204, (1995)]. Protein synthesis can be performed using manual techniques or by automation. Automated synthesis can be achieved, for example, using Applied Biosystems 431A Peptide Synthesizer (Perkin Elmer). Optionally, fragments of neoplastic disease-related marker polypeptides can be separately synthesized and combined using chemical methods to produce a full-length molecule.

The newly synthesized peptide can be substantially purified by preparative high performance liquid chromatography [Creighton, PROTEINS: STRUCTURES AND MOLECULAR PRINCIPLES, WH and Co., New York, N.Y., (1983)]. The composition of a synthetic neoplastic disease-related marker polypeptide can be confirmed by amino acid analysis or sequencing (e.g., the Edman degradation procedure; see Creighton. Additionally, any portion of the amino acid sequence of the neoplastic disease-related marker polypeptide can be altered during direct synthesis and/or combined using chemical methods with sequences from other proteins to produce a variant polypeptide or a fusion protein.

Hybridoma Development Protocol

Phase I: Immunization.

BALB/c mice and Swiss Webster mice (five per group) are immunized intraperitoneally with one of the above-identified neoplastic disease-related markers (different doses) emulsified with complete Freund's adjuvant (CFA) followed by three boosts (at two weeks interval) with immunogen emulsified with incomplete Freund's adjuvant. Mice are bled one week after each boost and sera titrated against the immunogen in ELISA. The mouse with the highest titer is selected for fusion.

Phase II: Cell Fusion and hybridoma selection.

The mouse selected for fusion is boosted with the same dose of antigen used in previous immunizations. The boost is given four days prior to splenectomy and cell fusion. The antigen preparation is given intraperitoneally without adjuvant.

On the day of fusion the mouse is sacrificed and the spleen is removed aseptically. The spleen is minced using forceps and strained through a sieve. The cells are washed twice using Iscove's modified Eagle's media (IMDM) and are counted using a hemacytometer.

The mouse myeloma cell line P3x63Ag8.653 is removed from static, log-pHAse culture, washed with IMDM and counted using a hemacytometer.

Myeloma and spleen cells are mixed in a 1:5 ratio and centrifuged. The supernatant is discarded. The cell pellet is gently resuspended by tapping the bottom of the tube. One milliliter of a 50% solution of PEG (MW 1450) is added drop by drop over a period of 30 seconds. The pellet is mixed gently for 30 seconds using a pipette. The resulting cell suspension is allowed to stand undisturbed for another 30 seconds. Five milliliters of IMDM is added over a period of 90 seconds followed by another 5 ml immediately. The resulting cell suspension is left undisturbed for 5 minutes. The cell suspension is spun and the pellet is re-suspended in HAT medium (IMDM containing 10% FBS, 2 mM L-glutamine, 0.6% 2-mercaptoethanol (0.04% solution), hypoxanthine, aminopterin, thymidine, and 10% Origen growth factor). The cells are resuspended to 5E5 cells per milliliter. Cells are plated into 96-well plates. Two hundred microliters or 2E5 cells are added to each well.

Plates are incubated at 37°C in a 7% $CO_2$ atmosphere with 100% humidity. Seven days after fusion, the media is removed and replaced with IMDM containing 10% FBS, 2 mM L-glutamine, 0.6% 2-mercaptoethanol stock (0.04%), hypoxanthine and thymidine. Typically, growing colonies of hybridomas are seen microscopically about seven days after the fusion. These colonies can be seen with the naked eye approximately 10-14 days after fusion.

Ten to fourteen days after fusion, the supernatant is taken from wells with growing hybridoma colonies. The volume of supernatant is approximately 150-200 microliters and contains 10-100 micrograms of antibody per milliliter. This supernatant is tested for specific antibody using the same assay(s) used to screen the sera. Positive hybridoma colonies are moved from the 96-well plate to a 24-well plate. Three to five days later, the supernatant from 24-well plate is tested to confirm the presence of specific antibody. The volume of supernatant from one well of a 24-well plate is approximately 2 mL and contains 10-100 micrograms/mL of antibody. Cells from positive wells are expanded in T-25 and T-75 flasks. Cells are frozen from T-75 flasks. Cells from positive wells are also cloned by limiting dilution. Hybridoma cells are plated onto 96-well plates at a density of 0.25 cells per well or one cell in every fourth well. Growing colonies are tested 10-14 days later using the same assay(s) used to initially select the hybridomas. Positive clones are expanded and frozen.

Phase III: Production.

Hybridoma cells expanded to T-162 flasks followed by transferring these to roller bottles for production of cell supernatant. The cells are grown in roller bottles for about two weeks until the cells are less than 10% viable. The culture supernatant is harvested from these roller bottles for purification.

Brief description of Immunoassays.

All antibodies are heterogenous ELISA-type assays formatted for the Bayer Immuno 1 system or 96 well plates. The system employs fluorescein-labeled capture antibodies (denoted R1) and alkaline phosphatase labled tag antibodies (denoted R2). The antibody conjugates are dissolved in a physiological buffer at a concentration between 2 and 50 mg/L. The immunoreactive reagents are incubated with a fixed amount of patient sample containing the antigen to be assayed. The patient sample is always pipetted first into a reaction cuvette followed by R1 thirty seconds later. R2 is normally added 30 seconds to 20 minutes after the R1 addition. The mixture is incubated for a maximum of 20 minutes although other embodiments of the immunoassays might require longer of shorter incubation times. Subsequently, immunomagnetic particles are added to the mixture. The particles consist of iron oxide containing polyacrylamide beads with anti-fluorescein antibodies conjugated to the particle surface. The particles are commercially available from Bayer HealthCare Diagnostics.

Upon incubation of the immunomagnetic particles with the sandwich immunocomplex formed from the antigen and the R1 and R2 conjugates, the sandwich immunocomplex is captured through the fluorescein label of the R1 antibody by the anti-fluorescein antibodies on the immuno-magnetic particles. The super-complex formed is precipitated by an external magnetic field. All unbound material, especially R2 alkaline phosphatate conjugate is removed by washing. The washed complex is then resuspended in p-nitrophenolphosphate solution. The rate of color formation is proportional to the amount of phosphatase left in the cuvette which is proportional to the amount of antigen. Quantification is achieved by recording a six-point calibration curve and a calibration curve, constructed by a cubic regression or a Rodbard fit.

(a) Assay Performance.

The performance of each of the assays is determined in isolation. The sensitivity and specificity, inter and intra-assay variation, interferences, linearity and parallelism are determined for each immunoassay. The ranges of results obtained for healthy subjects of both sexes and a range of ages from 18 to 75 years is determined to establish "normal" values. The assays are applied to subjects with a range of pathological disorders.

The invention is illustrated further in the following non-limiting examples.

EXAMPLE 1

Colorectal Cancer Patient Treatment

Summary

A statistically significant discrimination of patient overall survival (p less than about 0.05 level when calculated with Kaplan-Meier plots) was achieved (even in single parameter analysis) using methods of the invention. Elevated or decreased levels of serum markers were compared with normal control levels or adjusted mean levels of diseased cohorts. The significance of individual markers was determined by calculating the Kaplan-Meier plots from patients (using the upper or lower quartile of the individual marker levels). A decrease or increase in the levels of the markers in the cancer patient compared to the levels in normal controls indicated an increase in stage, grade, severity, advancement or progression of the patient's cancer and/or a lack of efficacy or benefit of the cancer treatment or therapy. In particular, high levels of Gastrin, CA 19-9, TIMP-1, and low level of EGFr, MMP-2 correlated with poor prognosis. In addition combined analysis of high levels of Collagen VI, Tenascin, uPA and low levels of PIIINP, VEGF correlated with good prognosis. Some singular serum parameters yielded statistically significant mean values and differentiated the cohorts according to differences in the study endpoints

Clinical Methodology

Forty-four patients suffering from colorectal carcinoma metastatic to the liver were studied. Primary carcinoma was confirmed histologically. Histological confirmation was also obtained for synchronous liver metastasis. When metachronous liver metastasis was identified, histological confirmation was only pursued when imaging techniques (spiral computerized tomography (CT) of the abdomen or MRT of the liver) did not show clear results.

Patients received first-line chemotherapy, consisting of a weekly 1-2 hour infusion of folinic acid (500 mg m$^{-2}$) followed by a 24-hour infusion of 5-fluorouracil (2600 mg m$^{-2}$). One cycle comprised six weekly infusions followed by 2 weeks of rest. A total of 23 patients received additional biweekly oxaliplatin (85 mg m$^{-2}$) and three patients also received irinotecan once per week (80 mg m$^{-2}$). Treatment response was monitored every 8 weeks by spiral CT and antitumour activity was evaluated in accordance with WHO criteria. Median treatment duration was 7 months. Table 3 below lists tumor sizes as adjusted by computertomography at each therapy cycle to assess tumor response to treatment.

TABLE 3

| | | | | Clinical assessment | | | | |
|---|---|---|---|---|---|---|---|---|
| Patient ID | PreTreatment | after 1st cycle | after 2nd cycle | after 3rd cycle | after 4th cycle | after 5th cycle | after 6th cycle | Tumor Size % of initial size |
| G 42 | 4.8 | 3.6 | 3.6 | 3.6 | 3.6 | 7.5 | | 75 |
| G 52 | 91.6 | 97.1 | 72.3 | 72.3 | 79.4 | | | 74.4 |
| G 53 | 132.8 | 54 | 16 | 54 | | | | 3.4 |
| G 56 | 10.6 | 10.6 | 10.6 | 2.4 | | | | 22.7 |
| G 60 | 36 | 26 | 21.1 | 18.9 | | | | 52.5 |
| G 226 | 9 | 12.3 | | | | | | 136 |
| G 73 | 15.2 | 11.2 | 8.4 | 4 | 4 | 6.3 | 18 | 26.3 |
| G 79 | 34 | 18.5 | 5.6 | 4.5 | 9.5 | | | 13.2 |
| G 85 | 180 | 104 | 69 | 61.8 | 65.2 | | | 34.3 |
| G 86 | 216.3 | 31.8 | 19.8 | 30.6 | | | | 9.1 |
| G 87 | 9.3 | 1.8 | 0 | | | | | 0 |
| G 88 | 182.2 | 73.2 | 43.3 | 26 | | | | 14.2 |
| G 92 | | | | | | | | |
| G 96 | 116.2 | 62.8 | 42 | 28.3 | | | | 24,2 |
| G 98 | 14.3 | 3 | 1.4 | 1.4 | | | | 9.8 |
| G100 | 13.3 | 9 | 6.3 | 1 | 0.3 | 0.25 | 0.3 | 1.9 |
| G101 | 9 | 3.3 | 3.2 | 1.4 | 1.2 | | | 13 |
| G103 | 3.3 | 1.8 | 2.3 | 2.3 | 3.8 | | | 54.5 |
| G111 | 15.2 | 11.8 | 8.4 | 8 | | | | 52.6 |
| G116 | 49 | 9 | | 2.4 | | | | 4.9 |
| G119 | 5.3 | 4 | 1.8 | | | | | 34 |
| G131 | 4 | 4 | 0.8 | 0.5 | | | | 12.5 |
| G218 | 12.3 | 0 | | | | | | 0 |
| G136 | 21 | 13.5 | 6.3 | 4 | 6 | | | 19 |
| G138 | 102 | 37.5 | 13.3 | | | | | 13 |
| G148 | 25 | 25 | 16 | 20.3 | | | | 64,0 |
| G151 | 60 | 33 | 22.4 | 20 | 20 | 30.3 | | 33 |
| G152 | 32 | 15.4 | 8.2 | 6.3 | | | | 19.7 |
| G154 | 110.3 | 36 | 18 | 10.6 | | | | 9.6 |
| G166 | 30 | 21.4 | 12 | 12 | | | | 40 |
| G169 | 45.3 | 27 | 27 | 35.3 | | | | 59.6 |
| G170 | 25 | 14.7 | 8.4 | 8.4 | 8.4 | 9 | 7.5 | 30 |
| G173 | 22 | 16 | 6 | 6.2 | | | | 27.2 |
| G177 | 3.2 | 5.3 | | | | | | 160 |
| G178 | 225 | 143 | 125.4 | | | | | 55.7 |

(continued)

| Clinical assessment | | | | | | | | |
|---|---|---|---|---|---|---|---|---|
| Patient ID | PreTreatment | after 1st cycle | after 2nd cycle | after 3rd cycle | after 4th cycle | after 5th cycle | after 6th cycle | Tumor Size % of initial size |
| G179 | 16 | 13.7 | 9 | 7.5 | 22.1 | | | 46.8 |

Serum was obtained from each patient immediately prior to treatment and longitudinal serum samples were taken at each cycle. The following serum and plasma parameters were determined: MMP2, TIMP1, MMP9, Collagen IV, CollagenVI, PIIINP, Tenascin, Laminin, CEA, CA15-3, CA19-9, sHer-2/neu, EGFR, uPA and PAI-1. Patients were classified according to their overall survival and disease free survival.

EXAMPLE 2

Determination of Predictor Values and Derivation of Related Algorithm

Summary

Serum samples obtained from each patient as described in Example 1 were analyzed and neoplastic disease marker level values were used to generate algorithmically predictor values which correlated with patient survival.

Data Transformations

Values for the following seventeen markers were reported prior to the start of chemotherapy and during each of the chemotherapy cycles described below: MMP2, TIMP1, MMP9, Collagen IV, CollagenVI, PIIINP, Tenascin, Laminin, CEA, CA19-9, sHer-2/neu, EGFR, uPA, PAI-1, Gastrin, IL2R, and IL6.

Tables 4A and 4B display experimental data as determined by duplicate or triplicate measurements for each of the 17 indicated markers in the pretreatment serum sample.

TABLE 4A

| | | | Experimental Data and Threshold determination | | | | | | | |
|---|---|---|---|---|---|---|---|---|---|---|
| | | | CO 1037.6 | CO 674 | CO 316 | CO 33,7 | CutOff 1083 | CutOff 9,17 | CutOff 7,2 | CutOff 15ng |
| Patient ID | Survival | Survival | TIMP-1 | MMP-2 | COLIV | Laminin | Tenascin | PIIINP | Col VI | Her2/neu |
| ID | status | month | [ng/ml] | [ng/ml] | [ng/ml] | [ng/ml] | [ng/ml] | [ng/ml] | [ng/ml] | [ng/ml] |
| G 111 | alive | 41 | 468,7 | 540,9 | 105,3 | 18,8 | 358,3 | 5,9 | 8,4 | 9,66 |
| G 60 | alive | 35 | 665,8 | 553,7 | 176,9 | 23,4 | 533,0 | 7,8 | 7,1 | 12,1 |
| G 208 | alive | 23 | 471,6 | 1128,7 | 151,6 | 12,5 | 287,1 | 6,6 | 5,5 | 12,55 |
| G 18 | alive | 42 | 653,8 | 819,3 | | | | | | 9,3 |
| G 20 | alive | 33 | 648,3 | 416,4 | 160,7 | 22,7 | 323,4 | 4,9 | 4,3 | 7,3 |
| G 226 | alive | 21 | 1242,2 | 432,6 | 229,7 | 35,5 | 774,5 | 25,1 | 6,0 | 8,9 |
| G 14 | alive | 33 | 1897,2 | 483,9 | 590,9 | 60,8 | 1122,8 | 30,1 | 5,7 | 16,9 |
| G 88 | alive | 30 | 1085,7 | 520,9 | 366,0 | 27,5 | 470,5 | 22,5 | 5,7 | 12,4 |
| G 116 | alive | 42 | 917,5 | 554,9 | 184,3 | 48,7 | 973,0 | 12,7 | 5,0 | 6,86 |
| G 13 | alive | 47 | 1022,9 | 541,5 | 216,7 | 45,6 | 2199,3 | 21,6 | 7,7 | 9,8 |
| G 87 | alive | 61 | 848,7 | 1620,1 | 671,6 | 108,0 | 2364,7 | 71,8 | 22,0 | 20,41 |
| G 100 | alive | 45 | 1528,4 | 1079,7 | 510,4 | 73,7 | 1021,9 | 29,8 | 17,0 | 11,22 |
| G 119 | alive | 42 | 640,6 | 920,0 | 232,3 | 32,0 | | | | 9,76 |
| G 57 | alive | 45 | 639,7 | 817,4 | 210,5 | 25,1 | 175,4 | 8,4 | 4,4 | 7,7 |
| G 98 | alive | 40 | 821,9 | 838,1 | 170,7 | 31,9 | 821,4 | 20,2 | 9,9 | 10,24 |
| G 148 | dead | 22 | 1420,2 | 464,4 | 329,1 | 31,3 | 979,4 | 19,8 | 4,9 | 10,18 |
| G 103 | dead | 26 | 502,7 | 757,2 | 117,5 | 19,2 | 315,5 | 5,2 | 8,4 | 9,62 |
| G 169 | dead | 9 | 1220,0 | 428,8 | 165,1 | 19,4 | 276,6 | 12,7 | 4,7 | 8,57 |
| G 182 | dead | 9 | 1580,0 | 465,5 | 247,6 | 24,8 | 851,1 | 16,0 | 6,6 | 13,99 |
| G 19 | dead | 6 | 671,1 | 542,6 | 149,9 | 24,3 | 728,2 | 6,0 | 5,9 | 6,6 |
| G 196 | dead | 15 | 1387,6 | 438,7 | 312,6 | 31,6 | 750,0 | 21,2 | 5,1 | 25,02 |
| G 42 | dead | 25 | 728,6 | 495,0 | 166,7 | 24,2 | 842,7 | 14,2 | 8,2 | 5,9 |

(continued)

| | | | CO1037.6 | CO 674 | CO 316 | CO 33,7 | CutOff 1083 | CutOff 9,17 | CutOff 7,2 | CutOff 15ng |
|---|---|---|---|---|---|---|---|---|---|---|
| Patient ID | Survival | Survival | TIMP-1 | MMP-2 | COLIV | Laminin | Tenascin | PIIINP | Col VI | Her2/neu |
| ID | status | month | [ng/ml] | [ng/ml] | [ng/ml] | [ng/ml] | [ng/ml] | [ng/ml] | [ng/ml] | [ng/ml] |
| G 92 | dead | 30 | 765,2 | 619,0 | 155,3 | 24,5 | 1150,8 | 8,4 | 5,3 | 10,38 |
| G 52 | dead | 11 | 1381,1 | 564,0 | 273,4 | 44,2 | 739,0 | 15,7 | 6,2 | 7,9 |
| G 33 | dead | 15 | 658,4 | 611,2 | 186,7 | 44,8 | 410,3 | 11,0 | 6,0 | 8,2 |
| G 49 | dead | 11 | 2020,1 | 643,0 | 559,6 | 53,3 | 2201,7 | 26,5 | 5,7 | 55,8 |
| G 178 | dead | 11 | 1523,5 | 451,3 | 341,8 | 29,4 | 829,3 | 25,4 | 4,6 | 11,2 |
| G 15 | dead | 16 | 1193,5 | 1173,4 | | | | | | 7,7 |
| G 79 | dead | 16 | 835,1 | 571,7 | 194,5 | 44,4 | 492,8 | 10,1 | 6,7 | 5,7 |
| G 85 | dead | 18 | 1297,0 | 522,7 | 294,0 | 32,1 | 1128,5 | 18,2 | 4,1 | 6,95 |
| G 96 | dead | 23 | 741,8 | 326,8 | 161,5 | 38,7 | 577,6 | 11,7 | 2,3 | 7,36 |
| G 218 | dead | 18 | 805,6 | 767,5 | 196,4 | 27,6 | 577,0 | 25,4 | 4,0 | 14,6 |
| G 86 | dead | 13 | 1801,1 | 753,2 | 581,7 | 76,4 | 849,7 | 27,3 | 7,2 | 8,48 |
| G 192 | dead | 15 | 553,7 | 577,9 | 128,4 | 10,5 | 331,5 | 4,7 | 3,9 | 13,28 |
| G 152 | dead | 37 | 461,5 | 313,1 | 112,5 | 12,7 | 315,0 | 4,1 | 2,4 | 2,33 |
| G 73 | dead | 22 | 623,9 | 591,6 | 136,2 | 16,9 | 678,0 | 4,9 | 2,4 | 4,6 |
| G 101 | dead | 35 | 720,3 | 662,7 | 171,6 | 28,1 | 341,9 | 3,8 | 10,7 | 9,76 |
| G 136 | dead | 15 | 852,3 | 588,0 | 148,0 | 36,5 | 327,5 | 6,9 | 5,0 | 14,60 |
| G 53 | dead | 7 | 1882,2 | 518,3 | 243,5 | 69,7 | 817,4 | 13,0 | 10,0 | 6,8 |
| G 179 | dead | 14 | 1068,0 | 460,0 | 204,5 | 29,0 | 563,9 | 11,4 | 5,8 | 10,70 |
| G 131 | dead | 58 | 587,4 | 790,2 | 199,0 | 24,6 | 448,7 | | | 9,3 |
| G 138 | dead | 14 | 1159,4 | 599,3 | 371,5 | 42,1 | 931,0 | 14,7 | 5,1 | 13,55 |
| G 170 | dead | 24 | 506,5 | 611,2 | 142,5 | 18,1 | 288,9 | 5,8 | 5,0 | 10,54 |
| G 151 | dead | 28 | 919,0 | 599,2 | 238,2 | 34,0 | 853,3 | 21,5 | 5,9 | 7,8 |

Experimental Data and Threshold determination

(continued)

| Experimental Data and Threshold determination | | | | | | | | | | |
|---|---|---|---|---|---|---|---|---|---|---|
| | | | CO 1037.6 | CO 674 | CO 316 | CO 33,7 | CutOff 1083 | CutOff 9,17 | CutOff 7,2 | CutOff 15ng |
| Patient ID | Survival | Survival | TIMP-1 | MMP-2 | COLIV | Laminin | Tenascin | PIIINP | Col VI | Her2/neu |
| ID | status | month | [ng/ml] | [ng/ml] | [ng/ml] | [ng/ml] | [ng/ml] | [ng/ml] | [ng/ml] | [ng/ml] |
| G 154 | dead | 14 | 515,0 | 765,6 | 226,9 | 20,2 | 489,5 | 9,6 | 6,6 | 10,62 |
| G 184 | dead | 28 | 566,5 | 527,1 | 138,3 | 15,5 | 315,9 | 3,7 | 5,8 | 11,33 |
| G 173 | dead | 32 | 570,4 | 395,0 | 127,0 | 15,4 | 454,6 | 9,1 | 3,3 | 5,9 |
| G 166 | dead | 7 | 511,7 | 486,7 | 114,6 | 17,5 | 234,6 | 8,4 | 2,7 | 7,44 |

TABLE 4B

| | Survival | Survival | EGFR | TIMP-1 | uPA | VEGF | CEA | CA 19-9 | IL2R | IL6 | Gastrin |
|---|---|---|---|---|---|---|---|---|---|---|---|
| | | | | | | | | | | | |
| | | | | | | | Experimental Data and Threshold determination | | | | |
| ID | status | month | [ng/ml] | [ng/ml] | [pg/ml] | [pg/ml] | | | | | |
| G 111 | alive | 41 | 49,12 | 142,9 | 803,6 | 162,5 | 1,9 | 10,0 | 382,0 | 5,0 | 58,0 |
| G 60 | alive | 35 | 58,42 | 218,8 | 1225,9 | 171,2 | 6,8 | 6,0 | 372,0 | 5,0 | |
| G 208 | alive | 23 | 61,53 | 53,3 | 749,5 | 166,0 | | | | | |
| G 18 | alive | 42 | 50,68 | 169,9 | 1490,5 | 162,1 | 0,6 | 26,0 | 1336,0 | 31,0 | 15,0 |
| G 20 | alive | 33 | 46,94 | 188,0 | 1307,5 | 169,5 | 894,0 | 2,0 | | | |
| G 226 | alive | 21 | 38,22 | 439,6 | 2206,6 | 169,1 | 1098,0 | 1449,0 | 1215,0 | 5,0 | |
| G 14 | alive | 33 | 49,89 | 573,3 | 2936,7 | 346,9 | 1614,0 | 4596,0 | 414,0 | 5,0 | |
| G 88 | alive | 30 | 50,21 | 377,3 | 1912,4 | 165,8 | 52,8 | 17,0 | 532,0 | 5,0 | |
| G 116 | alive | 42 | 54,28 | 337,9 | 946,7 | 292,9 | 6,0 | 3,0 | 847,0 | 5,0 | 15,0 |
| G 13 | alive | 47 | 53,05 | 320,8 | 1689,5 | 170,6 | 191,4 | 4136,0 | 640,0 | 14,6 | 19,0 |
| G 87 | alive | 61 | 106,21 | 321,8 | | 172,5 | 9,1 | 37,0 | | | 11,0 |
| G 100 | alive | 45 | 44,87 | 337,7 | 1623,1 | 165,8 | 45,1 | 166,0 | 519,0 | 5,0 | 23,0 |
| G 119 | alive | 42 | | | | | 3,3 | 2,0 | | | 22,0 |
| G 57 | alive | 45 | 20,57 | 244,0 | 1105,6 | 163,3 | 8,0 | 30,0 | 1251,0 | 5,0 | 16,0 |
| G 98 | alive | 40 | 62,7 | 316,0 | 1736,5 | 196,2 | 0,5 | 2,0 | 690,0 | 5,0 | 23,0 |
| G 148 | dead | 22 | 65,86 | 346,9 | 1685,6 | 436,9 | 5,6 | 72,0 | 769,0 | 13,1 | 30,0 |
| G 103 | dead | 26 | 43,12 | 86,3 | 888,6 | 170,6 | 12,1 | 12,0 | 350,0 | 5,0 | |
| G 169 | dead | 9 | 42,43 | 357,4 | 950,6 | 194,4 | 57,3 | 1833,0 | 971,0 | 5,0 | 36,0 |
| G 182 | dead | 9 | 42,2 | 417,1 | 1673,9 | 166,4 | 1700,0 | 30,0 | 834,0 | 5,0 | |
| G 19 | dead | 6 | 35,28 | 199,9 | 1000,9 | 162,5 | 6,8 | 2,0 | 606,0 | 5,0 | 18,0 |
| G 196 | dead | 15 | 100,1 | 523,3 | 2265,7 | 342,5 | 279,9 | 1964,0 | 1301,0 | 5,0 | -99 |
| G 42 | dead | 25 | 15,7 | 244,2 | 927,3 | 237,5 | 171,0 | 667,0 | 510,0 | 5,0 | 20,0 |
| G 92 | dead | 30 | 65,6 | 203,5 | 1412,6 | 674,8 | 33,4 | 17,0 | 367,0 | 5,0 | 13,0 |
| G 52 | dead | 11 | 44,1 | 382,8 | 1979,0 | 297,7 | 39,2 | 1618,0 | 765,0 | 5,0 | 132,0 |
| G 33 | dead | 15 | 58,30 | 173,3 | 772,6 | 196,0 | 4,4 | 53,0 | 328,0 | 5,0 | |
| G 49 | dead | 11 | 43,8 | 574,3 | 2018,2 | 294,6 | 2050,0 | 5866,0 | 756,0 | 5,0 | |
| G 178 | dead | 11 | 43,4 | 318,3 | 2537,4 | 167,4 | 2952,0 | 1102,0 | 1072,0 | 5,0 | 36,0 |
| G 15 | dead | 16 | 44,18 | 337,4 | 1787,2 | 230,9 | 210,0 | 120,0 | | | 75,0 |
| G 79 | dead | 16 | 36,18 | 253,5 | 1241,5 | 162,1 | 25,5 | 37,0 | 492,0 | 5,0 | 22,0 |
| G 85 | dead | 18 | 40,48 | 382,8 | 1709,0 | 304,0 | 1620,0 | 2,0 | 1132,0 | 5,0 | 33,0 |
| G 96 | dead | 23 | 39,7 | 209,4 | -99 | 162,2 | 7050,0 | 90,0 | 354,0 | 17,5 | 13,0 |
| G 218 | dead | 18 | 45,80 | 240,7 | 1024,2 | 165,4 | 4,4 | 91,0 | 463,0 | 5,0 | |
| G 86 | dead | 13 | 63,19 | 484,0 | 1709,0 | 316,1 | 690,0 | 175,0 | 1235,0 | 5,0 | |
| G 192 | dead | 15 | 64,0 | 170,9 | 927,3 | 166,9 | 7,7 | 4,0 | 417,0 | 5,0 | 19,0 |
| G 152 | dead | 37 | 29,83 | 110,1 | 502,6 | 162,4 | 335,8 | 690,0 | | | 19,0 |

(continued)

| | Survival | Survival | EGFR | TIMP-1 | uPA | VEGF | CEA | CA 19-9 | IL2R | IL6 | Gastrin |
|---|---|---|---|---|---|---|---|---|---|---|---|
| ID | status | month | [ng/ml] | [ng/ml] | [pg/ml] | [pg/ml] | | | | | |
| G 73 | dead | 22 | 28,23 | 182,5 | 1284,2 | 163,0 | 11,1 | 217,0 | | | 12,0 |
| G 101 | dead | 35 | 61,34 | 258,8 | 556,5 | 165,2 | 12,1 | 17,0 | | | 16,0 |
| G 136 | dead | 15 | 83,26 | 227,8 | 1292,0 | 170,3 | 3112,0 | 1582,0 | 549,0 | 5,0 | |
| G 53 | dead | 7 | 56,78 | 494,9 | 1517,8 | 167,3 | 59,9 | 96,0 | 1299,0 | 9,2 | 16,0 |
| G 179 | dead | 14 | 55,2 | 336,8 | 1245,3 | 366,4 | 25,6 | 439,0 | 2285,0 | 5,0 | 33,0 |
| G 131 | dead | 58 | 56,0 | 130,8 | | | 5 | 12 | | | |
| G 138 | dead | 14 | 49,17 | 375,6 | 1237,6 | 278,7 | 46,4 | 9,0 | 744,0 | 5,0 | 46,0 |
| G 170 | dead | 24 | 41,07 | 148,1 | 425,6 | 169,4 | 16,6 | 32,0 | 381,0 | 5,0 | 23,0 |
| G 151 | dead | 28 | 47,85 | 260,1 | 1218,2 | 274,4 | 67,4 | 2,0 | 616,0 | 5,0 | |
| G 154 | dead | 14 | 60,69 | 150,7 | 931,2 | 162,1 | 441,8 | 2,0 | 524,0 | 5,0 | 31,0 |
| G 184 | dead | 28 | 37,7 | 417,1 | 718,6 | 192,6 | 7,6 | 57,0 | 433,0 | 5,0 | |
| G 173 | dead | 32 | 25,3 | 189,5 | 707,0 | 169,1 | 1,5 | 9,0 | 1058,0 | 5,0 | 26,0 |
| G 166 | dead | 7 | 42,60 | 198,1 | 1066,8 | 205,3 | 277,2 | 782,0 | 393,0 | 5,0 | 40,0 |

To insure comparability among the markers, the natural logarithm (base e) of each marker value was obtained.

Data Imputation

As many as 18% of values for any given predictor variable were missing in the dataset. Missing Value Analysis (SPSS Version 11) was performed on the log transforms of the assay variables. Based on an overall multiple regression model missing values were imputed for incomplete cases.

Cox Regression Model

A Cox Regression model was developed using the full data set with imputed values. Backward stepwise elimination produced a model with five covariates.
Table 5 presents exemplary results of a cox regression analysis using all variables including imputed data.

TABLE 5

Cox Regression Results

Regression Models Selected by Score Criterion

| Number of Variables | Score Chi-Square | Variables Included in Model |
|---|---|---|
| 1 | 3.4467 | MMP_2ng_ml_Mittelwert |
| 1 | 3.3318 | Final_Tumor_as_of_Original_Tum |
| 1 | 2.4987 | Collagen_VI_ng_ml_Mittelwert |
| 1 | 2.2177 | Gastrin |
| 1 | 1.9357 | PIIINP_ng_ml_Mittelwert |
| 1 | 1.3859 | Tenascin_ng_ml_Mittelwert |
| 1 | 0.7419 | Laminin_ng_ml_Mittelwert |
| 1 | 0.6592 | VEGF_pg_ml_Mittelwert |
| 1 | 0.5635 | TIMP_1_ng_ml_Mittelwert |
| 1 | 0.4845 | IL2R |
| 1 | 0.4121 | CEA |

(continued)

Cox Regression Results
Regression Models Selected by Score Criterion

| 1 | 0.2969 | Gender_1 |
|---|---|---|
| 1 | 0.2802 | IL6 |
| 1 | 0.2521 | TIMP_1_ng_ml_Mittelwert_1 |
| 1 | 0.1229 | COLLAGEN_IV_ng_ml_Mittelwert |
| 1 | 0.1203 | CA_19_9 |
| 1 | 0.1062 | Age_at_initial_diagnosis |
| 1 | 0.0940 | Her2_neu_ng_ml_Mittelwert |
| 1 | 0.0691 | uPA_pg_ml_Mittelwert |
| 1 | 0.0297 | EGFR_ng_ml_Mittelwert |
| | | |
| 2 | 13.3130 | PIIINP_ng_ml_Mittelwert TIMP_1_ng_ml_Mittelwert |
| 2 | 6.9550 | PIIINP_ng_ml_Mittelwert TIMP_1_ng_ml_Mittelwert_1 |
| 2 | 6.1894 | Laminin_ng_ml_Mittelwert TIMP_1_ng_ml_Mittelwert |
| 2 | 5.3721 | Collagen_VI_ng_ml_Mittelwert TIMP_1_ng_ml_Mittelwert |
| 2 | 5.2526 | MMP_2_ng_ml_Mittelwert TIMP_1_ng_ml_Mittelwert |
| 2 | 4.9809 | MMP_2ng_ml_Mittelwert Tenascin_ng_ml_Mittelwert |
| 2 | 4.9108 | Gastrin MMP_2_ng_ml_Mittelwert |
| 2 | 4.7721 | Collagen_VI_ng_ml_Mittelwert Gastrin |
| 2 | 4.6743 | Collagen_VI_ng_ml_Mittelwert Final_Tumor_as_of_Original_Tum |
| 2 | 4.4602 | IL6 MMP_2_ng_ml_Mittelwert |
| 2 | 4.4306 | COLLAGEN_IV_ng_ml_Mittelwert MMP_2_ng_ml_Mittelwert |
| 2 | 4.4006 | Final_Tumor_as_of_Original_Tum Tenascin ng_ml_Mittelwert |
| 2 | 4.3305 | Final_Tumor_as_of_Original_Tum TIMP_1_ng_ml_Mittelwert |
| 2 | 4.3235 | Final_Tumor_as_of_Original_Tum MMP_2ng_ml_Mittelwert |
| 2 | 4.3036 | Age_at_initial_diagnosis MMP_2_ng_ml_Mittelwert |
| 2 | 4.2922 | Final Tumor as_of_Original_Tum PIIINP_ng_ml_Mittelwert |
| 2 | 4.2883 | Age_at_initial_diagnosis Final Tumor as_of_Original _Tum |
| 2 | 4.2579 | PIIINP_ng_ml_Mittelwert uPA_pg_ml_Mittelwert |
| 2 | 4.1569 | MMP_2ng_ml_Mittelwert TIMP_1_ng_ml_Mittelwert 1 |
| 2 | 4.1331 | Final Tumor as_of_Original_Tum TIMP_1_ng_ml_Mittelwert_1 |
| | | |
| 3 | 17.1662 | Age at initial diagnosis PIIINP_ng_ml_Mittelwert TIMP_1_ng_ml_Mittelwert |
| 3 | 14.5799 | Final Tumor as_of_Original_Tum PIIINP_ng_ml_Mittelwert TIMP_1_ng_ml_Mittelwert |
| 3 | 14.4887 | Laminin_ng_ml_Mittelwert PIIINP_ng_ml_Mittelwert TIMP_1_ng_ml_Mittelwert |
| 3 | 14.0809 | PIIINP_ng_ml_Mittelwert TIMP_1ng_ml_Mittelwert VEGF_pg_ml_Mittelwert |
| 3 | 13.9596 | Gender_1 PIIINP_ng_ml_Mittelwert TIMP_1_ng_ml_Mittelwert |
| 3 | 13.8212 | CA_19_9 PIIINP_ng_ml_Mittelwert TIMP_1ml_Mittelwert |
| 3 | 13.8127 | PIIINP_ng_ml_Mittelwert TIMP_1_ng_ml_Mittelwert uPA_pg_ml_Mittelwert |
| 3 | 13.7812 | IL2R PIIINP_ng_ml_Mittelwert TIMP_1_ng_ml_Mittelwert |
| 3 | 13.7473 | COLLAGEN_IV_ng_ml_Mittelwert PIIINP_ng_ml_Mittelwert TIMP_1_ng_ml_Mittelwert |
| 3 | 13.6423 | Gastrin PIIINP_ng_ml_Mittelwert TIMP_1_ng_ml_Mittelwert |
| 3 | 13.5334 | MMP_2_ng_ml_Mittelwert PIIINP_ng_ml_Mittelwert TIMP_1_ng_ml_Mittelwert |
| 3 | 13.4361 | CEA PIIINP_ng_ml_Mittelwert TIMP_1_ng_ml_Mittelwert |
| 3 | 13.3831 | IL6 PIIINP_ng_ml_Mittelwert TIMP_1_ng_ml_Mittelwert |

In the analysis increases in TIMP-1 and GASTRIN are associated with increases in the risk for failure. Increases in the values of Tenascin, Collagen VI and UPA are associated with decreases in the risk for failure. The Wald statistic was used to determine the significance of each parameter estimate. The statistic is computed as

$$Wald = \left(\frac{B}{s.e.B}\right)^2$$

The statistic is distributed as a chi-square distribution with one degree of freedom.

Determination of Predictor Values By Cox Regression

The parameter estimates listed in Table 6A were used to calculate a predictor value Z for each patient. The predictor value algorithm is:

$$Z = 4.48\ln(TIMP-1) + 0.92\ln(GASTRIN) - 2.08\ln(TENASCIN) - 1.1\ln(CollagenVI) - 1.56\ln(UPA)$$

These values were used in a ROC analysis. Table 6B demonstrates the coordinates of the ROC curve. The area under the curve (AUC) for these data was 0.8 (95%CI: 0.67 to 0.94) which indicates a significant association with failure.
Tables 6A and 6B, which list Cox Regression Parameter estimates and ROC coordinates which were determined in accordance with the experiment of Example 2 herein.

Cox Regression Parameter estimates and ROC coordinates

Bifurcation and Kaplan Meier Analysis

Predictor values Z were bifurcated at a value of 8.62. Examination of Tables 6A and 6B indicates that at this value the true positive fraction (TPF) is 0.81 and the true negative fraction (TNF) is 0.6. Table 6B illustrates the Kaplan-Meier Survival curves for this cohort split at a Z of 8.62. A log-rank test indicates that these curves are significantly different. (LR=11.08, p=0.0009). The median survival for patients whose predictor value Z was below 8.62 (BC) was 58 months. For patients with values above this cut-point (UC) the median survival was 18 months.

TABLE 6A

| Cox Regression Parameter Estimates | | | |
|---|---|---|---|
| Variable | Parameter (B) | Wald Statistic | p |
| Ln(TIMP-1) | 4.48 | 13.9 | 0.000 |
| Ln(GASTRIN) | 0.94 | 5.46 | 0.019 |
| Ln(TENASCIN) | -2.08 | 5.22 | 0.022 |
| Ln(Collagen VI) | -1.10 | 5.21 | 0.022 |
| Ln(UPA) | -1.56 | 4.36 | 0.037 |

TABLE 6B
ROC Coordinates

| Z | TPF | TNF | TP | TN | FP | FN |
|---|---|---|---|---|---|---|
| 4.08 | 100.0% | 6.7% | 33 | 1 | 14 | 0 |
| 6.69 | 100.0% | 13.3% | 33 | 2 | 13 | 0 |
| 7.18 | 97.0% | 13.3% | 32 | 2 | 13 | 1 |
| 7.70 | 97.0% | 20.0% | 32 | 3 | 12 | 1 |
| 7.71 | 97.0% | 26.7% | 32 | 4 | 11 | 1 |
| Z | TPF | TNF | TP | TN | FP | FN |
| 7.85 | 97.0% | 33.3% | 32 | 5 | 10 | 1 |
| 8.09 | 97.0% | 40.0% | 32 | 6 | 9 | 1 |
| 8.12 | 93.9% | 40.0% | 31 | 6 | 9 | 2 |

(continued)

| Z | TPF | TNF | TP | TN | FP | FN |
|---|-----|-----|-----|-----|-----|-----|
| 8.13 | 90.9% | 40.0% | 30 | 6 | 9 | 3 |
| 8.17 | 90.9% | 46.7% | 30 | 7 | 8 | 3 |
| 8.18 | 90.9% | 53.3% | 30 | 8 | 7 | 3 |
| 8.19 | 87.9% | 53.3% | 29 | 8 | 7 | 4 |
| 8.51 | 84.8% | 53.3% | 28 | 8 | 7 | 5 |
| 8.53 | 84.8% | 60.0% | 28 | 9 | 6 | 5 |
| 8.62 | 81.8% | 60.0% | 27 | 9 | 6 | 6 |
| 8.72 | 78.8% | 60.0% | 26 | 9 | 6 | 7 |
| 8.73 | 75.8% | 60.0% | 25 | 9 | 6 | 8 |
| 8.77 | 75.8% | 66.7% | 25 | 10 | 5 | 8 |
| 8.79 | 75.8% | 73.3% | 25 | 11 | 4 | 8 |
| 8.82 | 72.7% | 73.3% | 24 | 11 | 4 | 9 |
| 8.84 | 69.7% | 73.3% | 23 | 11 | 4 | 10 |
| 8.87 | 66.7% | 73.3% | 22 | 11 | 4 | 11 |
| 8.91 | 66.7% | 80.0% | 22 | 12 | 3 | 11 |
| 8.91 | 63.6% | 80.0% | 21 | 12 | 3 | 12 |
| 9.20 | 60.6% | 80.0% | 20 | 12 | 3 | 13 |
| 9.27 | 60.6% | 86.7% | 20 | 13 | 2 | 13 |
| 9.32 | 57.6% | 86.7% | 19 | 13 | 2 | 14 |
| 9.56 | 54.5% | 86.7% | 18 | 13 | 2 | 15 |
| 9.60 | 51.5% | 86.7% | 17 | 13 | 2 | 16 |
| 9.65 | 48.5% | 86.7% | 16 | 13 | 2 | 17 |
| 9.72 | 45.5% | 86.7% | 15 | 13 | 2 | 18 |
| 9.77 | 42.4% | 86.7% | 14 | 13 | 2 | 19 |
| 9.79 | 42.4% | 93.3% | 14 | 14 | 1 | 19 |
| 9.86 | 39.4% | 93.3% | 13 | 14 | 1 | 20 |
| 9.87 | 36.4% | 93.3% | 12 | 14 | 1 | 21 |
| 10.01 | 33.3% | 93.3% | 11 | 14 | 1 | 22 |
| 10.16 | 30.3% | 93.3% | 10 | 14 | 1 | 23 |
| 10.29 | 30.3% | 100.0% | 10 | 15 | 0 | 23 |
| 10.30 | 27.3% | 100.0% | 9 | 15 | 0 | 24 |
| 10.32 | 24.2% | 100.0% | 8 | 15 | 0 | 25 |
| 10.36 | 21.2% | 100.0% | 7 | 15 | 0 | 26 |
| 10.40 | 18.2% | 100.0% | 6 | 15 | 0 | 27 |
| 10.41 | 15.2% | 100.0% | 5 | 15 | 0 | 28 |
| 10.47 | 12.1% | 100.0% | 4 | 15 | 0 | 29 |
| 11.00 | 9.1% | 100.0% | 3 | 15 | 0 | 30 |
| 11.47 | 6.1% | 100.0% | 2 | 15 | 0 | 31 |
| 12.00 | 3.0% | 100.0% | 1 | 15 | 0 | 32 |
| 12.19 | 0.0% | 100.0% | 0 | 15 | 0 | 33 |

Kaplan Meier Analysis of singular and combined marker sets

For each singular marker cut-off, values were determined as set forth in Tables 4A and 4B. Subsequently, Kaplan Meier Analysis was performed for each of the singular markers. As depicted in FIGS. 2-7, this partitioning into "Below the cut-off point" ("BC")(which was set as the numerical value "0") and into "Above the cut-off point " ("UC")(which was set as the numerical value "1"), allowed bifurcation and statistical significant discrimination of patients with good and bad clinical outcome (i.e. overall survival time).

Table 5 presents the results of the single parameter Kaplan Meier Analysis by using the Cut-off values for each of the selected markers of Table 1.

As depicted in the FIGS 2-8, Gastrin, CA19-9, TIMP-1 (Immuno-1), MMP-2 and EGFR yielded statistically significant results at a level of p=0.05 for the indicated threshold values. VEGF and CEA did show a trend towards statistical significance at a level of 0.08 for the indicated threshold values. As shown in Table 1, the indicated cut-off values of each of the individual markers were transformed in the numerical values 1 or 0, depending on whether the individual measurements were above or below the cut-off value, respectively.

These values were used to develop simple algorithms based on dichotomous parameters. As set forth in Table 7, an exemplary algorithm "MCT-V" (row I) was derived by addition of the dichotomous values of MMP-2 (row L), Collagen VI (row N) and Tenascin (row P) and subtraction of the dichotomous value of VEGF (row R). Sum values were then used for partitioning into two groups ("UC" > 1 and "BC<1"), and Kaplan Meier analysis was subsequently employed.

Table 7 depicts the assessment of the MCT-V Algorithm values.

TABLE 7

| Combinatorial Analysis of dichotomous parameters | | | | | | | | | | |
|---|---|---|---|---|---|---|---|---|---|---|
| | | "MCT-V" | Survival | CutOff 674 | | CutOff 7,2 | | CutOff 1083 | | >Mean 221,1 | |
| | | Algorithm | Month | MMP-2 | | Collagen VI | | Tenascin | | VEGF | |
| ID | Survival | | | [ng/ml] | | [ng/ml] | | [ng/ml] | | [pg/ml] | |
| G 111 | 0 | 1 | 41 | 540,9 | 0 | 8,4 | 1 | 358,3 | 0 | 162,5 | 0 |
| G 60 | 0 | 0 | 35 | 553,7 | 0 | 7,1 | 0 | 533,0 | 0 | 171,2 | 0 |
| G 208 | 0 | 1 | 23 | 1128,7 | 1 | 5,5 | 0 | 287,1 | 0 | 166,0 | 0 |
| G 18 | 0 | 1 | 42 | 819,3 | 1 | | 0 | | 0 | 162,1 | 0 |
| G 20 | 0 | 0 | 33 | 416,4 | 0 | 4,3 | 0 | 323,4 | 0 | 169,5 | 0 |
| G 226 | 0 | 0 | 21 | 432,6 | 0 | 6,0 | 0 | 774,5 | 0 | 169,1 | 0 |
| G 14 | 0 | 0 | 33 | 483,9 | 0 | 5,7 | 0 | 1122,8 | 1 | 346,9 | 1 |
| G 88 | 0 | 0 | 30 | 520,9 | 0 | 5,7 | 0 | 470,5 | 0 | 165,8 | 0 |
| G 116 | 0 | -1 | 42 | 554,9 | 0 | 5,0 | 0 | 973,0 | 0 | 292,9 | 1 |
| G 13 | 0 | 2 | 47 | 541,5 | 0 | 7,7 | 1 | 2199,3 | 1 | 170,6 | 0 |
| G 87 | 0 | 3 | 61 | 1620,1 | 1 | 22,0 | 1 | 2364,7 | 1 | 172,5 | 0 |
| G 100 | 0 | 2 | 45 | 1079,7 | 1 | 17,0 | 1 | 1021,9 | 0 | 165,8 | 0 |
| G 119 | 0 | 1 | 42 | 920,0 | 1 | | 0 | | 0 | | 0 |
| G 57 | 0 | 1 | 45 | 817,4 | 1 | 4,4 | 0 | 175,4 | 0 | 163,3 | 0 |
| G 98 | 0 | 2 | 40 | 838,1 | 1 | 9,9 | 1 | 821,4 | 0 | 196,2 | 0 |
| G 148 | 1 | -1 | 22 | 464,4 | 0 | 4,9 | 0 | 979,4 | 0 | 436,9 | 1 |
| G 103 | 1 | 2 | 26 | 757,2 | 1 | 8,4 | 1 | 315,5 | 0 | 170,6 | 0 |
| G 169 | 1 | 0 | 9 | 428,8 | 0 | 4,7 | 0 | 276,6 | 0 | 194,4 | 0 |
| G 182 | 1 | 0 | 9 | 465,5 | 0 | 6,6 | 0 | 851,1 | 0 | 166,4 | 0 |
| G 19 | 1 | 0 | 6 | 542,6 | 0 | 5,9 | 0 | 728,2 | 0 | 162,5 | 0 |
| G 196 | 1 | -1 | 15 | 438,7 | 0 | 5,1 | 0 | 750,0 | 0 | 342,5 | 1 |
| G 42 | 1 | 0 | 25 | 495,0 | 0 | 8,2 | 1 | 842,7 | 0 | 237,5 | 1 |
| G 92 | 1 | 0 | 30 | 619,0 | 0 | 5,3 | 0 | 1150,8 | 1 | 674,8 | 1 |
| G 52 | 1 | -1 | 11 | 564,0 | 0 | 6,2 | 0 | 739,0 | 0 | 297,7 | 1 |
| G 33 | 1 | 0 | 15 | 611,2 | 0 | 6,0 | 0 | 410,3 | 0 | 196,0 | 0 |
| G 49 | 1 | 0 | 11 | 643,0 | 0 | 5,7 | 0 | 2201,7 | 1 | 294,6 | 1 |

(continued)

| ID | Survival | "MCT-V" Algorithm | Survival Month | CutOff 674 MMP-2 [ng/ml] | | CutOff 7,2 Collagen VI [ng/ml] | | CutOff 1083 Tenascin [ng/ml] | | >Mean 221,1 VEGF [pg/ml] | |
|---|---|---|---|---|---|---|---|---|---|---|---|
| G 178 | 1 | 0 | 11 | 451,3 | 0 | 4,6 | 0 | 829,3 | 0 | 167,4 | 0 |
| G 15 | 1 | 0 | 16 | 1173,4 | 1 | | 0 | | 0 | 230,9 | 1 |
| G 79 | 1 | 0 | 16 | 571,7 | 0 | 6,7 | 0 | 492,8 | 0 | 162,1 | 0 |
| G 85 | 1 | 0 | 18 | 522,7 | 0 | 4,1 | 0 | 1128,5 | 1 | 304,0 | 1 |
| G 96 | 1 | 0 | 23 | 326,8 | 0 | 2,3 | 0 | 577,6 | 0 | 162,2 | 0 |
| G 218 | 1 | 1 | 18 | 767,5 | 1 | 4,0 | 0 | 577,0 | 0 | 165,4 | 0 |
| G 86 | 1 | 0 | 13 | 753,2 | 1 | 7,2 | 0 | 849,7 | 0 | 316,1 | 1 |
| G 192 | 1 | 0 | 15 | 577,9 | 0 | 3,9 | 0 | 331,5 | 0 | 166,9 | 0 |
| G 152 | 1 | 0 | 37 | 313,1 | 0 | 2,4 | 0 | 315,0 | 0 | 162,4 | 0 |
| G 73 | 1 | 0 | 22 | 591,6 | 0 | 2,4 | 0 | 678,0 | 0 | 163,0 | 0 |
| G 101 | 1 | 1 | 35 | 662,7 | 0 | 10,7 | 1 | 341,9 | 0 | 165,2 | 0 |
| G 136 | 1 | 0 | 15 | 588,0 | 0 | 5,0 | 0 | 327,5 | 0 | 170,3 | 0 |
| G 53 | 1 | 1 | 7 | 518,3 | 0 | 10,0 | 1 | 817,4 | 0 | 167,3 | 0 |
| G 179 | 1 | -1 | 14 | 460,0 | 0 | 5,8 | 0 | 563,9 | 0 | 366,4 | 1 |
| G 131 | 1 | 1 | 58 | 790,2 | 1 | | 0 | 448,7 | 0 | | 0 |
| G 138 | 1 | -1 | 14 | 599,3 | 0 | 5,1 | 0 | 931,0 | 0 | 278,7 | 1 |
| G 170 | 1 | 0 | 24 | 611,2 | 0 | 5,0 | 0 | 288,9 | 0 | 169,4 | 0 |
| G 151 | 1 | -1 | 28 | 599,2 | 0 | 5,9 | 0 | 853,3 | 0 | 274,4 | 1 |
| G 154 | 1 | 1 | 14 | 765,6 | 1 | 6,6 | 0 | 489,5 | 0 | 162,1 | 0 |
| G 184 | 1 | 0 | 28 | 527,1 | 0 | 5,8 | 0 | 315,9 | 0 | 192,6 | 0 |
| G 173 | 1 | 0 | 32 | 395,0 | 0 | 3,3 | 0 | 454,6 | 0 | 169,1 | 0 |
| G 166 | 1 | 0 | 7 | 486,7 | 0 | 2,7 | 0 | 234,6 | 0 | 205,3 | 0 |

TABLE 8

| Comparison of Survival Curves (survival month/percent survival) | | | | | | | | | |
|---|---|---|---|---|---|---|---|---|---|
| | Gastrin CO 25,4 | CA 19-9 CO 37 | TIMP-1 Immuno CO 1037,6 | MMP-2 CO 675 | EGFr CO 45 | VEG F CO 221,1 | CEA CO 100 | MMP 2 Col6 Tenas cin - VEG F | TIMP-1 CO 949 and EGFr CO 45 |
| **Logrank Test** | | | | | | | | | |
| Chi square | 7.237 | 7.485 | 6.757 | 5.208 | 3.896 | 3.279 | 3.052 | 10.75 | 4.557 |
| df | 1 | 1 | 1 | 1 | 1 | 1 | 1 | 1 | 1 |
| P value | 0.0071 | 0.0062 | 0.0093 | 0.0225 | 0.0484 | 0.070 2 | 0.0806 | 0.001 0 | 0.0328 |
| P value summary | ** | ** | ** | * | * | ns | ns | ** | * |
| Are survival curves significantly different? | Yes | Yes | Yes | Yes | Yes | No | No | Yes | Yes |
| **Median survival** | | | | | | | | | |
| | Gastrin high | CA 19-9 high | TIMP-1 Immuno high | MMP2>6 75 | EGFr<4 5 | VEG F high | CEA>100 | MCT-V high | TIMP-1 high and EGFr low |
| Data 1: | 14.00 | 16.00 | 14.00 | 58.00 | 22.00 | 17.00 | 16.00 | 58.00 | 11.00 |
| | Gastrin low | CA 19-9 low | TIMP-1 Immuno low | MMP2<6 75 | EGFr>4 5 | VEG F low | CEA<100 | MCT-V low | TIMP-1 low and/or EGFr normal |
| Data 1: | 30.00 | 35.00 | 30.00 | 22.00 | 30.00 | 28.00 | 30.00 | 18.00 | 28.00 |
| Ratio | 0.4667 | 0.4571 | 0.4667 | 2.636 | 0.7333 | 0.607 1 | 0.5333 | 3.222 | 0.3929 |
| 95% CI of ratio | 0.01269 to 0.9206 | -0.04426 to 0.9585 | - 0.03078 to 0.9641 | 2.223 to 3.049 | 0.2294 to 1.237 | 0.115 1 to 1.099 | 0.03946 to 1.027 | 2.809 to 3.635 | - 0.04119 to 0.8269 |
| **Hazard Ratio** | | | | | | | | | |
| Ratio | 2.716 | 2.443 | 2.362 | 0.3913 | 1.928 | 1.865 | 1.821 | 0.275 3 | 2.346 |
| 95% CI of ratio | 1.440 to 10.18 | 1.342 to 5.936 | 1.327 to 7.509 | 0.1967 to 0.8839 | 1.005 to 4.282 | 0.938 6 to 4.964 | 0.9164 to 4.571 | 0.140 2 to 0.609 9 | 1.109 to 11.32 |

| | Gastrin high / Gastrin low | CA 19-9 high / CA 19-9 low | TIMP-1 Immuno high I TIMP-1 Immuno low | MMP2>6 75/ MMP2<6 75 | EGFr<4 5/ EGFr>4 5 | VEG F high / VEG Flow | CEA>100 / CEA<100 | MCT-V high / MCT-V low | TIMP-1 high and EGFr low / TIMP-1 low and/or EGFr normal |
|---|---|---|---|---|---|---|---|---|---|
| **Number or rows** | 48 48 | 48 48 | 48 48 | 48 48 | 48 48 | 48 48 | 48 48 | 48 48 | 48 48 |
| # of blank lines | 36 25 | 25 23 | 31 17 | 35 13 | 26 22 | 34 14 | 31 17 | 33 15 | 39 9 |
| # of rows with impossible data | 0 0 | 0 0 | 0 0 | 0 0 | 0 0 | 0 0 | 0 1 | 0 0 | 0 0 |
| # censored subjects | 1 9 | 4 11 | 4 11 | 7 8 | 4 11 | 2 13 | 4 12 | 9 6 | 2 13 |
| # deaths / events | 11 14 | 19 14 | 13 20 | 6 27 | 18 15 | 12 21 | 13 19 | 6 27 | 7 26 |
| **Median survival** | 14 30 | 16 35 | 14 30 | 58 22 | 22 30 | 17 28 | 16 30 | 58 18 | 11 28 |

FIGS 10 and 10A depict the Kaplan Meier Analysis of the respective "MCT-V" algorithm values.

Multiple statistical tests

Serum data were also transformed for analysis in Genedata Expressionist™ software. The patient population has been divided in either in "responders" and "non responders" as depicted in row D or "survivors (survival of greater than 40 month)" and "non-survivors (dead within 18 month)" as depicted in row G.. Subsequently, multiple statistical tests have been performed by using T-Test, Welch, Kologorov-Smirnov and Wilcoxon statistical techniques. Resulting p values for the respective statistical tests are displayed.

Table 9 displays the results of multiple statistical testing to discriminate patients with metastatic CRC surviving for more than 40 month or less than 18 month since primary treatment by assessing serum parameters.

TABLE 9

| Multiple Statistical Tests - Overall Survival Analysis | | | | | |
|---|---|---|---|---|---|
| CEA | 0.005 | 0.003 | 0.044 | 0.007 | 1 |
| cm2 pre-therapy | 0.025 | 0.030 | 0.013 | 0.022 | 2 |
| Collagen VI | 0.008 | 0.078 | 0.011 | 0.043 | 3 |
| MMP-2 | 0.011 | 0.049 | 0.030 | 0.039 | 4 |
| Gastrin | 0.054 | 0.046 | 0.053 | 0.059 | 5 |
| TIMP-1 | 0.154 | 0.127 | 0.116 | 0.147 | 6 |
| CA 19-9 | 0.167 | 0.181 | 0.266 | 0.176 | 7 |
| Laminin | 0.236 | 0.281 | 0.430 | 0.275 | 8 |
| VEGF | 0.221 | 0.160 | 0.609 | 0.318 | 9 |
| Tenascin | 0.326 | 0.457 | 0.160 | 0.231 | 10 |
| PIIINP | 0.275 | 0.401 | 0.617 | 0.417 | 11 |
| TIMP-1 | 0.581 | 0.537 | 0.193 | 0.422 | 12 |
| IL6 | 0.190 | 0.448 | 0.996 | 0.380 | 13 |
| uPA | 0.609 | 0.614 | 0.877 | 0.605 | 14 |
| EGFr | 0.959 | 0.970 | 0.433 | 0.524 | 15 |
| IL2R | 0.635 | 0.600 | 0.882 | 0.687 | 16 |
| Her-2/neu | 0.764 | 0.709 | 0.816 | 1.000 | 17 |
| Collagen_IV_A1 | 0.87949997 | 0.89459997 | 0.8664 | 0.93790001 | 18 |

Respective p-values are indicated for each of the markers measured. Rank sum test has been performed to choose optimal markers for subsequent analysis such as principal component analysis. As indicated CEA, initial tumor size, Collagen VI, MMP-2 and Gastrin were statistically significant to discriminate between "survivors" and "non-survivors" by using the diverse statistical test for analyzing the continuous variables.

FIG. 10 and FIG 10A displays the initial partitioning into two groups when using all 17 parameters of Table 9. "Survivors" are displayed as green balls and "non-survivors" are displayed as red balls.

FIG. 11 and FIG. 11A display the improved partitioning into two groups by Principal Component Analysis (PCA) when using the Top 5 discriminating parameters (i.e. CEA, initial tumor size, Collagen VI, MMP-2 and Gastrin) depicted in Table 9. "Survivors" are displayed as green balls and "non-survivors" are displayed as red balls.

EXAMPLE 3

Expression analysis of primary and metastatic tumor tissue by analysis of paraffin-embedded tumor tissue

Summary

Paraffin embedded, Formalin-fixed tissues of surgical resectates of patient as described in Example 1 were analyzed and neoplastic disease marker level values were determined by qRT-PCR techniques and correlated with patient survival.

Expression profiling utilizing quantitative kinetic RT-PCR

RNA was isolated from paraffin-embedded, formalin-fixed tissues (= FFPE tissues). Those skilled in the art are able to perform RNA extraction procedures. For example, total RNA from a 5 to 10 μm curl of FFPE tumor tissue can be extracted using the High Pure RNA Paraffin Kit (Roche, Basel, Switzerland), quantified by the Ribogreen RNA Quantitation Assay (Molecular Probes, Eugene, OR) and qualified by real-time fluorescence RT-PCR of a fragment of RPL37A. In general 0.5 to 2 ng RNA of each qualified RNA extraction was assayed by qRT-PCR as described below. For a detailed analysis of gene expression by quantitative PCR methods, one will utilize primers flanking the genomic region of interest and a fluorescent labeled probe hybridizing in-between. Using the PRISM 7700 or 7900 Sequence Detection System of PE Applied Biosystems (Perkin Elmer, Foster City, CA, USA) with the technique of a fluorogenic probe, consisting of an oligonucleotide labeled with both a fluorescent reporter dye and a quencher dye, one can perform such a expression measurement. Amplification of the probe-specific product causes cleavage of the probe, generating an increase in reporter fluorescence. Primers and probes were selected using the Primer Express software and localized mostly across exon/intron borders and large intervening non-transcriped sequences (> 800 bp) to guarantee RNA-specificity or within the 3' region of the coding sequence or in the 3' untranslated region. Primer design and selection of an appropriate target region is well known to those with skills in the art. Predefined primer and probes for the genes listed in Table 2 can also be obtained from suppliers e.g. PE Applied Biosystems. All primer pairs were checked for specificity by conventional PCR reactions and gel electrophoresis. To standardize the amount of sample RNA, GAPDH, RPL37A, RPL9 and CD63 were selected as references, since they were not differentially regulated in the samples analyzed. To perform such an expression analysis of genes within a biological samples the respective primer/probes are prepared by mixing 25 μl of the 100 μM stock solution "Upper Primer", 25 μl of the 100 μM stock solution "Lower Primer" with 12,5 μl of the 100 μM stock solution TaqMan-probe (FAM/Tamra) and adjusted to 500 μl with aqua dest (Primer/probe-mix). For each reaction 1,25 μl cDNA of the patient samples were mixed with 8,75 μl nuclease-free water and added to one well of a 96 Well-Optical Reaction Plate (Applied Biosystems Part No. 4306737). 1,5 μl of the Primer/Probe-mix described above, 12,5μl Taq Man Universal-PCR-mix (2x) (Applied Biosystems Part No. 4318157) and 1 μl Water are then added. The 96 well plates are closed with 8 Caps/Strips (Applied Biosystems Part Number 4323032) and centrifuged for 3 minutes. Measurements of the PCR reaction are done according to the instructions of the manufacturer with a TaqMan 7700 from Applied Biosystems (No. 20114) under appropriate conditions (2 min. 50°C, 10 min. 95°C, 0.15min. 95°C, 1 min. 60°C; 40 cycles). Prior to the measurement of so far unclassified biological samples control experiments will e.g. cell lines, healthy control samples, samples of defined therapy response could be used for standardization of the experimental conditions.

TaqMan validation experiments were performed showing that the efficiencies of the target and the control amplifications are approximately equal which is a prerequisite for the relative quantification of gene expression by the comparative ΔΔCT method, known to those with skills in the art. Herefore the softwareSDS 2.0 from Applied Biosystems can be used according to the respective instructions. CT-values are then further analyzed with appropriate software (Microsoft Excel™) of statistical software packages (SAS).

As well as the technology described above, provided by Perkin Elmer, one may use other technique implementations like Lightcycler™ from Roche Inc. or iCycler from Stratagene Inc.capable of real time detection of an RT-PCR reaction.

FIG. 12 and FIG. 12A displays the relative expression of the ERB receptor tyrosine kinase family members in FFPE tissues from primary tumor resectates of patients as described in Example 1 and as determined by qRT-PCR profiling. Genes are displayed in lines. Survival of patients is depicted above each row, with 1 or 0 meaning "dead" or "alive" and the numbers in brackets meaning month of survival since primary diagnosis.

As depicted, expression of EGFR family members correlates with clinical response of liver metastasis of CRC patients being treated with 5'FU based regimen as determined by CT determinations of the metastatic lesions. Clinical Response is denoted as "Partial Response" (= PR or green color bar on top), "Stable Disease" (= SD or orange color bar on top) and "Progressive Disease" (= PD or dark red color bar on top). Survival is depicted for each patient above each column (survival = 0 or death =1 followed by month of survival in brackets [x month]). Clearly overexpression of at least one ERB family member is evident in the bad prognosis group, i.e. the non responding SD and PD patient cohort. Particularly high expression of EGFR in the primary tumor correlates with non-favorable response to antitumor treatment. This was further demonstrated by doing multiple statistical tests as depicted in Table 10 (independent of normalization method).

Table 10 displays the results of multiple statistical testing to discriminate patients with metastatic CRC whose metastatic lesions respond to 5' FU based regimen (Partial Response) or do not respond (Stable Disease and Progressive Disease) by determining RNA of EGFR family member in FFPE tissue samples.

TABLE 10

Multiple Statistical Tests - Clinical Response - FFPE Analysis of ERB family members

| Gene Name | Gene Description | T-Test | Welch | Kolmogorov-Smirnov | Wilcoxon | Rank Sum |
|---|---|---|---|---|---|---|
| Her2/neu | normalized to mean of RPL37A | 0.01977 | 0.02106 | 0.05303 | 0.03788 | 1 |
| EGFR | normalized to mean of RPL37A | 0.02762 | 0.02805 | 0.05303 | 0.02622 | 2 |
| EGFR II | normalized to mean of RPL37A, GAPDH, RPL9, CD63 | 0.0397 | 0.03977 | 0.2121 | 0.05303 | 3 |
| EGFR I | normalized to mean of GAPDH | 0.05634 | 0.05636 | 0.2121 | 0.09732 | 4 |
| Her2/neu I | normalized to mean of GAPDH | 0.15549999 | 0.1556 | 0.05303 | 0.07284 | 5 |
| ERBB3 II | normalized to mean of RPL37A, GAPDH, RPL9, CD63 | 0.0906 | 0.09065 | 0.2121 | 0.12819999 | 6 |
| ERBB3 | normalized to mean of RPL37A | 0.06432 | 0.06656 | 0.57520002 | 0.2243 | 7 |
| Her2/neu II | normalized to mean of RPL37A, GAPDH, RPL9, CD63 | 0.083 | 0.08317 | 0.57520002 | 0.1649 | 8 |
| VEGF-C I | normalized to mean of GAPDH | 0.22149999 | 0.2237 | 0.2121 | 0.21969999 | 9 |
| VEGF-C II | normalized to mean of RPL37A, GAPDH, RPL9, CD63 | 0.2326 | 0.235 | 0.2121 | 0.1373 | 10 |
| VEGF-C | normalized to mean of RPL37A | 0.23989999 | 0.243 | 0.2121 | 0.1543 | 11 |

The high mRNA expression of EGFR in primary tumors of bad prognosis patients contrasts the low serum level of EGFr in serum of bad prognosis patients. However, as the EGFr and TIMP-1 serum levels were simultaneously high in bad prognosis patients, the comparatively low levels of serum EGFr apparently reflect the reduced degradation of EGFr by proteinases rather than reduced expression within the tumor tissue, which are surprisingly elevated. This is of critical importance for therapeutic strategies targeted anti EGF receptor family members (like e.g. Iressa®, Erbitux® or Herceptin®), which are unexpectedly in particular useful in patients with low levels of serum EGFr. In addition, according to the data depicted in FIG. 12 and FIG. 12A, the organization of the ERB family member network is of pivotal importance for the clinical outcome. Colorectal tumors expressing high levels of EGFR and simultaneously low levels of Her-2/neu do have a significantly shorter overall survival, than patients with high EGFR and Her-2/neu levels. This seems to reflect very different biological impacts of hetero- or homodimerized ERB receptors on tumorigenesis and clinical outcome of anti cancer therapies. Putatively, the composition of the ERB network influences inter alias proliferation rate thereby being of major importance for anti proliferative chemotherapeutic agents such as 5' FU based regimens. This would explain in part the surprising finding, that Her-2/neu positive CRC tumors do have a better prognosis than Her-2/neu negative tumors.

In line with this, the combined analysis of TIMP-1 and EGFr in pretreatment serum samples did identify a high risk population of patients with high TIMP-1 and low EGFr levels, which exhibited worse outcome (overall survival of 11 month) compared to single parameter assessment.

Table 11 displays experimental data as determined by duplicate or triplicate measurements for TIMP-1 and EGFr in the pretreatment serum sample and combined analysis thereof.

TABLE 11

Serum Data of TIMP-1 and EGFr

| ID | Age at diagnosis | Response | Response | Survival status | Survival [Month]l | Survival | TIMP-1 high and EGFR low | TIMP-1 [ng/ml] | | EGFR [ng/ml] | |
|---|---|---|---|---|---|---|---|---|---|---|---|
| G 111 | 39 | SD | 0 | alive | 41 | 0 | 0 | 468,7 | 0 | 49,12 | 0 |
| G 60 | 60 | SD | 0 | alive | 35 | 0 | 0 | 665,8 | 0 | 58,42 | 0 |
| G 208 | 62 | -99 | 0 | alive | 23 | 0 | 0 | 471,6 | 0 | 61,53 | 0 |
| G 18 | 63 | SD | 0 | alive | 42 | 0 | 0 | 653,8 | 0 | 50,68 | 0 |
| G 20 | 63 | SD | 0 | alive | 33 | 0 | 0 | 648,3 | 0 | 46,94 | 0 |
| G 226 | 72 | PD | 0 | alive | 21 | 0 | 1 | 1242,2 | 1 | 38,22 | 1 |
| G 14 | 43 | PR | 1 | alive | 33 | 0 | 0 | 1897,2 | 1 | 49,89 | 0 |
| G 88 | 50 | PR | 1 | alive | 30 | 0 | 0 | 1085,7 | 1 | 50,21 | 0 |
| G 116 | 52 | PR | 1 | alive | 42 | 0 | 0 | 917,5 | 0 | 54,28 | 0 |
| G 13 | 60 | PR | 1 | alive | 47 | 0 | 0 | 1022,9 | 0 | 53,05 | 0 |
| G 87 | 60 | CR | 1 | alive | 61 | 0 | 0 | 848,7 | 0 | 106,21 | 0 |
| G 100 | 61 | PR | 1 | alive | 45 | 0 | 1 | 1528,4 | 1 | 44,87 | 1 |
| G 119 | 67 | PR | 1 | alive | 42 | 0 | 0 | 640,6 | 0 | | 0 |
| G 57 | 71 | PR | 1 | alive | 45 | 0 | 0 | 639,7 | 0 | 20,57 | 1 |
| G 98 | 71 | PR | 1 | alive | 40 | 0 | 0 | 821,9 | 0 | 62,7 | 0 |
| G 148 | 34 | SD | 0 | dead | 22 | 1 | 0 | 1420,2 | 1 | 65,86 | 0 |
| G 103 | 52 | SD | 0 | dead | 26 | 1 | 0 | 502,7 | 0 | 43,12 | 1 |
| G 169 | 55 | SD | 0 | dead | 9 | 1 | 1 | 1220,0 | 1 | 42,43 | 1 |
| G 182 | 59 | SD | 0 | dead | 9 | 1 | 1 | 1580,0 | 1 | 42,2 | 1 |
| G 19 | 61 | SD | 0 | dead | 6 | 1 | 0 | 671,1 | 0 | 35,28 | 1 |
| G 196 | 61 | SD | 0 | dead | 15 | 1 | 0 | 1387,6 | 1 | 100,1 | 0 |
| G 42 | 62 | SD | 0 | dead | 25 | 1 | 0 | 728,6 | 0 | 15,7 | 1 |
| G 92 | 63 | -99 | 0 | dead | 30 | 1 | 0 | 765,2 | 0 | 65,6 | 0 |

(continued)

| | | | | | | | | | | | | |
|---|---|---|---|---|---|---|---|---|---|---|---|---|
| Serum Data of TIMP-1 and EGFr | | | | | | | | | | | | |
| | Age at | | | Survival | Survival | | TIMP-1 high | TIMP-1 | | EGFR | |
| ID | diagnosis | Response | Response | status | [Month]l | Survival | and EGFR low | [ng/ml] | | [ng/ml] | |
| G 52 | 66 | SD | 0 | dead | 11 | 1 | 1 | 1381,1 | 1 | 44,1 | 1 |
| G 33 | 70 | SD | 0 | dead | 15 | 1 | 0 | 658,4 | 0 | 58,30 | 0 |
| G 49 | 70 | -99 | 0 | dead | 11 | 1 | 1 | 2020,1 | 1 | 43,8 | 1 |
| G 178 | 70 | SD | 0 | dead | 11 | 1 | 1 | 1523,5 | 1 | 43,4 | 1 |
| G 15 | 74 | SD | 0 | dead | 16 | 1 | 1 | 1193,5 | 1 | 44,18 | 1 |
| G 79 | 43 | PR | 1 | dead | 16 | 1 | 0 | 835,1 | 0 | 36,18 | 1 |
| G 85 | 46 | PR | 1 | dead | 18 | 1 | 1 | 1297,0 | 1 | 40,48 | 1 |
| G 96 | 46 | PR | 1 | dead | 23 | 1 | 0 | 741,8 | 0 | 39,7 | 1 |
| G 218 | 51 | CR | 1 | dead | 18 | 1 | 0 | 805,6 | 0 | 45,80 | 0 |
| G 86 | 57 | PR | 1 | dead | 13 | 1 | 0 | 1801,1 | 1 | 63,19 | 0 |
| G 192 | 57 | PR | 1 | dead | 15 | 1 | 0 | 553,7 | 0 | 64,0 | 0 |
| G 152 | 58 | PR | 1 | dead | 37 | 1 | 0 | 461,5 | 0 | 29,83 | 1 |
| G 73 | 59 | PR | 1 | dead | 22 | 1 | 0 | 623,9 | 0 | 28,23 | 1 |
| G 101 | 59 | PR | 1 | dead | 35 | 1 | 0 | 720,3 | 0 | 61,34 | 0 |
| G 136 | 59 | PR | 1 | dead | 15 | 1 | 0 | 852,3 | 0 | 83,26 | 0 |
| G 53 | 61 | PR | 1 | dead | 7 | 1 | 0 | 1882,2 | 1 | 56,78 | 0 |
| G 179 | 62 | PR | 1 | dead | 14 | 1 | 0 | 1068,0 | 1 | 55,2 | 0 |
| G 131 | 64 | PR | 1 | dead | 58 | 1 | 0 | 587,4 | 0 | 56,0 | 0 |
| G 138 | 66 | PR | 1 | dead | 14 | 1 | 0 | 1159,4 | 1 | 49,17 | 0 |
| G 170 | 66 | PR | 1 | dead | 24 | 1 | 0 | 506,5 | 0 | 41,07 | 1 |
| G 151 | 67 | PR | 1 | dead | 28 | 1 | 0 | 919,0 | 0 | 47,85 | 0 |
| G 154 | 70 | PR | 1 | dead | 14 | 1 | 0 | 515,0 | 0 | 60,69 | |
| G 184 | 72 | PR | 1 | dead | 28 | 1 | 0 | 566,5 | 0 | 37,7 | 1 |

(continued)

Serum Data of TIMP-1 and EGFr

| ID | Age at diagnosis | Response | Response | Survival status | Survival [Month]l | Survival | TIMP-1 high and EGFR low | TIMP-1 [ng/ml] | TIMP-1 | EGFR [ng/ml] | EGFR |
|---|---|---|---|---|---|---|---|---|---|---|---|
| G 173 | 73 | PR | 1 | dead | 32 | 1 | 0 | 570,4 | 0 | 25,3 | 1 |
| G 166 | 75 | PR | 1 | dead | 7 | 1 | 0 | 511,7 | 0 | 42,60 | 1 |

FIG. 13 illustrates Kaplan-Meier survival curves of combined analysis of serum levels of TIMP-1 and EGFr

EXAMPLE 4

Expression analysis of primary and metastatic tumor tissue by analysis of fresh tumor tissue biopsies

Summary

Biopsies of patient as described in Example 1 were analyzed and genome wide expression analysis was performed by array technologies and correlated with patient survival.
Probes specific to the polynucleotide sequences of Table 2 and Table 11 are obtained as follows.
Polynucleotide probes are immobilized on a DNA chip in an organized array. Oligonucleotides can be bound to a solid support by a variety of processes, including lithography. For example a chip can hold up to 410,000 oligonucleotides (GeneChip, Affymetrix).
A biological sample (e.g., a biopsy sample which is optionally fractionated by cryostat sectioning to enrich diseased cells to about 80% of the total cell population, or a sample from body fluids such as serum or urine, serum or cell containing liquids, e.g. derived from fine needle aspirates) is obtained. DNA or RNA is then extracted, amplified, and analyzed with a DNA chip to determine the presence or absence of marker polynucleotide sequences. The polynucleotide probes are spotted onto a substrate in a two-dimensional matrix or array. Samples of polynucleotides are labeled and then hybridized to the probes. Double-stranded polynucleotides, comprising the labeled sample polynucleotides bound to probe polynucleotides, can be detected once the unbound portion of the sample is washed away.
The probe polynucleotides can be spotted on substrates including glass, nitrocellulose, etc. The probes can be bound to the substrate by either covalent bonds or by non-specific interactions, such as hydrophobic interactions. The sample polynucleotides can be labeled using radioactive labels, fluorophores, chromophores, etc. Techniques for constructing arrays and methods of using these arrays are described in EP0 799 897; WO 97/29212; WO 97/27317; EP 0 785 280; WO 97/02357; U.S. Pat. No. 5,593,839; U.S. Pat. No. 5,578,832; EP 0 728 520; U.S. Pat. No. 5,599,695; EP 0 721 016; U.S. Pat. No. 5,556,752; WO 95/22058; and U.S. Pat. No. 5,631,734. Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a cancer specific protein.

Data analysis from expression profiling experiments

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artifacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with cancer versus biological samples from the normal population. With other algorithms than the Affymetrix one we have obtained different numerical values representing the same expression values and expression differences upon comparison.
The differential expression E in one of the cancer groups compared to the normal population is calculated as follows. Given n average difference values d1, d2, ..., dn in the cancer population and m average difference values c1, c2, ..., cm in the population of normal individuals, it is computed by the equation:

$$E \equiv \exp\left( \frac{1}{m} \sum_{i=1}^{m} \ln(c_i) - \frac{1}{n} \sum_{i=1}^{n} \ln(d_i) \right) \text{ (equation 1)}$$

If dj<50 or ci<50 for one or more values of i and j, these particular values ci and/or dj are set to an "artificial" expression value of 50. These particular computation of E allows for a correct comparison to TaqMan results.

**EP 1 920 071 B1**

A gene is called up-regulated in cancer of good or bad outcome, if E >= average change factor 2 and if the number of absolute calls equal to 'P' in the cancer population is greater than n/2.

FIGS. 14 and 14A display the relative expression of acute phase and immune markers in fresh tumor samples of patients as described in Example 1 and as determined by Affymetrix GeneChip analysis. Response of metastatic lesions as determined by computertomography is depicted as "PR"= Partial response, "SD" = Stable Disease and "PD"= Progressive Disease. Expression levels of adjacent normal tissues (Muc = Mucosa; Liv = liver) are presented. Absolute expression levels normalized by global scaling of each indicated gene are depicted in lines. Patients are depicted in rows, starting with the patient number followed by the tumor type (primary tumor "PR" or metastatic lesion "LM"). Colour code is depicted on the upper left side to visualize tumor response.

As depicted in FIGS. 14 and 14A, expression of acute phase and immune markers correlate with clinical response of liver metastasis of CRC patients being treated with 5'FU based regimen as determined by CT determinations of the metastatic lesions. Sample type is denoted as follows: Muc1-3 = normal mucosa tissue 1-3, LIV1 = normal liver tissue, LM = Liver metastasis, PR = Primary tumor. Clinical response is denoted as follows: "Partial Response" (= PR or green color bar on top), "Stable Disease" (= SD or orange color bar on top) and "Progressive Disease" (= PD or red color bar on top). Expression of acute phase and immune markers is solely observed in the metastatic lesion and not in the primary tumor tissue. Expression is specifically elevated in metastatic lesions non responding to anti cancer regimen.

FIGS. 15 and 15A display the relative expression of candidate genes being itself acute phase and immune markers or being co-regulated in fresh tumor samples of patients as described in Example 1 and as determined by Affymetrix GeneChip analysis. Response of metastatic lesions as determined by computertomography is depicted as "PR"= Partial response, "SD" = Stable Disease and "PD"= Progressive Disease. Expression levels of adjacent normal tissues (Muc = Mucosa; Liv = liver) are presented. Absolute expression levels normalized by global scaling of each indicated gene are depicted in lines. Patients are depicted in rows, starting with the patient number followed by the tumor type (primary tumor "PR" or metastatic lesion "LM"). Colour code is depicted on the upper left side to visualize tumor response.

As depicted in FIGS. 15 and 15A, expression of acute phase markers and corregulated genes correlate with clinical response of liver metastasis of CRC patients being treated with 5'FU based regimen as determined by CT determinations of the metastatic lesions.

Table 12 lists representative nucleotide sequences of acute phase and immune markers which can be expressed to yield markers which are useful in methods of the invention.

TABLE 12

Exemplary acute phase and immune marker set and coregulated genes

| Gene Symbol | Ref. Sequences Description | Ref. Sequences | Unigene_ID | OMIM |
|---|---|---|---|---|
| APOB | apolipoprotein B precursor | NM_000384 | Hs.585 | 107730 |
| APOC1 | apolipoprotein C-I precursor | NM_001645 | Hs.268571 | 107710 |
| APOE | apolipoprotein E complement component 1, q | NM_000041 | Hs.169401 | 107741 |
| C1QA | subcomponent, alpha polypeptide precursor | NM_015991 | Hs.9641 | 120550 |
| C1QB | complement component 1, q subcomponent, beta polypeptide precursor | NM_000491 | Hs.8986 | 120570 |
| C3 | complement component 3 precursor | NM_000064 | Hs.284394 | 120700 |
| C4A | complement component 4A preproprotein | NM_007293 | Hs.278625 | 120810 |
| CRP | C-reactive protein, pentraxin-related | NM_000567 | Hs.76452 | 123260 |
| F2 | coagulation factor II precursor | NM_000506 | Hs.76530 | 176930 |
| F5 | coagulation factor V precursor | NM_000130 | Hs.30054 | 227400 |
| FGA | fibrinogen, alpha chain isoform alpha-E preproprotein | NM_000508 | Hs.90765 | 134820 |
| FGB | fibrinogen, beta chain preproprotein | NM_005141 | Hs.7645 | 134830 |
| FGG | fibrinogen, gamma chain isoform gamma-A precursor | NM_000509 | Hs.75431 | 134850 |
| ITIH3 | pre-alpha (globulin) inhibitor, H3 polypeptide | NM_002217 | Hs.76716 | 146650 |
| ITIH4 | inter-alpha (globulin) inhibitor H4 (plasma Kallikrein-sensitive glycoprotein) | NM_002218 | Hs.76415 | 600564 |
| ORM1 | orosomucoid 1 precursor | NM_000607 | Hs.572 | 138600 |
| ORM2 | orosomucoid 2 | NM_000608 | Hs.278388 | 138610 |
| SAA2 | serum amyloid A1 | NM_000331 | Hs.18162 | 104750 |

(continued)

Exemplary acute phase and immune marker set and coregulated genes

| Gene Symbol | Ref. Sequences Description | Ref. Sequences | Unigene_ID | OMIM |
|---|---|---|---|---|
| TF | transferrin | NM_001063 | Hs.284176 | 190000 |
| APCS | serum amyloid P component precursor | NM_001639 | Hs.1957 | 104770 |
| ARL7 | ADP-ribosylation factor-like 7 | NM_005737 | Hs.111554 | 604787 |
| BBOX1 | gamma-butyrobetaine hydroxylase | NM_003986 | Hs.9667 | 603312 |
| C4B | complement component 4B preproprotein | NM_000592 | Hs.278625 | 120820 |
| C4BPA | complement component 4 binding protein, alpha | NM_000715 | Hs.1012 | 120830 |
| C8B | complement component 8, beta polypeptide | NM_000066 | Hs.38069 | 120960 |
| CAST | calpastatin isoform a | NM_001750 | Hs.279607 | 114090 |
| CPB2 | plasma carboxypeptidase B2 isoform a preproprotein | NM_001872 | Hs.274495 | 603101 |
| FBP17 | formin binding protein 1 | NM 015033 | Hs.301763 | 606191 |
| FGL1 | fibrinogen-like 1 precursor | NM_004467 | Hs.107 | 605776 |
| FLJ11560 | hypothetical protein FLJ11560 | NM_025182 | Hs.301696 | - |
| FSTL3 | follistatin-like 3 glycoprotein | NM_005860 | Hs.25348 | 605343 |
| GC | group-specific component (vitamin D binding protein) | NM_000583 | Hs.198246 | 139200 |
| HXB | tenascin C (hexabrachion) | NM_002160 | Hs.289114 | 187380 |
| IGFBP1 | insulin-like growth factor binding protein 1 | NM_000596 | Hs.102122 | 146730 |
| ITIH2 | inter-alpha (globulin) inhibitor, H2 polypeptide | NM_002216 | Hs.75285 | 146640 |
| KMO | kynurenine 3-monooxygenase (kynurenine 3-hydroxylase) | NM_003679 | Hs.107318 | 603538 |
| MAGP2 | microfibril-as sociated glycoprotein 2 | NM_003480 | Hs.512842 | 601103 |
| MGC4638 | inhibin beta E | NM_031479 | Hs.279497 | - |
| NNMT | nicotinamide N-methyltransferase | NM_006169 | Hs.76669 | 600008 |
| PBX3 | pre-B-cell leukemia transcription factor 3 | NM_006195 | Hs.294101 | 176312 |
| PCDH17 | protocadherin 17 | NM_014459 | Hs.106511 | - |
| PLOD | procollagen-lysine 5-dioxygenase | NM_000302 | Hs.75093 | 153454 |
| PPP3R1 | protein phosphatase 3, regulatory subunit B, alpha isoform 1 | NM_000945 | Hs.278540 | 601302 |
| PRKCDBP | protein kinase C, delta binding protein | NM_145040 | Hs.85181 | - |
| SERPINA1 | serine (or cysteine) proteinase inhibitor, clade A (alpha-1 antiproteinase, antitrypsin), member 1 | NM_000295 | Hs.297681 | 107400 |
| SERPINE1 | plasminogen activator inhibitor-1 | NM_000602 | Hs.82085 | 173360 |
| SERPING1 | complement component 1 inhibitor precursor | NM_000062 | Hs.151242 | 606860 |
| TEGT | testis enhanced gene transcript (BAX inhibitor 1) | NM_003217 | Hs.74637 | 600748 |
| TUBB | tubulin, beta polypeptide UDP | NM_001069 | Hs.179661 | 191130 |
| UGT2B4 | glycosyltransferase 2 family, polypeptide B4 | NM_021139 | Hs.89691 | 600067 |

Table 13 displays expression levels of acute phase and immune markers discriminating between responding and non responding tumors as determined by gene expression profiling by using Affymetrix GeneChip HG U133A.

Expression data of candidate genes comparing Responding (Resp) versus non-Responding (Non-Resp) patients being treated with 5-FU based palliative chemotherapy

The average fold change factors in are depicted for those patients suffering a tumor responding (sample group 1, responding liver metastasis), or non-responding to a 5-FU based regimen (sample group 2, non responding liver metastasis). Average signal intensity within each subgroup, fold change ("Fc") ratio between the two subgroups, statistical significance according to Student's t-test and direction of change is indicated for each gene specified by name and abbreviation.

TABLE 13

|  | Affy Nr | Avg Responder | Avg Non-Responder | Fc_ Resp vs Non-Resp | T test | Direction Resp vs Non-Resp | Gene name | Gene |
|---|---|---|---|---|---|---|---|---|
| 1 | 202953_at | 305,67 | 1092,85 | -3,58 | 0,033 | Down | complement component 1, q subcomponent, beta polypeptide | C1QB |
| 2 | 203382_s_ at | 132,48 | 513,97 | -3,88 | 0,001 | Down | apolipoprotein E | APOE |
| 3 | 204416_x_ at | 856,1 | 3347,48 | -3,91 | 0,002 | Down | apolipoprotein C-I | APOC1 |
| 4 | 204714_s_ at | 231,23 | 1197,85 | -5,18 | 0,005 | Down | coagulation factor V (proaccelerin, labile factor) | F5 |
| 5 | 204988_at | 2708 | 13973,7 2 | -5,16 | 0,031 | Down | fibrinogen, B beta polypeptide | FGB |
| 6 | 205041_s_ at | 196,2 | 2513,82 | - 12,81 | 0,021 | Down | orosomucoid 1 | ORM1 |
| 7 | 205108_s_ at | 209,98 | 845,3 | -4,03 | 0,025 | Down | apolipoprotein B (including Ag(x) antigen) | APOB |
| 8 | 205650_s_ at | 493,55 | 2904,28 | -5,88 | 0,041 | Down | fibrinogen, A alpha polypeptide | FGA |
| 9 | 205754_at | 209,75 | 662,38 | -3,16 | 0,026 | Down | coagulation factor II (thrombin) | F2 |
| 10 | 214063_s_ at | 237,9 | 1677,93 | -7,05 | 0,046 | Down | transferrin | TF |
| 11 | 214428_x_ at | 779,75 | 2975,48 | -3,82 | 0,005 | Down | complement component 4A | C4A |
| 12 | 214456_x_ at | 264,88 | 4909,38 | - 18,53 | 0,038 | Down | serum amyloid A2 | SAA2 |
| 13 | 214465_at | 66,25 | 762,43 | - 11,51 | 0,026 | Down | orosomucoid 2 | ORM2 |
|  |  |  |  |  |  |  |  |  |
| 14 | 217767_at | 953,25 | 5588,37 | - 5,86 | 0,038 | Down | complement component 3 | C3 |
| 15 | 218232_at | 93,93 | 390,22 | - 4,15 | 0,005 | Down | complement component 1, q subcomponent, alpha polypeptide | C1QA |
| 16 | 219612_s_ at | 1133,45 | 7741,42 | - 6,83 | 0,038 | Down | fibrinogen, gamma polypeptide | FGG |
| 17 | 37020_at | 462,3 | 3024,4 | - 6,54 | 0,029 | Down | C-reactive protein, pentraxin-related | CRP |

Fold changes greater than 1 refers to a difference in gene expression between the first and second sample cohort. This regulation factors are mean values and may differ individually, here the combined profiles of 17 genes listed in Table 12 in a cluster analysis or a principle component analysis (PCA) will indicate the classification group for such sample.

**Data Filtering:**

Raw data of gene array analysis were acquired using Microsuite 5.0 software of Affymetrix and normalized following a standard practice of scaling the average of all gene signal intensities to a common arbitrary value. 59 Genes corresponding to Affymetrix controls (housekeeping genes, etc.) were removed from the analysis. The only exception has been done for the genes for GAPDH and Beta-actin, which expression levels were used for the normalization purposes. One hundred genes, which expression levels are routinely used in order to normalized between HG-U133A and HG-U133B GeneChips, were also removed from the analysis. Genes with potentially high levels of noise (81 probe sets), which is observed for genes with low absolute expression values (genes, which expression levels did not achieve 30 RLU (TGT=100) through all experiments), were removed from the data set. The remaining genes were preprocessed to eliminate the genes (3196 probe sets) whose signal intensities were not significantly different from their background levels and thus labeled as "Absent" by Affymetrix MicroSuite 5.0 in all experiments. We eliminated genes that were not present in at least 10% of samples (3841 probe sets). Data for remaining 15,006 probe sets were subsequently analyzed by statistical methods.

**Statistical Analysis:**

In order to optimize prediction of outcome one may use this class from the training cohort and run multiple statistical tests, suitable for group comparison including nonparametric Wilcoxon rank sum test, two-sample independent Students' t-test, Welch test, Kolmogorov-Smirnov test (for variance), and SUM-Rank test As shown, we can identify such genes with a differential expression in the responding vs non-responding group and a significance level (p-value) below 0.05. Hereby we verified statistical significance of the selected candidate genes displayed in Table 12.
Additionally one may apply correction for multiple testing errors such as Benjamini-Hochberg and may apply tests for False Discovery Detection such as permutations with Bootstrap or Jack-knife algorithms.

EXAMPLE 5

Serum analysis of CRP in serial serum samples of tumor patients suffering metastatic colorectal cancer before and during 5'FU based chemotherapy

Summary

Serial serum samples obtained from each patient as described in Example 1 were analyzed for acute phase protein levels (i.e. CRP) by using the commercially available wide range test for CRP (#74038) from Bayer Diagnostics on the ADVIA 2400 platform accorcing to manufacturers instructions and compared to clinically determined size of the metastatic tumor lesion.
As can be seen from FIGS. 16A and 16B serial measurements of serum samples of several patients revealed an increase in serum levels of CRP (red columns [mg/l]) in patients who suffered progression of metastatic disease lateron as depicted by tumor size changes (grey columns [cm$^2$]). Pretreatment samples are depicted as "A". Thereafter serum samples were obtained before each cycle of chemotherapy. As can be seen for patient G73, the increase of CRP from 14,7 mg /l at timepoint "E" to 47,5 mg/l at timepoint "F"preceeds massive progression of the metastatic liver lesion one month later at time point "G" from 6,3 to 18 cm$^2$. Similiarly for patient 179, elevation of CRP from 0,4 mg/ml at timepoint "C" to 4,3 mg/ml at time point "D"preceeds tumor growth at time point "E" from 7,5 cm$^2$ to 22,1 cm$^2$. We therefore have found, that the increase of inflammatory processes is a very early reaction to tumor recurrence/progression before it can be determined by clinical gold standard evaluation possibilities (i.e. CT Scan). However early identification of tumor progression can be used to modify applied treatment schedules and therefore can be used to monitor therapy effectiveness and optimize anti tumor regimen in order to early defeat resistance mechanisms and ultimately save time and potentially result in survival benefit.

**Claims**

1.  A computer-implemented method for predicting a clinical outcome related to a patient suffering from colorectal cancer comprising: (a) determining a predictor value algorithmically using patient sample values for (1) at least one marker selected from the group consisting of tumor markers, immune markers, acute phase markers, and (2) at least one marker that is (i) selected from the group consisting of an extracellular matrix (ECM) marker, a marker that is indicative of extracellular matrix synthesis (fibrogenesis), and a marker that is indicative of extracellular matrix degradation (fibrolysis), and (b) predicting the clinical outcome of the colorectal cancer by evaluating the predictor value wherein the marker of (1) and the marker of (2) are not the same, wherein the predictor value is (1) determined using an algorithm derived by Cox Regression Analysis and (2) is used to assess the probability that the patient will respond favorably to an antineoplastic treatment regimen.

2. A method of claim 1, wherein values for the following markers are used: (1) values for at least one tumor marker, immune marker, or acute phase markers selected from the group consisting of CEA, CAI5-3, CAI9-9, EGFR, ERBB2, ERBB3, ERBB4, c-Kit, KDR, FLT4, FLT3, c-Met, a member of the FGFR superfamily, a member of the FGFR ligand family and related splice variants, a member of the growth factor family, members of the VEGFR superfamily (KDR, FLT3, FLT4), members of the VEGFR ligand family (VEGFA, VEGFB, VEGFC, VEGFD), shedded domains of members of growth factors, interleukins, interleukin receptors, complement factors, acute phase proteins and hormones; and (2) at least one marker that is: (i) an extracellular matrix (ECM) marker selected from the group consisting of collagens, basal adhesion proteins, entactin, proteoglycans, and glycosaminoglycans P, a member of the collagen superfamily; or (ii) a marker that is indicative of extracellular matrix synthesis (fibrogenesis) selected from the group consisting of preforms of collagens, basal adhesion proteins, entactin, proteoglycans, and glycosaminoglycans or prepro-peptides thereof; or (iii) a marker that is indicative of extracellular matrix degradation (fibrolysis) selected from the group consisting of the MMP superfamily, or associated inhibitors thereof including the TIMP superfamily.

3. A method of claim 1, wherein the patient suffers from colorectal cancer and wherein values for one or more of the following markers are used: PIIINP, Collagen IV, Collagen VI, Tenascin, Laminin, HA, CRP, MMP-2, TIMP-I, MMP-9/TEVIP-I complex, CEA, CAI5-3, CAI9-9, IL2R, IL6, Gastrin, Her-2/neu, EGFr, uPA, and VEGFI65.

4. A method of claim 1, further comprising comparing a predictor value to a comparative data set comprised of one or more numerical values, or range of numerical values, that are associated with a colorectal cancer.

5. A method of claim 1, wherein the predictor value is determined using a linear or non linear function algorithm which is derived by: (a) compiling a data set comprising neoplastic disease-related marker data for a first group of subjects, wherein the neoplastic disease-related marker data relates to (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis); (b) deriving a linear or nonlinear function algorithm from the compiled data set through application of at least one analytical methodology selected from the group consisting of discriminant function analysis, nonparametric regression analysis, classification trees, and neural networks; (c) calculating validation predictor values for a second group of subjects by inputting data comprising neoplastic disease-related marker data for the second group of subjects into the algorithm derived in step (b); (d) comparing validation predictor values calculated in step (c) with neoplastic disease-related scores for the second group of subjects; and (e) if the validation predictor values determined in step (c) do not correlate within a clinically-acceptable tolerance level with validation predictor values for the second group of subjects, performing the following operations (i)-(iii) until such tolerance is satisfied: (i) modifying the algorithm on a basis or bases comprising (1) revising the data set for the first group of subjects, and (2) revising or changing the analytical methodology (ii) calculating validation predictor values for the second group of subjects by inputting data comprising neoplasm-related marker data for the second group of subjects into the modified algorithm (iii) assessing whether validation biopsy score values calculated using the modified algorithm correlate with predictor values for the second group of subjects within the clinically-acceptable tolerance level.

6. A computer readable medium having stored thereon a data structure comprising a data field containing data representing an algorithm which generates predictor values that can be used to predict a patient's response to an antineoplastic treatment regimen, wherein the algorithm uses values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

7. A data structure stored in a computer-readable medium that may be read by a microprocessor and that comprises at least one code that uniquely identifies predictor values determined in accordance with claim 1.

8. A data structure comprising a data field containing data representing an algorithm which generates predictor values that can be used to predict a patient's response to an anti-neoplastic treatment regimen, said data structure being stored in a computer-readable medium that may be read by a microprocessor and that comprises at least one code that uniquely identifies data representing values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

9. A kit comprising: (a) a data structure comprising a data field containing data representing an algorithm which generates predictor values that can be used to predict a patient's response to an anti-neoplastic treatment regimen, said data structure being stored in a computer-readable medium that may be read by a microprocessor and that comprises at least one code that uniquely identifies predictor values determined in accordance with claim 1; and (b) one or more immunoassays comprising immunoreactive reagents, wherein the immunoassays detect and determine values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

10. A kit comprising one or more immunoassays that detect and determine values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

11. A system comprising: (a) a data structure stored in a computer-readable medium that may be read by a microprocessor and that comprises at least one code that uniquely identifies predictor values determined in accordance with claim 1; and (b) one or more immunoassays that detect and determine values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

12. A system comprising: (a) a data structure comprising a data field containing data representing an algorithm which generates predictor values that can be used to predict a patient's response to an anti-neoplastic treatment regimen, said data structure being stored in a computer-readable medium that may be read by a microprocessor and that comprises at least one code that uniquely identifies values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis); and (b) one or more immunoassays that detect and determine values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

13. A computer-implemented method comprising predicting the status or progress of a colorectal cancer in a patient by evaluating two or more predictor values determined at one or more time points by a method of claim 1.

14. A computer-implemented method comprising predicting the status or progress of a colorectal cancer in a patient by evaluating two or more predictor values for (1) at least one tumor marker or at least one immune marker, and (2) at least one marker that is (i) an extracellular matrix (ECM) marker (ii) a marker that is indicative of extracellular matrix synthesis (fibrogenesis), or (iii) a marker that is indicative of extracellular matrix degradation (fibrolysis).

15. A computer-implemented method for assessing the prognosis of a patient suffering from, or at risk of developing, a colorectal cancer comprising evaluating predictor values determined at two or more time points, wherein: (a) predictor values are determined algorithmically using patient sample values for (1) at least one marker selected from the group consisting of tumor markers , immune markers, and acute phase markers, and (2) at least one marker that is (i) an extracellular matrix (ECM) marker (ii) a marker that is indicative of extracellular matrix synthesis (fibrogenesis), or (iii) a marker that IS indicative of extracellular matrix degradation (fibrolysis); and (b) the patient's prognosis is assessed by evaluating the predictor values determined using an algorithm derived by Cox Regression Analysis.

**EP 1 920 071 B1**

**Patentansprüche**

1. Computerimplementiertes Verfahren zur Vorhersage eines klinischen Ergebnisses in Bezug auf einen Patienten, der an einem Kolorektalkarzinom leidet, umfassend: (a) algorithmisches Bestimmen eines Vorhersagewerts unter Verwendung von Patientenprobenwerten für (1) mindestens einen Marker, ausgewählt aus der Gruppe, bestehend aus Tumor-Markern, Immun-Markern, Akutphasen-Markern, und (2) mindestens einen Marker, der (i) aus der Gruppe ausgewählt ist, bestehend aus einem Marker für die extrazelluläre Matrix (Extracellular Matrix, ECM), einem Marker, der eine Synthese extrazellulärer Matrix (Fibrogenese) anzeigt, und einem Marker, der eine Zersetzung extrazellulärer Matrix (Fibrolyse) anzeigt, und (b) Vorhersagen des klinischen Ergebnisses des Kolorektalkarzinoms durch Auswerten des Vorhersagewerts, wobei der Marker von (1) und der Marker von (2) nicht dieselben sind, wobei der Vorhersagewert (1) unter Verwendung eines Algorithmus bestimmt wird, der durch eine Cox-Regressionsanalyse abgeleitet wird, und (2) verwendet wird, um die Wahrscheinlichkeit zu bewerten, dass der Patient vorteilhaft auf ein antineoplastisches Behandlungsregime reagiert.

2. Verfahren nach Anspruch 1, wobei Werte für die folgenden Marker verwendet werden: (1) Werte für mindestens einen Tumor-Marker, Immun-Marker oder Akutphasen-Marker, ausgewählt aus der Gruppe, bestehend aus CEA, CAI5-3, CAI9-9, EGFR, ERBB2, ERBB3, ERBB4, c-Kit, KDR, FLT4, FLT3, c-Met, ein Mitglied der FGFR-Überfamilie, ein Mitglied der FGFR-Ligandenfamilie und darauf bezogene Splice-Varianten, ein Mitglied der Wachstumsfaktorfamilie, Mitglieder der VEGFR-Überfamilie (KDR, FLT3, FLT4) Mitglieder der VEGFR-Ligandenfamilie (VEGFA, VEGFB, VEGFC, VEGFD), durch Shedding abgetrennte Domänen von Elementen von Wachstumsfaktoren, Interleukine, Interleukinrezeptoren, Komplementfaktoren, Akutphasenproteine und -hormone; und (2) mindestens einen Marker, der Folgendes ist: (i) ein Marker für die extrazelluläre Matrix (ECM), ausgewählt aus der Gruppe, bestehend aus Collagenen, basalen Adhäsionsproteinen, Entactin, Proteoglycanen und Glycosaminoglycanen P, einem Mitglied der Collagen-Überfamilie; oder (ii) ein Marker, der eine Synthese extrazellulärer Matrix (Fibrogenese) anzeigt, ausgewählt aus der Gruppe, bestehend aus Vorformen von Collagenen, basalen Adhäsionsproteinen, Entactin, Proteoglycanen und Glycosaminoglycanen oder Präpropeptiden davon; oder (iii) ein Marker, der eine Zersetzung extrazellulärer Matrix (Fibrolyse) anzeigt, ausgewählt aus der Gruppe, bestehend aus der MMP-Überfamilie oder zugehörigen Inhibitoren davon, umfassend die TIMP-Überfamilie.

3. Verfahren nach Anspruch 1, wobei der Patient an einem Kolorektalkarzinom leidet und wobei Werte für einen oder mehrere der folgenden Marker verwendet werden: PIIINP, Collagen IV, Collagen VI, Tenascin, Laminin, HA, CRP, MMP-2, TIMP-I, MMP-9/TEVIP-I-Komplex, CEA, CAI5-3, CAI9-9, IL2R, IL6, Gastrin, Her-2/neu, EGFr, uPA und VEGFI65.

4. Verfahren nach Anspruch 1, ferner umfassend Vergleichen eines Vorhersagewerts mit einem Vergleichsdatensatz, der aus einem oder mehreren numerischen Werten oder einem Bereich von numerischen Werten besteht, die mit einem Kolorektalkarzinom verbunden sind.

5. Verfahren nach Anspruch 1, wobei der Vorhersagewert unter Verwendung eines linearen oder nichtlinearen Funktionsalgorithmus bestimmt wird, welcher abgeleitet wird durch: (a) Kompilieren eines Datensatzes, welcher Daten eines Markers umfasst, der auf eine neoplastische Erkrankung bezogen ist, für eine erste Gruppe von Subjekten, wobei sich die Daten des Markers, der auf eine neoplastische Erkrankung bezogen ist, beziehen auf: (1) mindestens einen Tumor-Marker oder mindestens einen Immun-Marker und (2) mindestens einen Marker, welcher (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt; (b) Ableiten eines linearen oder nichtlinearen Funktionsalgorithmus aus dem kompilierten Datensatz durch Anwendung mindestens einer analytischen Methodik, ausgewählt aus der Gruppe, bestehend aus Diskriminanzfunktionsanalyse, nichtparametrischer Regressionsanalyse, Klassifikationsbäumen und neuralen Netzen; (c) Berechnen von Validations-Vorhersagewerten für eine zweite Gruppe von Subjekten durch Eingeben von Daten, welche Daten eines auf eine neoplastische Erkrankung bezogenen Markers für die zweite Gruppe von Subjekten umfassen, in den im Schritt (b) abgeleiteten Algorithmus; (d) Vergleichen der im Schritt (c) berechneten Validations-Vorhersagewerte mit auf eine neoplastische Erkrankung bezogenen Ergebnissen für die zweite Gruppe von Subjekten; und (e) wenn die im Schritt (c) bestimmten Validations-Vorhersagewerte nicht innerhalb eines klinisch akzeptablen Toleranzniveaus mit den Validations-Vorhersagewerten für die zweite Gruppe von Subjekten korrelieren, Durchführen der folgenden Operationen (i) bis (iii), bis eine solche Toleranz eingehalten ist: (i) Modifizieren des Algorithmus auf einer Basis oder Basen, umfassend: (1) Überarbeiten des Datensatzes für die erste Gruppe von Subjekten und (2) Überarbeiten oder Ändern der analytischen Methodik, (ii) Berechnen von Validations-Vorhersagewerten für die zweite Gruppe von Subjekten durch Eingeben von Daten, welche Neoplasie-bezogene Marker-Daten für die zweite Gruppe von Subjekten umfassen, in den modifizierten Algorithmus, (iii) Bewerten, ob unter Verwendung des modifizierten Algorithmus berechnete Validations-Biopsie-Ergebniswerte innerhalb des klinisch akzeptablen Toleranzniveaus mit Vorhersagewerten für die zweite Gruppe von Subjekten korrelieren.

6.  Computerlesbares Medium mit einer darauf gespeicherten Datenstruktur, welche ein Datenfeld umfasst, das Daten enthält, die einen Algorithmus repräsentieren, welcher Vorhersagewerte erzeugt, die verwendet werden können, um die Reaktion eines Patienten auf ein antineoplastisches Behandlungsregime vorherzusagen, wobei in dem Algorithmus Werte für (1) mindestens einen Tumor-Marker oder mindestens einen Immun-Marker und (2) mindestens einen Marker verwendet werden, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

7.  Datenstruktur, gespeichert in einem computerlesbaren Medium, welches von einem Mikroprozessor gelesen werden kann und welches mindestens einen Code umfasst, der eindeutig Vorhersagewerte identifiziert, die gemäß Anspruch 1 bestimmt werden.

8.  Datenstruktur, umfassend ein Datenfeld, das Daten enthält, die einen Algorithmus repräsentieren, welcher Vorhersagewerte erzeugt, die verwendet werden können, um die Reaktion eines Patienten auf ein antineoplastisches Behandlungsregime vorherzusagen, wobei die Datenstruktur in einem computerlesbaren Medium gespeichert ist, welches von einem Mikroprozessor gelesen werden kann und welches mindestens einen Code umfasst, der eindeutig Daten identifiziert, die Werte für (1) mindestens einen Tumor-Marker oder mindestens einen Immun-Marker und (2) mindestens einen Marker repräsentieren, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

9.  Kit, umfassend: (a) eine Datenstruktur, welche ein Datenfeld umfasst, das Daten enthält, die einen Algorithmus repräsentieren, welcher Vorhersagewerte erzeugt, die verwendet werden können, um die Reaktion eines Patienten auf ein antineoplastisches Behandlungsregime vorherzusagen, wobei die Datenstruktur in einem computerlesbaren Medium gespeichert ist, welches von einem Mikroprozessor gelesen werden kann und welches mindestens einen Code umfasst, der eindeutig Vorhersagewerte identifiziert, die gemäß Anspruch 1 bestimmt werden; und (b) ein oder mehrere Immunoassays, welche immunreaktive Reagenzien umfassen, wobei die Immunoassays Werte für (1) mindestens einen Tumor-Marker oder mindestens einen Immun-Marker und (2) mindestens einen Marker erfassen und bestimmen, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

10. Kit, umfassend ein oder mehrere Immunoassays, welche Werte für (1) mindestens einen Tumor-Marker oder mindestens einen Immun-Marker und (2) mindestens einen Marker erfassen und bestimmen, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

11. System, umfassend: (a) eine Datenstruktur, gespeichert in einem computerlesbaren Medium, welches von einem Mikroprozessor gelesen werden kann und welches mindestens einen Code umfasst, der eindeutig Vorhersagewerte identifiziert, die gemäß Anspruch 1 bestimmt werden; und (b) ein oder mehrere Immunoassays, welche Werte für (1) mindestens einen Tumor-Marker oder mindestens einen Immun-Marker und (2) mindestens einen Marker erfassen und bestimmen, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

12. System, umfassend: (a) eine Datenstruktur, welche ein Datenfeld umfasst, das Daten enthält, die einen Algorithmus repräsentieren, welcher Vorhersagewerte erzeugt, die verwendet werden können, um die Reaktion eines Patienten auf ein antineoplastisches Behandlungsregime vorherzusagen, wobei die Datenstruktur in einem computerlesbaren Medium gespeichert ist, welches von einem Mikroprozessor gelesen werden kann und welches mindestens einen Code umfasst, der eindeutig Werte für (1) mindestens einen Tumor-Marker oder mindestens einen Immun-Marker und (2) mindestens einen Marker identifiziert, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt; und (b) ein oder mehrere Immunoassays, welche Werte für (1) mindestens einen Tumor-Marker oder mindestens einen Immun-Marker und (2) mindestens einen Marker erfassen und bestimmen, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

13. Computerimplementiertes Verfahren, umfassend Vorhersagen des Status oder Fortschritts eines Kolorektalkarzinoms bei einem Patienten durch Auswerten von zwei oder mehr Vorhersagewerten, die zu einem oder mehreren Zeitpunkten durch ein Verfahren nach Anspruch 1 bestimmt werden.

14. Computerimplementiertes Verfahren, umfassend Vorhersagen des Status oder Fortschritts eines Kolorektalkarzinoms bei einem Patienten durch Auswerten von zwei oder mehr Vorhersagewerten für (1) mindestens einen Tumor-Marker oder mindestens einen Immun-Marker und (2) mindestens einen Marker, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt.

15. Computerimplementiertes Verfahren zum Bewerten der Prognose für einen Patienten, der an einem Kolorektalkarzinom leidet oder das Risiko aufweist, ein solches zu entwickeln, umfassend Auswerten von Vorhersagewerten, die zu einem oder mehreren Zeitpunkten bestimmt werden, wobei (a) die Vorhersagewerte algorithmisch bestimmt werden unter Verwendung von Patientenprobenwerten für (1) mindestens einen Marker, ausgewählt aus der Gruppe, bestehend aus Tumor-Markern, Immun-Markern und Akutphasen-Markern, und (2) mindestens einen Marker, der (i) ein Marker für die extrazelluläre Matrix (ECM), (ii) ein Marker, der eine Synthese extrazellulärer Matrix (Fibrogenese) anzeigt, oder (iii) ein Marker ist, der eine Zersetzung extrazellulärer Matrix (Fibrolyse) anzeigt; und (b) die Prognose des Patienten bewertet wird durch Auswerten der Vorhersagewerte, die unter Verwendung eines Algorithmus bestimmt werden, der durch eine Cox-Regressionsanalyse abgeleitet wird.

**Revendications**

1. Procédé mis en oeuvre par ordinateur pour prédire un résultat clinique lié à un patient atteint d'un cancer colorectal comprenant les étapes consistant à : (a) déterminer une valeur de prédicteur algorithmiquement en utilisant des valeurs d'échantillons de patient pour (1) au moins un marqueur choisi dans le groupe constitué de marqueurs tumoraux, de marqueurs immunitaires, de marqueurs de phase aiguë, et (2) au moins un marqueur qui est (i) choisi dans le groupe constitué d'un marqueur de matrice extracellulaire (ECM), d'un marqueur qui est indicatif d'une synthèse de matrice extracellulaire (fibrogenèse), et d'un marqueur qui indique la dégradation de matrice extracellulaire (fibrolyse), et (b) prédire le résultat clinique du cancer colorectal en évaluant la valeur de prédicteur dans laquelle le marqueur de (1) et le marqueur de (2) ne sont pas identiques, où la valeur de prédicteur est (1) déterminée à l'aide d'un algorithme dérivé de l'analyse de régression de Cox et (2) est utilisée pour évaluer la probabilité que le patient réponde favorablement à un régime de traitement antinéoplasique.

2. Procédé selon la revendication 1, dans lequel les valeurs pour les marqueurs suivants sont utilisées : (1) valeurs pour au moins un marqueur tumoral, un marqueur immunitaire, ou des marqueurs de phase aiguë choisis dans le groupe constitué par un CEA, un CAI5-3, un CAI9-9, un EGFR, un ERBB2, un ERBB3, un ERBB4, un c-Kit, un KDR, un FLT4, un FLT3, un c-Met, un membre de la superfamille FGFR, un membre de la famille des ligands FGFR et variants d'épissage associés, un membre de la famille des facteurs de croissance, des membres de la superfamille VEGFR (KDR, FLT3, FLT4), des membres de la famille des ligands VEGFR (VEGFA, VEGFB, VEGFC, VEGFD), des domaines englobés des membres des facteurs de croissance, des interleukines, des récepteurs d'interleukine, des facteurs de complément, des protéines et hormones de phase aiguë ; et (2) au moins un marqueur qui est : (i) un marqueur de matrice extracellulaire (ECM) choisi dans le groupe constitué par les collagènes, les protéines d'adhésion basale, l'entactine, les protéoglycanes et les glycosaminoglycanes P, un membre de la superfamille du collagène ; ou (ii) un marqueur qui est indicatif de la synthèse de matrice extracellulaire (fibrogenèse) choisie dans le groupe constitué par les préformes de collagènes, les protéines d'adhésion basale, l'entactine, les protéoglycanes et les glycosaminoglycanes ou leurs prépro-peptides ; ou (iii) un marqueur qui est indicatif de la dégradation de matrice extracellulaire (fibrolyse) choisi dans le groupe constitué par la superfamille des MMP, ou ses inhibiteurs associés, y compris la superfamille de TIMP.

3. Procédé selon la revendication 1, dans lequel le patient souffre d'un cancer colorectal et dans lequel des valeurs pour un ou plusieurs des marqueurs suivants sont utilisées : PIIINP, Collagène IV, Collagène VI, Ténascine, Laminine, HA, CRP, MMP-2, TIMP-I, complexe MMP-9/TEVIP-I, CEA, CAI5-3, CAI9-9, IL2R, IL6, Gastrine, Her-2/neu, EGFr, uPA, et VEGFI65.

**4.** Procédé selon la revendication 1, comprenant en outre le fait de comparer une valeur de prédicteur à un ensemble de données comparatives comprenant une ou plusieurs valeurs numériques, ou une plage de valeurs numériques, qui sont associées à un cancer colorectal.

**5.** Procédé selon la revendication 1, dans lequel la valeur de prédicteur est déterminée en utilisant un algorithme de fonction linéaire ou non linéaire qui est obtenu en : (a) compilant un ensemble de données comprenant des données de marqueur liées à une maladie néoplasique pour un premier groupe de sujets, dans lequel les données de marqueur liées à une maladie néoplasique concernent (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse) ou (iii) un marqueur qui est indicatif de la dégradation de matrice extracellulaire (fibrolyse) ; (b) dérivant un algorithme de fonction linéaire ou non linéaire à partir de l'ensemble de données compilées en appliquant au moins une méthodologie analytique choisie dans le groupe constitué par l'analyse de fonction discriminante, l'analyse de régression non paramétrique, les arbres de classification, et les réseaux neuronaux ; (c) calculant des valeurs de prédicteur de validation pour un second groupe de sujets en introduisant des données comprenant des données de marqueur liées à une maladie néoplasique pour le second groupe de sujets dans l'algorithme dérivé à l'étape (b) ; (d) comparant les valeurs de prédicteur de validation calculées à l'étape (c) avec les scores liés à une maladie néoplasique pour le second groupe de sujets ; et (e) si les valeurs de prédicteur de validation déterminées à l'étape (c) ne correspondent pas à un niveau de tolérance cliniquement acceptable avec les valeurs de prédicteur de validation pour le second groupe de sujets, effectuant les opérations suivantes (i) - (iii) jusqu'à ce que cette tolérance est satisfaite : (i) en modifiant l'algorithme sur une base ou des bases comprenant (1) la révision de l'ensemble de données pour le premier groupe de sujets, et (2) la révision ou la modification de la méthodologie analytique (ii) le calcul des valeurs prédictives de validation pour le second groupe de sujets en introduisant des données comprenant des données de marqueur liées au néoplasme pour le second groupe de sujets dans l'algorithme modifié (iii) l'évaluation si les valeurs de score de biopsie de validation calculées en utilisant l'algorithme modifié correspondent avec les valeurs de prédicteur pour le second groupe de sujets à l'intérieur du niveau de tolérance cliniquement acceptable.

**6.** Support lisible par ordinateur sur lequel est stockée une structure de données comprenant un champ de données contenant des données représentant un algorithme qui génère des valeurs de prédicteur pouvant être utilisées pour prédire la réponse d'un patient à un régime de traitement antinéoplasique, dans lequel l'algorithme utilise des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

**7.** Structure de données stockée dans un support lisible par ordinateur qui peut être lu par un microprocesseur et qui comprend au moins un code identifiant de manière unique des valeurs de prédicteur déterminées conformément à la revendication 1.

**8.** Structure de données comprenant un champ de données contenant des données représentant un algorithme qui génère des valeurs de prédicteur pouvant être utilisées pour prédire la réponse d'un patient à un régime de traitement antinéoplasique, ladite structure de données étant stockée dans un support lisible par ordinateur pouvant être lu par un microprocesseur et comprenant au moins un code qui identifie de manière unique des données représentant des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

**9.** Kit comprenant : (a) une structure de données comprenant un champ de données contenant des données représentant un algorithme qui génère des valeurs de prédicteur pouvant être utilisées pour prédire la réponse d'un patient à un régime de traitement antinéoplasique, ladite structure de données étant stockée dans un support lisible par ordinateur qui peut être lu par un microprocesseur et qui comprend au moins un code identifiant de manière unique des valeurs de prédicteur déterminées conformément à la revendication 1 ; et (b) un ou plusieurs immunoessais comprenant des réactifs immunoréactifs, où les immunoessais détectent et déterminent des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

**10.** Kit comprenant un ou plusieurs immunoessais qui détectent et déterminent des valeurs pour (1) au moins un

**EP 1 920 071 B1**

marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

11. Système comprenant: (a) une structure de données stockée dans un support lisible par ordinateur qui peut être lu par un microprocesseur et qui comprend au moins un code identifiant de manière unique des valeurs de prédicteur déterminées conformément à la revendication 1 ; et (b) un ou plusieurs immunoessais qui détectent et déterminent des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

12. Système comprenant : (a) une structure de données comprenant un champ de données contenant des données représentant un algorithme qui génère des valeurs de prédicteur pouvant être utilisées pour prédire la réponse d'un patient à un régime de traitement antinéoplasique, ladite structure de données étant stockée dans un support lisible par ordinateur qui peut être lu par un microprocesseur et qui comprend au moins un code qui identifie de manière unique des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif d'une dégradation de matrice extracellulaire (fibrolyse) ; et (b) un ou plusieurs immunoessais qui détectent et déterminent des valeurs pour (1) au moins un marqueur tumoral ou au moins un marqueur immunitaire, et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse).

13. Procédé mis en oeuvre par ordinateur
comprenant l'étape consistant à prédire l'état ou la progression d'un cancer colorectal chez un patient en évaluant deux ou plus valeurs de prédicteur déterminées à un ou plusieurs moments par un procédé selon la revendication 1.

14. Procédé mis en oeuvre par ordinateur
Comprenant une étape consistant à prédire l'état ou la progression d'un cancer colorectal chez un patient en évaluant deux ou plus valeurs de prédicteur pour (1) au moins un marqueur tumoral ou au moins un marqueur immunitaire et (2) au moins un marqueur qui est (i) un marqueur de matrice extracellulaire (ECM) (ii) un marqueur qui est indicatif de la synthèse de matrice extracellulaire (fibrogenèse), ou (iii) un marqueur qui est indicatif de la dégradation de matrice extracellulaire (fibrolyse)

15. Procédé mis en oeuvre par ordinateur
pour évaluer le pronostic d'un patient atteint ou à risque de développer un cancer colorectal consistant à évaluer des valeurs de prédicteur déterminées à deux moments ou plus, où : (a) les valeurs de prédicteur sont déterminées par algorithme en utilisant des valeurs d'échantillon du patient pour (1) au moins un marqueur choisi dans le groupe constitué de marqueurs tumoraux, marqueurs immunitaires et marqueurs de phase aiguë, et (2) au moins un marqueur qui est (!) un marqueur de matrice extracellulaire (ECM) (ii) un marqueur qui est indicatif de la synthèse de matrice extracellulaire (fibrogenèse) ou (iii) un marqueur qui est indicatif de la dégradation de matrice extracellulaire (fibrolyse) ; et (b) le pronostic du patient est évalué en évaluant les valeurs de prédicteur déterminées en utilisant un algorithme dérivé de l'analyse de régression de Cox.

# FIG. 1

## KAPLAN MEIER ANALYSIS OF ALGORITHM

# FIG. 2

KAPLAN MEIER ANALYSIS OF GASTRIN

- - - - GASTRIN HIGH
—▲— GASTRIN LOW

GASTRIN CO 25,4

# FIG. 3

<u>KAPLAN MEIER ANALYSIS OF CA 19-9</u>

---- CA 19-9 HIGH
——▲—— CA 19-9 LOW

CA 19-9 CO 37

# FIG. 4

KAPLAN MEIER ANALYSIS OF TIMP-1

- - - - TIMP 1 IMMUNO HIGH
—▲— TIMP 1 IMMUNO LOW

TIMP-1 IMMUNO CO 1037,6

# FIG. 5

KAPLAN MEIER ANALYSIS OF MMP-2

- - - - MMP-2 > 675
——▲—— MMP-2 < 675

MMP-2 CO 675

# FIG. 6

KAPLAN MEIER ANALYSIS OF EGFr

---- EGFr < 45
—▲— EGFr > 45

EGFr CO 45

# FIG. 7

## KAPLAN MEIER ANALYSIS OF VEGF

- - - - VEGF HIGH
—▲— VEGF LOW

VEGF CO 221,1

# FIG. 8

## KAPLAN MEIER ANALYSIS OF CEA

---- CEA > 100

—▲— CEA < 100

CEA CO 100

# FIG. 9

KAPLAN MEIER ANALYSIS OF MCT-V ALGORITHM VALUES

```
----  MCT-V HIGH
--▲-- MCT-V LOW
```

MMP2 COL6 TENASCIN-VEGF

FIG. 10

PRINCIPAL COMPONENT ANALYSIS BY USING ALL PARAMETERS

# FIG. 10A

## PRINCIPAL COMPONENT ANALYSIS BY USING ALL PARAMETERS

**Genedata Expressionist Analyst**

Experiment groups
☐ Dead < 19 mo
■ Alive > 40 mo w/o Ø18
Gene groups
☐ All Genes

Created by 'diagnostics' during session 'SerumStudy WIRA II 2003/2004' on Mon Sep 20 16:00:33 CEST 2004 using Genedata Expressionist Analyst, EPro 1.0.16.

# FIG. 11

PRINCIPAL COMPONENT ANALYSIS BY USING TOP 5 PARAMETERS

GENEDATA EXPRESSIONIST ANALYST

EXPERIMENT GROUPS

☐ DEAD < 19 mo

▦ ALIVE > 40 mo w/o G18

GENE GROUPS

■ FROM DEAD < 19 vs ALIVE > 40: MULTIPLE TESTS:
WELCH, KOLMOGOROV–SMIMOV, WILCOXER

# FIG. 11A

## PRINCIPAL COMPONENT ANALYSIS BY USING TOP 5 PARAMETERS

# FIG. 12

## qRT-EXPRESSION ANALYIS OF ERB FAMILY MEMBERS IN FFPE TISSUE FROM PRIMARY TUMORS

# FIG. 12A

qRT-EXPRESSION ANALYIS OF ERB FAMILY MEMBERS IN FFPE TISSUE FROM
PRIMARY TUMORS

# FIG. 13

KAPLAN MEIER ANALYSIS OF TIMP-1 AND EGFr IN SERUM

- - - - TIMP 1 HIGH AND EGFr LOW

-▲- TIMP 1 LOW AND/OR EGFr NORMAL

TIMP-1 CO 949 AND EGFr CO 45

# FIG. 14

## EXPRESSION PROFILING OF PREDICTIVE SERUM MARKERS RESEMBLING LIVER REACTION TO METASTATIC LESION

# FIG. 14A

## EXPRESSION PROFILING OF PREDICTIVE SERUM MARKERS RESEMBLING LIVER REACTION TO METASTATIC LESION

**Genedata Expressionist Analyst**

Created by 'diagnostics' during session 'WIRA' on Fri Jun 17 14:47:37 CEST 2005 using Genedata Expressionist Analyst, EPro 1.0.31.

# FIG. 15

## EXPRESSION PROFILING OF PREDICTIVE SERUM MARKERS RESEMBLING LIVER REACTION TO METASTATIC LESION

# FIG. 15A

## EXPRESSION PROFILING OF PREDICTIVE SERUM MARKERS RESEMBLING LIVER REACTION TO METASTATIC LESION

## FIG. 16A

## FIG. 16B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030219842 A
- US 20030180819 A
- US 20040157278 A
- US 20040146921 A
- US 20040146879 A
- US 6262333 B
- US 20040018525 A1
- US 20030082652 A1
- EP 0799897 A
- WO 9729212 A
- WO 9727317 A
- EP 0785280 A
- WO 9702357 A
- US 5593839 A
- US 5578832 A
- EP 0728520 A
- US 5599695 A
- EP 0721016 A
- US 5556752 A
- WO 9522058 A
- US 5631734 A

**Non-patent literature cited in the description**

- **HANKE et al.** *British Journal of Cancer,* 2003, vol. 88, 1248-50
- **N. YUKAWA et al.** Plasma concentration of tissue inhibitor of matrix metalloproteinase 1 in patients with colorectal carcinoma. *Brit. J. of Surgery,* 2001, vol. 88, 1596-1601
- **GLOJNARIC.** *Clin. Chem. Lab. Med. 2001,* February 2001, vol. 39 (2), 129-133
- **SAMBROOK et al.** MOLECULAR CLONING: A LABORATORY MANUAL. 1989
- **AUSUBEL et al.** CURRENT PROTOCOLS IN MOLECULAR BIOLOGY. John Wiley & Sons, 1989
- **VAN HEEKE ; SCHUSTER.** *J. Biol. Chem.,* 1989, vol. 264, 5503-5509
- **TAKAMATSU.** *EMBO J.,* 1987, vol. 6, 307-311
- **CORUZZI et al.** *EMBO J.,* 1984, vol. 3, 1671-1680
- **BROGLIE et al.** *Science,* 1984, vol. 224, 838-843
- **WINTER et al.** *Results Probl. Cell Differ.,* 1991, vol. 17, 85-105
- MCGRAW HILL YEARBOOK OF SCIENCE AND TECHNOLOGY. McGraw Hill, 1992, 191-196
- **ENGELHARD et al.** *Proc. Nat. Acad. Sci.,* 1994, vol. 91, 3224-3227
- **LOGAN ; SHENK.** *Proc. Natl. Acad. Sci.,* 1984, vol. 81, 3655-3659
- **SCHARF et al.** *Results Probl. Cell Differ.,* 1994, vol. 20, 125-162
- ANIMAL CELL CULTURE. 1986
- **WIGLER et al.** *Cell,* 1977, vol. 11, 223-232
- **LOWY et al.** *Cell,* 1980, vol. 22, 817-823
- **WIGLER et al.** *Proc. Natl. Acad. Sci.,* 1980, vol. 77, 3567-3570
- **COLBERE-GARAPIN et al.** *J. Mol. Biol.,* 1981, vol. 150, 114
- **HARTMAN ; MULLIGAN.** *Proc. Natl. Acad. Sci.,* 1988, vol. 85, 8047-8051
- **RHODES et al.** *Methods Mol. Biol.,* 1995, vol. 55, 121-131
- **HAMPTON et al.** SEROLOGICAL METHODS: A LABORATORY MANUAL. APS Press, 1990
- **MADDOX et al.** *J. Exp. Med.,* 1983, vol. 158, 1211-1216
- **PORATH et al.** *Prot. Exp. Purif.,* 1992, vol. 3, 263-281
- **KROLL et al.** *DNA Cell Biol.,* 1993, vol. 12, 441-453
- **CARUTHERS et al.** *Nucl. Acids Res. Symp. Ser.,* 1980, 215-223
- **HORN et al.** *Nucl. Acids Res. Symp. Ser.,* 1980, 225-232
- **MERRIFIELD.** *J. Am. Chem. Soc.,* 1963, vol. 85, 2149-2154
- **ROBERGE et al.** *Science,* 1995, vol. 269, 202-204
- **CREIGHTON.** PROTEINS: STRUCTURES AND MOLECULAR PRINCIPLES. WH and Co, 1983